(19) Europäisches Patentamt
European Patent Office
Office européen des brevets

(11) EP 4 428 121 A1

(12) **EUROPEAN PATENT APPLICATION**

(43) Date of publication:
**11.09.2024 Bulletin 2024/37**

(21) Application number: **23160615.3**

(22) Date of filing: **07.03.2023**

(51) International Patent Classification (IPC):
***C07D 213/36*** *(2006.01)* ***C07D 239/42*** *(2006.01)*
***C07D 239/47*** *(2006.01)* ***C07D 239/94*** *(2006.01)*
***C07D 241/20*** *(2006.01)* ***C07D 241/44*** *(2006.01)*
***C07D 257/04*** *(2006.01)* ***C07D 401/12*** *(2006.01)*
***C07D 403/12*** *(2006.01)* ***C07D 471/04*** *(2006.01)*
***A61P 25/18*** *(2006.01)* ***A61P 25/28*** *(2006.01)*
***A61P 35/00*** *(2006.01)* ***A61K 31/44*** *(2006.01)*
***C07C 15/04*** *(2006.01)*

(52) Cooperative Patent Classification (CPC):
**C07D 401/12; A61P 25/18; A61P 25/28; A61P 35/00; C07C 229/36; C07C 309/04; C07D 213/36; C07D 239/42; C07D 239/47; C07D 239/94; C07D 241/20; C07D 241/44; C07D 257/04; C07D 403/12; C07D 471/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Vesper Bio Aps**
**1610 København V (DK)**

(72) Inventors:
- **Cases-Thomas, Manuel Javier**
  **2100 Copenhagen (DK)**
- **Kjølby, Mads Fuglsang**
  **2100 Copenhagen (DK)**
- **Nykjær, Anders**
  **2100 Copenhagen (DK)**
- **Little, Paul Brian**
  **2100 Copenhagen (DK)**
- **Pickles, James**
  **2100 Copenhagen (DK)**

(74) Representative: **Gill Jennings & Every LLP**
**The Broadgate Tower**
**20 Primrose Street**
**London EC2A 2ES (GB)**

Remarks:
The complete document including Reference Table(s) and the Sequence Listing(s) can be downloaded from the EPO website

(54) **MODULATORS OF SORTILIN ACTIVITY**

(57) The present invention relates to compounds of Formula (I) or a pharmaceutically acceptable salt, solvate, hydrate, tautomer, optical isomer, N-oxide, and/or prodrug thereof. The present invention also relates to pharmaceutical compositions comprising the compounds of the invention, and to their use in the treatment or prevention of medical conditions in which modulation of sortilin is beneficial.

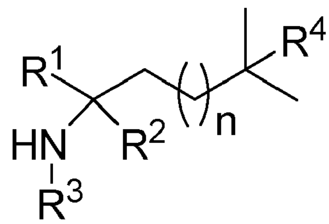

(I)

EP 4 428 121 A1

## Description

## FIELD OF THE INVENTION

The invention relates to compounds of Formula (I), which have surprisingly been found to modulate the activity of sortilin. The invention also relates to pharmaceutical compositions comprising these compounds, and to their use in the treatment or prevention of medical conditions in which modulation of sortilin activity is beneficial. In particular, the compounds of the invention may cross the blood brain barrier and may therefore be particularly useful in the treatment of diseases of the central nervous system.

## BACKGROUND

Sortilin is a Type I transmembrane protein that acts as a receptor of several ligands (Petersen et al., 1997). Sortilin is abundantly expressed in neurons and microglia of the nervous system, the inner ear, and in some peripheral tissues involved in metabolic control (Tauris et al., 2020; Goettsch et al., 2017; Willnow et al., 2011; Kjolby et al., 2010). Besides acting as a receptor involved in signaling, sortilin mediates sorting of select cargo between the cell surface trans-Golgi network, and endosomal pathway (Nykjaer & Willnow, 2012; Willnow, Petersen, & Nykjaer, 2008). Sortilin harbors a large extracellular domain denoted VPS10 that defines the family of receptors named sortilins or VS10p domain receptors. The VPS10P domain in sortilin is homologous to yeast VPS10P and is made up by a 10-bladed beta-propeller structure and a cysteine-rich 10CC module (Nykjaer & Willnow, 2012; Zheng, Brady, Meng, Mao, & Hu, 2011).

Sortilin binds multiple ligands, including pro-nerve growth factor (pro-NGF), pro-BDNF, pro-neurotrophin-3, neurotensin, and ApoB (Chen et al., 2005; Kjolby et al., 2010; Mazella et al., 1998; Nykjaer et al., 2004; Quistgaard et al., 2009; Yano, Torkin, Martin, Chao, & Teng, 2009). Furthermore, Sortilin binds progranulin (PGRN), a secreted protein involved in many cellular functions including securing lysosomal processes, anti-inflammatory responses, and neurotrophic stimulation (Galimberti, Fenoglio, & Scarpini, 2018). Sortilin targets PGRN for rapid endocytosis and degradation, and it is now well established that sortilin is the most important clearance receptor for PGRN (Hu et al., 2010). Thus, sortilin negatively regulates the extracellular levels of PGRN in the periphery as well as in the brain. Indeed, lack of or blocking the receptor increases plasma PGRN levels both in mice and humans (Carrasquillo et al., 2010; Gass, Prudencio, Stetler, & Petrucelli, 2012; Hu et al., 2010; Lee et al., 2014; Miyakawa et al., 2020; Pottier et al., 2018).

Frontotemporal dementia is a highly heritable dementia and haploinsufficiency of the PGRN gene accounts for up to 25% of all cases (Gijselinck, Van Broeckhoven, & Cruts, 2008). Patients with heterozygous loss-of-function mutations in PGRN have >50% reduced extracellular levels of the protein and will invariably develop FTD, making PGRN a causal gene for the disease (Baker et al., 2006; Carecchio et al., 2011; Cruts & Van Broeckhoven, 2008; Galimberti et al., 2010). In addition, PGRN mutant alleles have been identified in Alzheimer's (AD) patients (Brouwers et al., 2008; Sheng, Su, Xu, & Chen, 2014) and high levels of extracellular PGRN are protective in models of ALS, Parkinson's disease, stroke, arthritis, and atherosclerosis (Egashira et al., 2013; Laird et al., 2010; Martens et al., 2012; Tang et al., 2011; Tao, Ji, Wang, Liu, & Zhu, 2012; Van Kampen, Baranowski, & Kay, 2014).

Sortilin is, however, not required for PGRN to elicit its functions. Hence, neurons devoid in sortilin expression are equally responsive to PGRN-induced neuronal outgrowth (De Muynck et al., 2013; Gass, Lee, et al., 2012). Further, PGRN is successfully delivered to neuronal lysosomes in sortilin-deficient cells, suggesting the existence of alternative trafficking pathways. Indeed, PGRN can bind to the lysosomal protein, prosaposin (PSAP). When PSAP binds to its cognate receptors, the cation-independent mannose-6-phosphate receptor and LRP1, it brings along PGRN to the lysosomes (Zhou et al., 2015). Finally, in a phase II clinical trial with a monoclonal anti-sortilin antibody, markers for lysosomal integrity were normal (NCT03987295).

The functional PGRN receptor remains to be identified. However, studies suggest that PGRN promotes neuronal survival, reduces inflammation and increases Aβ endocytosis by microglia (Martens et al., 2012; Pickford et al., 2011; Yin et al., 2010).

Binding of PGRN to sortilin requires the three amino acids in the C-terminal of PGRN (QLL in human, PLL in mouse), and a peptide derived from the last 24 amino acids of PGRN binds with similar affinity as the full-length protein (Zheng et al., 2011). It was proposed that this mode of binding is structurally similar to Neurotensin binding (Zheng et al., 2011), i.e. binding in the NTIS1 binding site of sortilin. There has been a successful small molecule screen that identified a blocker of Neurotensin to sortilin binding done in collaboration with Aarhus University (Andersen et al., 2014; Schroder et al., 2014).

Sortilin exists as a full-length and sorting competent receptor but is also capable of forming multimeric signalling receptor-ligand. Portions of sortilin can also be liberated from the plasma membrane to scavenge ligands (NT in pain) and control the activity of ligands. For example, sortilin is involved in synaptic plasticity by controlling the conversion rate of pro-BDNF into BDNF. This may also apply to other proneurotrophins.

Finally, the propeptide of sortilin, also named spadin, that is a ligand of the receptor has been demonstrated

to control the activity of the membrane transporter TREK-1, which is a target for major depression, among other diseases. Structurally, sortilin has an amino acid sequence according to SEQ ID NO: 1 and comprises a signal peptide, a propeptide, the Vps10p domain, a 10cc domain (10CCa + 10CCb), a transmembrane domain and a cytoplasmic tail. The luminal domain of sortilin has 6 potential *N*-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (pro-NGF), brain derived neurotrophic factor (pro-BDNF), and neurotrophin-3 (proNT3), respectively. In complex with the protein p75NTR (p75 neurotrophin receptor), sortilin has been reported to form the receptor for proneurotrophin-mediated apoptotic effects leading to degeneration and cell death in cellular and animal models (Jansen et al., 2007; Tenk et al., 2005; Nykjaer et al., 2004).

Previous work has suggested a role for sortilin in cellular sorting and signalling associated with diseases such as diabetes and obesity (Huang et al., 2013). Sortilin facilitates translocation of GLUT4 to the plasma membrane and rescues it from degradation in the lysosomes (Pan et al., 2017). Sortilin levels have been shown to be modulated by the level of inflammation associated with these diseases. The pro-inflammatory cytokine, TNF$\alpha$, reduces both mRNA levels and protein levels of sortilin in cultured mouse and human adipocytes, as well as *in vivo* when injected into mice (Kaddai et al., 2009). Sortilin can also influence cytokine secretion: targeting sortilin in immune cells has been proposed to attenuate inflammation and reduce atherosclerosis disease progression (Mortensen et al., 2014). Additionally, US 2016/0331746 describes various scaffolds of small molecules capable of binding to the active site of sortilin. Sortilin is involved in the regulation of glucose uptake (Shi & Kandror. 2005) and the development of lipid disorder diseases (Gao et al., 2017).

Further, plasma sortilin levels have been reported to be a potential biomarker for identifying patients with either coronary heart disease or diabetes mellitus (Oh et al., 2017; Møller et al., 2021). Patients that showed increased sortilin levels within their plasma, and therefore identifiable as suffering from the above conditions, also displayed enhanced glucose levels suggesting sortilin as a therapeutic target for treating these conditions. Soluble sortilin is also proposed as a treatment for type II diabetes (WO2021116290 A1, 2021).

TAR DNA-binding protein 43 (TDP-43) has been implicated in various neurodegenerative diseases. For example, TDP-43 inclusion bodies have been found in cases of amyotrophic lateral sclerosis (ALS), frontotemporal lobar degeneration (FTLD), and Alzheimer's disease (AD) (Meneses et al., 2021).

TDP-43 regulates the splicing of several gene products, including Sortilin. In humans, the splicing involves inclusion of a cryptic exon 17b (between exon 17 and 18), which introduces a stopcodon in the stalk, potentially generating a non-membrane bound fragment (Prudencio et al., 2012). Furthermore, it has been shown that PGRN can reduce levels of insoluble TDP-43 and slow down axonal degeneration (Beel et al., 2018). Sortilin inhibition increases PGRN levels and is therefore beneficial in the treatment of neurodegenerative diseases in which TDP-43 is implicated.

Sortilin has been linked to various conditions affecting the central nervous system (CNS). Some studies suggest a role of circulating sortilin in patients with psychiatric disorders such as depression, which may relate to altered activity of neurotrophic factors (Buttenshon et al., 2015); and it has also been reported that sortilin plays a role in brain aging, Alzheimer's Disease and frontotemporal dementia (Xu et al., 2019). However, delivering therapeutic agents that are capable of crossing the blood-brain barrier to the CNS presents a major challenge.

The blood-brain barrier is a highly selective semipermeable border of endothelial cells that prevents solutes in the circulating blood from non-selectively crossing into the extracellular fluid of the CNS where neurons reside. Therapy of neurological diseases is therefore limited due to the restricted penetration of therapeutic agents across the blood-brain barrier.

Therapeutic agents for treating CNS diseases therefore must be capable of crossing the blood-brain barrier. In addition to this, they must also have a sufficient unbound drug concentration in the brain, as the free drug hypothesis states that only unbound compound is able to interact with and elicit a pharmacological effect.

To determine the unbound fraction of a test compound ($F_{ub}$), sample supernatants may be analysed by methods such as liquid chromatography with tandem mass spectrometry (LC-MS/MS). The unbound fraction may then be calculated from the peak area ratios obtained for each matrix according to the following formula:

$$F_{ub} = C_{PBS} / C_{plasma}$$

where $C_{PBS}$ and $C_{plasma}$ are the analyte concentrations in PBS (receiver) and plasma (donor), respectively.

Recovery samples may be prepared in each condition but without dialysis, and may be used for evaluation of recovery from dialysis experiments using the following formula:

$$\% \text{ Recovery} = 100\times(V_{PBS}\times C_{PBS}+V_{plasma}\times C_{plasma})/V_{plasma}\times C_{recovery}$$

where $V_{PBS}$ is the volume on the receiver side (PBS) and $V_{plasma}$ is the volume on donor side (plasma) of the dialysis device. $C_{recovery}$ is the analyte concentration measured from the recovery sample. Compounds such as propranolol or fluoxetine, may be included in experiments as controls.

The unbound fraction in brain ($F_{ub, brain}$) may be calculated from the measured value in brain homogenate ($F_{ub, meas}$), taking into account the dilution factor used in preparing the brain homogenate:

$$F_{ub,\,brain} = \frac{1/D}{\left(\frac{1}{F_{ub,\,meas}}\right) - 1 + (\frac{1}{D})}$$

where D = dilution factor.

The brain/plasma unbound partition coefficient ($K_{puu}$) may be determined as a ratio between the free compound concentrations in plasma and brain:

$$K_{puu} = \frac{C_{ub,brain}}{C_{ub,plasma}}$$

Where $C_{u,brain}$ = unbound concentration in brain ($C \times F_{ub, brain}$); wherein
C = concentration at steady state; and
$C_{ub,plasma}$ = unbound concentration in plasma ($C \times F_{ub}$).

Alternatively a ratio of AUCs can be used to determine $K_{puu}$. The compound of interest being dosed to a relevant species of interest at a known concentration by oral or intravenous route. Timecourses of concentration of compound in the plasma and cerebral spinal fluid (CSF) are measured. The plasma concentration is corrected to account for unbound fraction of compound. Areas under the CSF concentration curve and free fraction in plasma concentration curve are calculated by known methods and a ratio is determined to give:

$$K_{puu} = \text{AUC0-infcsf} / (\text{AUC0-infplasma} \times (\%\text{Fuplasma}/100))$$

For the treatment of CNS diseases, it is desirable for the $K_{puu}$ to have the highest possible value, above 0. A value around 1 indicating that the free fraction compound freely permeates the blood brain barrier; a value above 1 suggesting that an active influx transport mechanism at the blood brain barrier is involved; and less than 1, which indicates that the free fraction compound is either poorly permeable or is recognized by an active efflux mechanism, reducing the exposure in the CNS while passing back through the blood-brain barrier to plasma or the CSF. A $K_{puu}$ value of 0 or close to 0, indicates a poorly permeable compound or a highly active efflux mechanism that in any case will make highly improbable to reach a meaningful exposure in the CNS of the desired active species.

In view of the above, there is an unmet need for compounds that may be used in the treatment and prevention of medical conditions in which modulation of sortilin is beneficial. In particular, there is an unmet need for sortilin modulators that are capable of crossing the blood brain barrier and are therefore useful in the treatment of diseases of the central nervous system.

## BRIEF DESCRIPTION OF THE FIGURES

Figures 1 to 6 depict x-ray derived images of Example 8 bound to h-Sortilin.

Figures 7A to 7C depict electron density maps from the x-ray diffraction data for Example 8.

## DISCLOSURE OF THE INVENTION

Surprisingly, it has been found that compounds of Formula (I) modulate the activity of sortilin and are therefore

useful in the treatment or prevention of conditions in which modulation of sortilin is beneficial. Furthermore, the compounds may cross the blood brain barrier and may therefore be particularly useful in the treatment of diseases of the central nervous system.

In a first aspect of the invention, there is provided a compound of Formula (I):

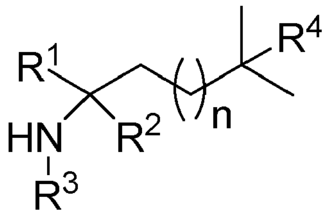

(I)

or a pharmaceutically acceptable salt, solvate, hydrate, tautomer, optical isomer, N-oxide, and/or prodrug thereof; wherein

$R^1$ is 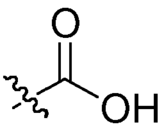 or 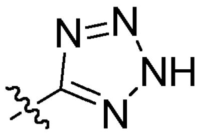 ;

$R^2$ is H or $-CH_3$;

$R^3$ is selected from the group consisting of $C_6$-$C_{10}$ aryl and 5- to 10-membered heteroaryl, optionally substituted with one or more substituents independently selected from -OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, $C_1$-$C_4$ hydroxyalkoxy, acetyl, cyano, $C_6$-$C_{10}$ aryl, 5- to 10-membered heteroaryl, 5- to 10-membered heterocycloalkyl, -O-($C_6$-$C_{10}$ aryl), $-O-CH_2-$($C_6$-$C_{10}$ aryl), and $-NR^5R^6$;

$R^4$ is H or $-CH_3$;

$R^5$ and $R^6$ are each independently H or$C_1$-$C_4$ alkyl; and

n is 0 or 1.

Surprisingly, the applicant has found that when $R^1$ is $-CO_2H$, $R^4$ is $-CH_3$, and n is 1, the compound of Formula (I) exhibits improved binding to sortilin. In a preferred aspect of the invention, the compound of Formula (I) therefore has the following formula:

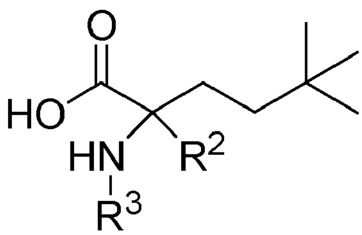

In the compounds of the invention, it is preferred that $R^2$ is H.

In a preferred aspect of the invention, $R^3$ is selected from the group consisting of phenyl, naphthyl, 5- or 6-membered monocyclic heteroaryl, and 9- or 10-membered fused bicyclic heteroaryl, preferably phenyl, naphthyl, 6-membered monocyclic heteroaryl, and 9- or 10-membered fused bicyclic heteroaryl, more preferably 10-membered fused bicyclic heteroaryl, wherein each group is optionally substituted as defined above.

As used herein, the term "5- or 6-membered monocyclic heteroaryl" refers to an aromatic ring having 5 or 6 ring atoms, wherein at least one ring atom is a heteroatom and the remaining ring atoms are carbon.

As used herein, the term "9- or 10-membered fused bicyclic heteroaryl" refers to an aromatic ring system

containing two rings fused together such that they share two adjacent ring atoms. Preferably, the 9- or 10-membered fused bicyclic heteroaryl group contains a 6-membered ring fused to a 5- or 6-membered ring.

The 9- or 10-membered fused bicyclic heteroaryl group has 9 or 10 ring atoms, wherein at least one ring atom is a heteroatom and the remaining ring atoms are carbon.

Preferably, each ring atom in the 5- or 6-membered monocyclic heteroaryl group and the 9- or 10-membered fused bicyclic heteroaryl group of $R^3$ is independently C or N. More preferably, one to three ring atoms are N and the remaining ring atoms are C.

More preferably, one or two ring atoms in the 5- or 6-membered monocyclic heteroaryl group of $R^3$ are N and the remaining ring atoms are C.

Examples of the 6-membered monocyclic heteroaryl group of $R^3$ include pyridyl, pyrimidinyl, pyrazinyl, pyridazinyl, and triazinyl, preferably pyridyl, pyrimidinyl, and pyrazinyl. More preferably, the 6-membered monocyclic heteroaryl group is one of the following groups:

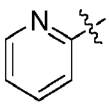, 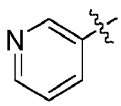, 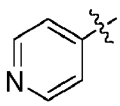, 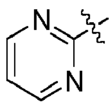, 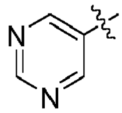, 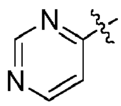, and 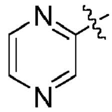,

wherein each group is optionally substituted.

Examples of the 9- or 10-membered fused bicyclic heteroaryl group of $R^3$ include indolyl, indazolyl, benzimidazolyl, benzotriazolyl, azaindolyl, azaindazolyl, pyrazolopyrimidinyl, quinolinyl, isoquinolinyl, quinoxalinyl, phthalazinyl, quinazolinyl, cinnolinyl, naphthyridinyl, pyridopyrimidinyl, and pyridopyrazinyl, preferably azaindolyl, pyridopyrimidinyl, quinolinyl, isoquinolinyl, quinoxalinyl, and quinazolinyl. More preferably, the 9- or 10-membered fused bicyclic heteroaryl group is one of the following groups:

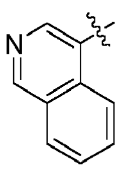, 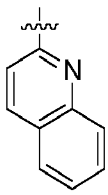, 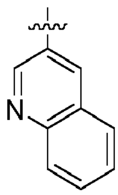, 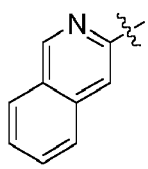, 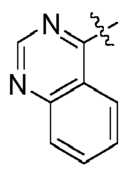,

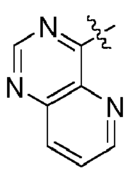, 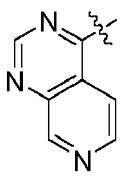, 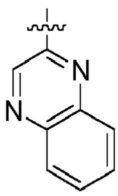, and 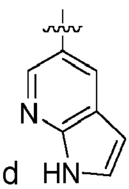,

wherein each group is optionally substituted.

More preferably, the 9- or 10-membered fused bicyclic heteroaryl group of $R^3$ is

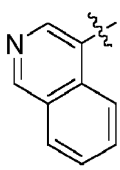 or 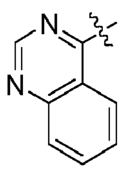,

wherein each group is optionally substituted. The applicant has surprisingly found that these groups improve the binding of the compound to sortilin.

Any of the $R^3$ groups in the compounds of Formula (I) may be optionally substituted with one or more substituents as defined above. Preferably, the optional substituents for $R^3$ are independently selected from the group consisting of halo, cyano, $C_1$-$C_4$ alkyl, $C_1$-$C_4$ alkoxy, $C_1$-$C_4$ haloalkyl, phenyl, 6-membered heterocycloalkyl, -O-phenyl, and -O-$CH_2$-phenyl.

More preferably, the optional substituents for $R^3$ are independently selected from the group consisting of halo,

cyano, $C_1$-$C_2$ alkyl, $C_1$-$C_2$ alkoxy, $C_1$-$C_2$ haloalkyl, phenyl, morpholinyl, -O-phenyl, and -O-$CH_2$-phenyl.

In a preferred aspect of the invention, $R^3$ is selected from the group consisting of:

(i) phenyl and 6-membered monocyclic heteroaryl, optionally substituted with one or more substituents independently selected from the group consisting of halo, $C_1$-$C_2$ alkyl, $C_1$-$C_2$ alkoxy, phenyl, -O-phenyl, -O-$CH_2$-phenyl, and morpholinyl; and

(ii) naphthyl and 9- or 10-membered fused bicyclic heteroaryl, optionally substituted with one or more substituents independently selected from the group consisting of halo, cyano, $C_1$-$C_2$ alkyl, $C_1$-$C_2$ alkoxy, $C_1$-$C_2$ haloalkyl, and phenyl.

In a more preferred aspect of the invention, $R^3$ is selected from the group consisting of:

(i) phenyl optionally substituted with one or more substituents independently selected from the group consisting of halo and -O-phenyl;

(ii) 6-membered monocyclic heteroaryl optionally substituted with one or more substituents independently selected from the group consisting of halo, $C_1$-$C_2$ alkyl, $C_1$-$C_2$ alkoxy, phenyl, -O-phenyl, -O-$CH_2$-phenyl, and morpholinyl;

(iii) naphthyl optionally substituted with one or more halo atoms; and

(iv) 9- or 10-membered fused bicyclic heteroaryl optionally substituted with one or more substituents independently selected from the group consisting of halo, cyano, $C_1$-$C_2$ alkyl, $C_1$-$C_2$ alkoxy, $C_1$-$C_2$ haloalkyl, and phenyl.

In a more preferred aspect of the invention, $R^3$ is selected from the group consisting of:

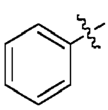, 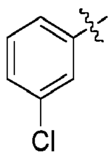, 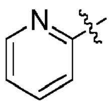, 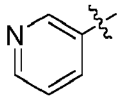, 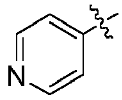, 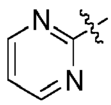, 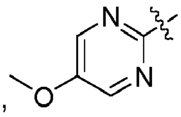, 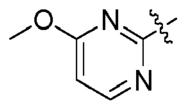,

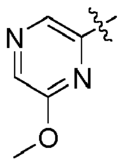, 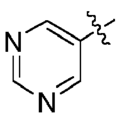, 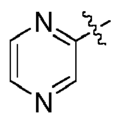, 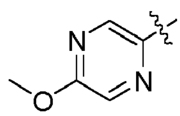, 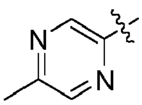, 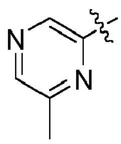, 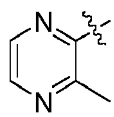, 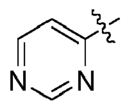,

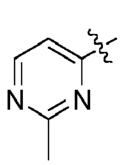, 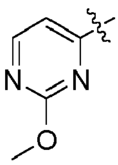, 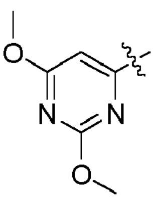, 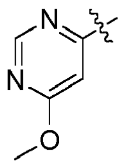, 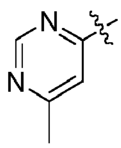, 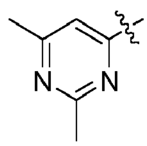, 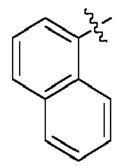, 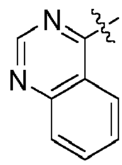,

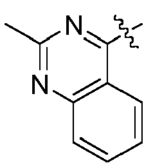, 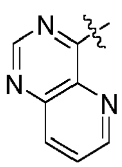, 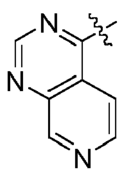, 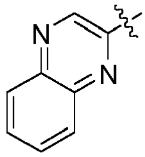, 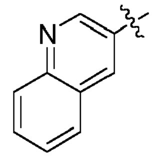, 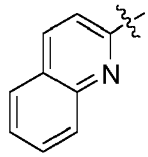, 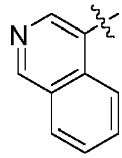,

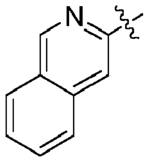, 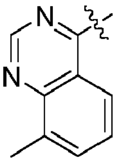, 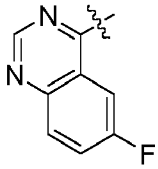, 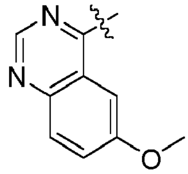, 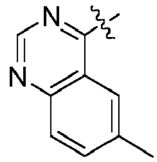, 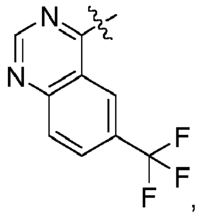,

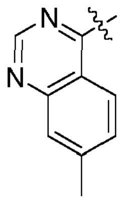, 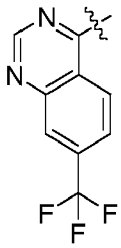, 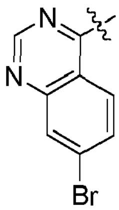, 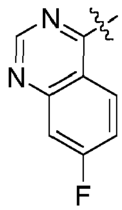, 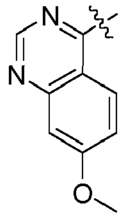, 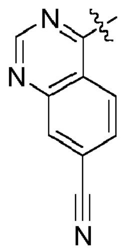, 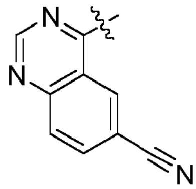,

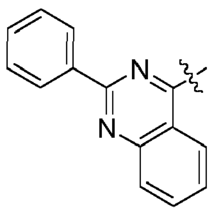, 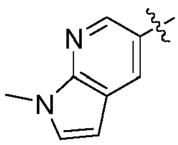, 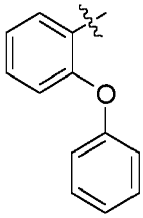, 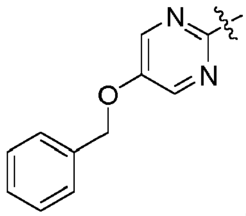, 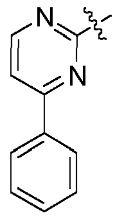, 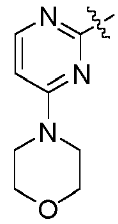,

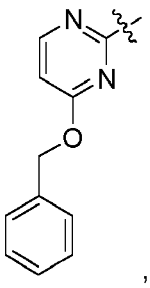, 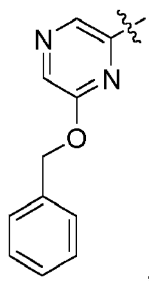, 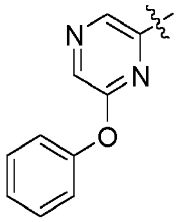, and 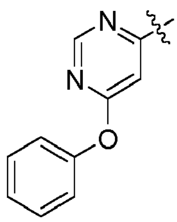.

Particular compounds of the first aspect of the invention are those listed below.

(S)-2-anilino-5,5-dimethylhexanoic acid;
(S)-5,5-dimethyl-2-(2-pyridylamino)hexanoic acid;
(S)-5,5-dimethyl-2-(4-pyridylamino)hexanoic acid;
(S)-5,5-dimethyl-2-(2-pyrazinylamino)hexanoic acid;
(S)-5,5-dimethyl-2-(2-pyrimidinylamino)hexanoic acid;
(S)-5,5-dimethyl-2-(5-pyrimidinylamino)hexanoic acid;
(S)-5,5-dimethyl-2-(4-pyrimidinylamino)hexanoic acid;
(S)-5,5-dimethyl-2-(4-quinazolinylamino)hexanoic acid;
(S)-5,5-dimethyl-2-(1,3,5-triaza-4-naphthylamino)hexanoic acid;
(S)-2-[5-(benzyloxy)-2-pyrimidinylamino]-5,5-dimethylhexanoic acid
(S)-2-(5-methoxy-2-pyrimidinylamino)-5,5-dimethylhexanoic acid
(S)-2-(4-methoxy-2-pyrimidinylamino)-5,5-dimethylhexanoic acid
(S)-5,5-dimethyl-2-(4-morpholino-2-pyrimidinylamino)hexanoic acid
(S)-5,5-dimethyl-2-(2-quinoxalinylamino)hexanoic acid
(S)-5,5-dimethyl-2-(5-methyl-2-pyrazinylamino)hexanoic acid
(S)-5,5-dimethyl-2-(3-methyl-2-pyrazinylamino)hexanoic acid
(S)-2-(2-methoxy-4-pyrimidinylamino)-5,5-dimethylhexanoic acid
(S)-2-(6-methoxy-4-pyrimidinylamino)-5,5-dimethylhexanoic acid

(S)-5,5-dimethyl-2-(2-methyl-4-pyrimidinylamino)hexanoic acid
(S)-5,5-dimethyl-2-(6-methyl-4-pyrimidinylamino)hexanoic acid
(S)-2-(2,6-dimethyl-4-pyrimidinylamino)-5,5-dimethylhexanoic acid
(S)-5,5-dimethyl-2-(8-methyl-4-quinazolinylamino)hexanoic acid
(S)-5,5-dimethyl-2-(6-methyl-2-pyrazinylamino)hexanoic acid
(S)-5,5-dimethyl-2-(6-phenoxy-2-pyrazinylamino)hexanoic acid
(S)-2-(4-isoquinolylamino)-5,5-dimethylhexanoic acid
(S)-2-(m-chlorophenylamino)-5,5-dimethylhexanoic acid
(S)-5,5-dimethyl-2-(3-pyridylamino)hexanoic acid
[4,4-dimethyl-1-(2H-tetraazol-5-yl)pentyl]-4-isoquinolylamine
[4,4-dimethyl-1-(2H-tetraazol-5-yl)pentyl]-3-quinolylamine;
[4,4-dimethyl-1-(2H-tetraazol-5-yl)pentyl]-2-quinolylamine;
[4,4-dimethyl-1-(2H-tetraazol-5-yl)pentyl]-1-naphthylamine;
N-phenyl[4-methyl-1-(2H-tetraazol-5-yl)pentyl]amine;
N-2-pyridyl[4-methyl-1-(2H-tetraazol-5-yl)pentyl]amine;
[4-methyl-1-(2H-tetraazol-5-yl)pentyl]-1-naphthylamine;
N-phenyl[4,4-dimethyl-1-(2H-tetraazol-5-yl)pentyl]amine;
[3-methyl-1-(2H-tetraazol-5-yl)butyl]-2-quinolylamine;
[3-methyl-1-(2H-tetraazol-5-yl)butyl]-2-pyrazinylamine;
[3-methyl-1-(2H-tetraazol-5-yl)butyl]-3-isoquinolylamine;
N-phenyl[3,3-dimethyl-1-(2H-tetraazol-5-yl)butyl]amine;
[3,3-dimethyl-1-(2H-tetraazol-5-yl)butyl]-3-quinolylamine;
N-2-pyrimidinyl[3,3-dimethyl-1-(2H-tetraazol-5-yl)butyl]amine;
[3-methyl-1-(2H-tetraazol-5-yl)butyl]-4-isoquinolylamine;
N-2-pyrimidinyl[4,4-dimethyl-1-(2H-tetraazol-5-yl)pentyl]amine;
[3,3-dimethyl-1-(2H-tetraazol-5-yl)butyl]-4-isoquinolylamine;
[3-methyl-1-(2H-tetraazol-5-yl)butyl]-5-pyrimidinylamine;
N-5-pyrimidinyl[3,3-dimethyl-1-(2H-tetraazol-5-yl)butyl]amine;
N-2-pyrazinyl[3,3-dimethyl-1-(2H-tetraazol-5-yl)butyl]amine;
[3,3-dimethyl-1-(2H-tetraazol-5-yl)butyl](1-methyl-1H-1,7-diazainden-5-yl)amine;
[4,4-dimethyl-1-(2H-tetraazol-5-yl)pentyl](1-methyl-1H-1,7-diazainden-5-yl)amine;
[3,3-dimethyl-1-(2H-tetraazol-5-yl)butyl]-2-quinolylamine;
[4-methyl-1-(2H-tetraazol-5-yl)pentyl]-3-quinolylamine;
[3-methyl-1-(2H-tetraazol-5-yl)butyl]-3-quinolylamine;
N-2-pyrimidinyl[4-methyl-1-(2H-tetraazol-5-yl)pentyl]amine;
[4-methyl-1-(2H-tetraazol-5-yl)pentyl]-2-quinolylamine;
N-3-pyridyl[4-methyl-1-(2H-tetraazol-5-yl)pentyl]amine;
[3,3-dimethyl-1-(2H-tetraazol-5-yl)butyl]-3-isoquinolylamine;
N-2-pyridyl[3,3-dimethyl-1-(2H-tetraazol-5-yl)butyl]amine;
N-3-pyridyl[3,3-dimethyl-1-(2H-tetraazol-5-yl)butyl]amine;
[3-methyl-1-(2H-tetraazol-5-yl)butyl]-3-pyridylamine;
[4-methyl-1-(2H-tetraazol-5-yl)pentyl]-4-isoquinolylamine;
[3-methyl-1-(2H-tetraazol-5-yl)butyl]-2-pyrimidinylamine;
[3-methyl-1-(2H-tetraazol-5-yl)butyl]-2-pyridylamine;
[3-methyl-1-(2H-tetraazol-5-yl)butyl]aniline;
N-2-pyridyl[4,4-dimethyl-1-(2H-tetraazol-5-yl)pentyl]amine;
N-3-pyridyl[4,4-dimethyl-1-(2H-tetraazol-5-yl)pentyl]amine;
[4,4-dimethyl-1-(2H-tetraazol-5-yl)pentyl](6-fluoro-4-quinazolinyl)amine;
[(R)-4,4-dimethyl-1-(2H-tetraazol-5-yl)pentyl](6-fluoro-4-quinazolinyl)amine;
[(S)-4,4-dimethyl-1-(2H-tetraazol-5-yl)pentyl](6-fluoro-4-quinazolinyl)amine;
[3,3-dimethyl-1-(2H-tetraazol-5-yl)butyl]-4-quinazolinylamine;
[4,4-dimethyl-1-(2H-tetraazol-5-yl)pentyl](6-methoxy-4-quinazolinyl)amine;
[4-methyl-1-(2H-tetraazol-5-yl)pentyl](6-methoxy-4-quinazolinyl)amine;
[4-methyl-1-(2H-tetraazol-5-yl)pentyl](6-methyl-4-quinazolinyl)amine;
[4-methyl-1-(2H-tetraazol-5-yl)pentyl](6-fluoro-4-quinazolinyl)amine;
[4,4-dimethyl-1-(2H-tetraazol-5-yl)pentyl](7-fluoro-4-quinazolinyl)amine;
[4,4-dimethyl-1-(2H-tetraazol-5-yl)pentyl](6-methyl-4-quinazolinyl)amine;
[4,4-dimethyl-1-(2H-tetraazol-5-yl)pentyl](7-methyl-4-quinazolinyl)amine;

[4,4-dimethyl-1-(2H-tetraazol-5-yl)pentyl](7-methoxy-4-quinazolinyl)amine;
[4,4-dimethyl-1-(2H-tetraazol-5-yl)pentyl][6-(trifluoromethyl)-4-quinazolinyl]amine;
[4,4-dimethyl-1-(2H-tetraazol-5-yl)pentyl][7-(trifluoromethyl)-4-quinazolinyl]amine;
[4,4-dimethyl-1-(2H-tetraazol-5-yl)pentyl]-1,3,7-triaza-4-naphthylamine;
[4,4-dimethyl-1-(2H-tetraazol-5-yl)pentyl](2-methyl-4-quinazolinyl)amine;
[4,4-dimethyl-1-(2H-tetraazol-5-yl)pentyl](7-bromo-4-quinazolinyl)amine;
[4-methyl-1-(2H-tetraazol-5-yl)pentyl](7-methoxy-4-quinazolinyl)amine;
[4,4-dimethyl-1-(2H-tetraazol-5-yl)pentyl](2-phenyl-4-quinazolinyl)amine;
[(S)-4,4-dimethyl-1-(2H-tetraazol-5-yl)pentyl]-4-isoquinolylamine;
[(R)-4,4-dimethyl-1-(2H-tetraazol-5-yl)pentyl]-4-isoquinolylamine;
[(R)-4,4-dimethyl-1-(2H-tetraazol-5-yl)pentyl]-2-quinolylamine;
[(S)-4,4-dimethyl-1-(2H-tetraazol-5-yl)pentyl]-2-quinolylamine;
[(S)-4,4-dimethyl-1-(2H-tetraazol-5-yl)pentyl]-4-quinazolinylamine;
[(R)-4,4-dimethyl-1-(2H-tetraazol-5-yl)pentyl]-4-quinazolinylamine;
N-3-pyridyl[(S)-4,4-dimethyl-1-(2H-tetraazol-5-yl)pentyl]amine;
N-3-pyridyl[(R)-4,4-dimethyl-1-(2H-tetraazol-5-yl)pentyl]amine;
[4-methyl-1-(2H-tetraazol-5-yl)pentyl]-4-quinazolinylamine;
[4,4-dimethyl-1-(2H-tetraazol-5-yl)pentyl]-4-quinazolinylamine;
[3-methyl-1-(2H-tetraazol-5-yl)butyl]-4-quinazolinylamine;
[3-methyl-1-(2H-tetraazol-5-yl)butyl]-4-pyridylamine;
[3-methyl-1-(2H-tetraazol-5-yl)butyl]-4-pyrimidinylamine;
4-[4,4-dimethyl-1-(2H-tetraazol-5-yl)pentylamino]-7-quinazolinecarbonitrile;
4-[4,4-dimethyl-1-(2H-tetraazol-5-yl)pentylamino]-6-quinazolinecarbonitrile;
(S)-5,5-dimethyl-2-(o-phenoxyphenylamino)hexanoic acid;
(S)-2-anilino-4,4-dimethylvaleric acid;
(S)-2-anilino-2,5,5-trimethylhexanoic acid;
(S)-5,5-dimethyl-2-(4-phenyl-2-pyrimidinylamino)hexanoic acid;
(S)-2-[6-(benzyloxy)-2-pyrazinylamino]-5,5-dimethylhexanoic acid;
(S)-2-(6-methoxy-2-pyrazinylamino)-5,5-dimethylhexanoic acid;
(S)-2-(5-methoxy-2-pyrazinylamino)-5,5-dimethylhexanoic acid;
(S)-2-[4-(benzyloxy)-2-pyrimidinylamino]-5,5-dimethylhexanoic acid;
(S)-5,5-dimethyl-2-(6-phenoxy-4-pyrimidinylamino)hexanoic acid;
(S)-2-(2,6-dimethoxy-4-pyrimidinylamino)-5,5-dimethylhexanoic acid;
or a pharmaceutically acceptable salt, solvate, hydrate, tautomer, optical isomer, N-oxide, and/or prodrug thereof.

According to a second aspect of the invention, there is a pharmaceutical composition comprising a compound according to the invention and a pharmaceutically acceptable carrier, excipient, and/or diluent.

According to a third aspect of the invention, there is provided a compound or pharmaceutical composition according to the invention for use in therapy.

According to a fourth aspect of the invention, there is provided a compound or pharmaceutical composition according to the invention for use in the treatment or prevention of a neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular diseases, kidney disease, psoriasis, hereditary eye conditions, chronic pain, hearing loss or diseases characterized by misfolded tau.

Preferably, the neurodegenerative disorder is selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy.

The neurodegenerative disorder is preferably a neurodegenerative disorder characterised by misfolded TAR DNA binding protein 43 (tdp-43). In other words, the neurodegenerative disease is characterized by truncated tdp-43 and inclusion bodies. Examples of such diseases include amyotrophic lateral sclerosis, Alzheimer's disease, Frontotemporal Lobar Degeneration, and frontotemporal dementia.

Preferably, the psychiatric disorder is selected from bipolar disorder, major depression, post-traumatic stress disorder, and anxiety disorders;

Preferably, the inflammatory disorder may be selected from inflammatory diseases and neuroinflammation.

Preferably, the lysosomal storage disorder is selected from the group consisting of NCL/Batten Disease caused by mutations in CLN gene CLN1 (PPT1), CLN2 (TPP1), CLN3, CLN4 (DNAJC5), CLN5, CLN6, CLN7 (MFSD8), CLN8,

CLN10 (CTSD), CLN11, CLN12 (ATP13A2), CLN13 (CTSF), CLN14 (KCTD7), CLCN6, and/or SGSH; Pompe disease, Fabry disease, Gaucher disease, Niemann-Pick disease Types A, B, and C; GM1 gangliosidosis, GM2 gangliosidosis (including Sandhoff and Tay-Sachs), mucopolysachariddoses (MPS) types I (Hurler disease)/II (Hunter disease)/IIIa (Sanfilippo A)/IIIB (Sanfilippo B)/IIIc (Sanfilippo C)/IIId (Sanfilippo D)/IVA (Morqui" A)/'VB/VI/VII (Sly)/IX, mucolipisosis III (I-cell) and IV, multiple sulfatase deficiency; sialidosis, galactosialidosis, α-mannosidosis, β-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Danon disease, Griscelli disease Types ½/3, Hermansky Pudliak Disease, and Chédiak-Higashi syndrome.

Preferably, the cancer is selected from breast cancer, lung cancer, ovarian cancer, prostate cancer, thyroid cancer, pancreatic cancer, glioblastoma and colorectal cancer.

Preferably, the cardiovascular disease is selected from atherosclerosis, cardiomyopathy, heart attack, arrhythmias, heart failure, and ischemic heart disease.

Preferably, the hearing loss is selected from noise-induced hearing loss, ototoxicity induced hearing loss, age-induced hearing loss, idiopathic hearing loss, tinnitus and sudden hearing loss.

According to a sixth aspect of the invention, there is provided the use of the compound according to the invention for the manufacture of a medicament for the treatment or prevention of neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular diseases, kidney disease, psoriasis, hereditary eye conditions, chronic pain, hearing loss or diseases characterized by misfolded tau.

According to a sixth aspect of the invention, there is provided a method for the treatment or prevention of a disease or condition responsive to sortilin modulation comprising administering a therapeutically effective amount of a compound or pharmaceutical composition according to the invention.

The compounds of the invention may include isotopically-labelled and/or isotopically-enriched forms of the compounds. The compounds of the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulfur, chlorine, such as $^{2}H$, $^{3}H$, $^{11}C$, $^{13}C$, $^{14}C$, $^{13}N$, $^{15}O$ $^{17}O$ $^{32}P$, $^{35}S$ , $^{18}F$ , $^{36}Cl$.

The compounds of the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable basic addition salts by treating the acid form with an appropriate base. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, N-oxides, and/or prodrug forms thereof. It is to be understood that the compounds of the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2Hand 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereogenic centres). All stereoisomers, such as enantiomers and diastereomers, are intended unless otherwise indicated. Compounds of the present invention that contain asymmetrically substituted carbon atoms can be isolated in optically active or racemic forms.

Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds of the present invention are described and may be isolated as a mixture of isomers or as separated isomeric forms.

In the case of the compounds which contain an asymmetric carbon atom, the invention relates to the D form, the L form, and D,L mixtures and also, where more than one asymmetric carbon atom is present, to the diastereomeric forms. Those compounds of the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

Preferably, the compounds of Formula (I) are compounds of Formula (Ia):

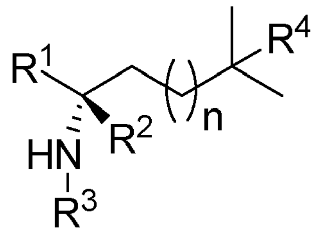

(Ia)

The term "prodrugs" refers to compounds that may be converted under physiological conditions or by solvolysis to a biologically active compound of the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound of the invention may be prepared by modifying functional groups, such as a hydroxy, amino or mercapto groups, present in a compound of the invention in such a way that the modifications are cleaved, either in routine manipulation or in vivo, to the parent compound of the invention. Examples of prodrugs include, but are not limited to, acetate, formate and succinate derivatives of hydroxy functional groups or phenyl carbamate derivatives of amino functional groups.

The compounds of the invention may be sortilin inhibitors, binders, modulators or antagonists. As used herein, the term "sortilin antagonist", "sortilin inhibitor", "sortilin binder" or "sortilin modulator" (used interchangeably) refers to a substance that interferes with, blocks, or otherwise attenuates the effect of, a sortilin protein binding to progranulin, or neurotensin or another extracellular ligand, or a pro-neurotrophin (e.g., pro-NGF, proNT3, pro-BDNF) or preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not pro-NGF) and p75NTR can simultaneously bind the NGF domain of pro-NGF. However, the resulting trimeric complex may be of lower affinity for its receptor and as a result have significantly reduced capacity to stimulate apoptosis via the mechanism described above. Skeldal et al. (2012) demonstrated that the apoptotic function of the trimeric complex is abolished when sortilin is devoid in its intracellular domain. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency. Skeldal *et al* showed that complex formation between sortilin and p75NTR relies on contact points in the extracellular domains of the receptors and that the interaction critically depends on an extracellular juxtamembrane 23-amino acid sequence of p75NTR. Thus, the sortilin antagonist may interfere with this 23-amino acid sequence or proximal sequences in the molecules. "Sortilin antagonists" may act as ligand cellular uptake inhibitors wherein the ligands may be progranulin, neurotensin, BDNF etc.

The compounds of the invention may cross the blood brain barrier and therefore may be particularly useful in the treatment or prevention of diseases of the central nervous system, including neurodegenerative disorders selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury,

spinal cord injury and stroke; a psychiatric disorder selected from bipolar disorder, major depression, post-traumatic stress disorder, and anxiety disorders; hearing loss selected from noise-induced hearing loss, ototoxicity induced hearing loss, age-induced hearing loss, idiopathic hearing loss, tinnitus and sudden hearing loss; brain tumours, retinopathies, glaucoma, neuroinflammation, chronic pain and diseases characterized by misfolded tau.

The compounds of the invention may have a $K_{puu}$ of more than 0.1, such as between 0.1 and 10, between 0.1 and 5, between 0.1 and 3, between 0.1 and 2, between 0.1 and 1, between 0.1 and 0.8, between 0.1 and 0.6, between 0.1 and 0.5, between 0.1 and 0.4, between 0.1 and 0.3, or between 0.1 and 0.2.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabelling/assay techniques, blotting/ chemiluminescence methods, real-time PCR, and the like.

For clinical use, the compounds disclosed herein are formulated into pharmaceutical compositions (or formulations) for various modes of administration. It will be appreciated that compounds of the invention may be administered together with a physiologically acceptable carrier, excipient, and/or diluent (i.e. one, two, or all three of these). The pharmaceutical compositions disclosed herein may be administered by any suitable route, preferably by oral, rectal, nasal, topical (including ophthalmic, buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, Etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow-release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

**DEFINITIONS**

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" are used interchangeably herein. It is understood that sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells, and controlling growth cone retraction of projecting axons (Jansen et al., 2007; Nykjaer et al., 2004; Santos et al., 2012; Skeldal et al., 2012).

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include pro-NGF, pro-BDNF, proNT3 and proNT4. Pro-neurotrophins may also control synaptic plasticity. Whereas mature neurotrophins induce synaptic strength, in their proforms they may weaken synapses.

"Optional" or "optionally" means that the subsequently described event or circumstance may, but need not, occur, and that the description includes instances where the event or circumstance occurs and instances in which it does not.

The term "heteroatom" means O, N, or S.

The term "$(C_1-C_n)$alkyl" denotes a straight, branched, cyclic, or partially cyclic alkyl group having from 1 to n carbon atoms, i.e. 1, 2, 3... or n carbon atoms. For the "$(C_1-C_n)$alkyl" group to comprise a cyclic portion it should be formed of at least three carbon atoms. For parts of the range "$(C_1-C_n)$alkyl" all subgroups thereof are contemplated. For example, in the range $(C_1-C_4)$alkyl, all subgroups such as $(C_1-C_4)$alkyl, $(C_1-C_3)$alkyl, $(C_1-C_2)$alkyl, $(C_1)$alkyl, $(C_2-C_4)$alkyl, $(C_2-C_3)$alkyl, $(C_2)$alkyl, $(C_3-C_4)$alkyl, $(C_3)$alkyl, and $(C_4)$alkyl. Examples of "$C_1-C_4$ alkyl" include methyl, ethyl, n-propyl, isopropyl, cyclopropyl, n-butyl, isobutyl, sec-butyl, t-butyl, cyclobutyl etc.

The term "$(C_1-C_n)$ haloalkyl" denotes a $C_1-C_n$ alkyl as described above substituted with at least one halogen atom, which is preferably, F, Cl, Br and I, more preferably F and Cl, and most preferably F.

The term "$(C_1-C_n)$ hydroxyalkyl" denotes a $C_1-C_n$ alkyl as described above substituted with at least one -OH group.

The term "$(C_1-C_n)$alkoxy" denotes -O-($(C_1-C_n)$alkyl) in which a $(C_1-C_n)$alkyl group is as defined above and is attached to the remainder of the compound through an oxygen atom.

The term "$(C_1-C_n)$ haloalkoxy" denotes a $C_1-C_n$ alkoxy as described above substituted with at least one halogen atom, which is preferably, F, Cl, Br and I, more preferably F and Cl, and most preferably F.

The term "$(C_1-C_n)$ hydroxyalkoxy" denotes a $C_1-C_n$ alkoxy as described above substituted with at least one -OH group.

When a term denotes a range, for instance "1 to 4 carbon atoms" in the definition of $(C_1-C_4)$alkyl, each integer is considered to be disclosed, i.e. 1, 2, 3, and 4.

The term "halo" means a halogen atom, and is preferably, F, Cl, Br and I, more preferably F, C!, and Br.

The term "$C_6-C_{10}$" aryl denotes an aromatic monocyclic or fused bicyclic hydrocarbon ring system comprising 6 to 10 ring atoms.

The term "5- to 10-membered heterocycloalkyl" denotes a non-aromatic ring system having 5 to 10 ring atoms, in which at least one ring atom is a heteroatom and the remaining ring atoms are carbon. Preferably each heteroatom is independently selected from N, S, or O, more preferably N or O. Preferably, no more than 2 ring atoms are a heteroatom.

"An effective amount" refers to an amount of a compound of the invention that confers a therapeutic effect on

the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, intraocular, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds of the invention may be disclosed by the name or chemical structure. If a discrepancy exists between the name of a compound and its associated chemical structure, then the chemical structure prevails.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilise the present invention to its fullest extent. All references and publications cited herein are hereby incorporated by reference in their entirety.

## PREPARATION OF COMPOUNDS OF THE INVENTION

The compounds of the invention can be prepared according to the following General Synthetic Procedures scheme by methods well known and appreciated in the art. Suitable reaction conditions are well known in the art and appropriate substitutions of solvents and co-reagents are within the common general knowledge of the person skilled in the art. Likewise, it will be appreciated by those skilled in the art that synthetic intermediates may be isolated and/or purified by various well-known techniques as needed or desired, and that frequently, it will be possible to use various intermediates directly in subsequent synthetic steps with little or no purification. Furthermore, the skilled person will appreciate that in some circumstances, the orders in which moieties are introduced is not critical. The particular order of steps required to produce the compounds of formula (I) is dependent upon the particular compound being synthesized, the starting compound, and the relative liability of the substituted moieties as is well appreciated by those of ordinary skill in the art. All substituents, unless otherwise indicated, are as previously defined, and all reagents are well known and appreciated in the art.

## GENERAL SYNTHETIC PROCEDURES

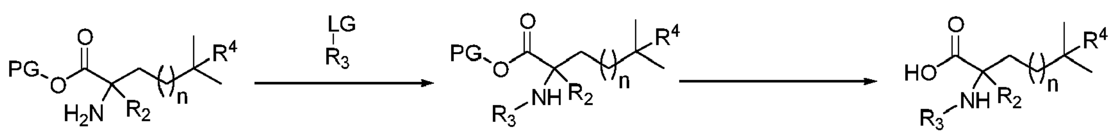

* PG = protecting group; LG=leaving group.

**Scheme 1**

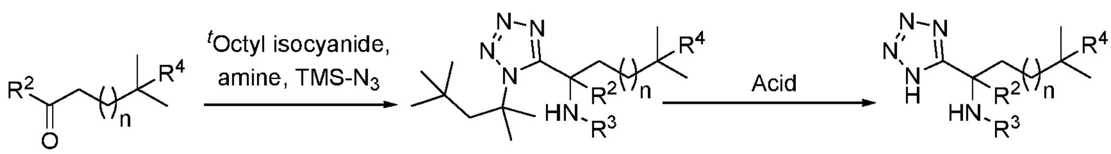


**Scheme 2**

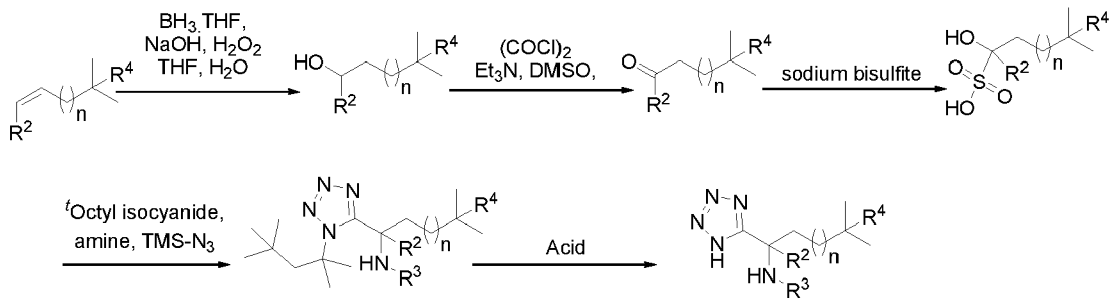


## Scheme 3

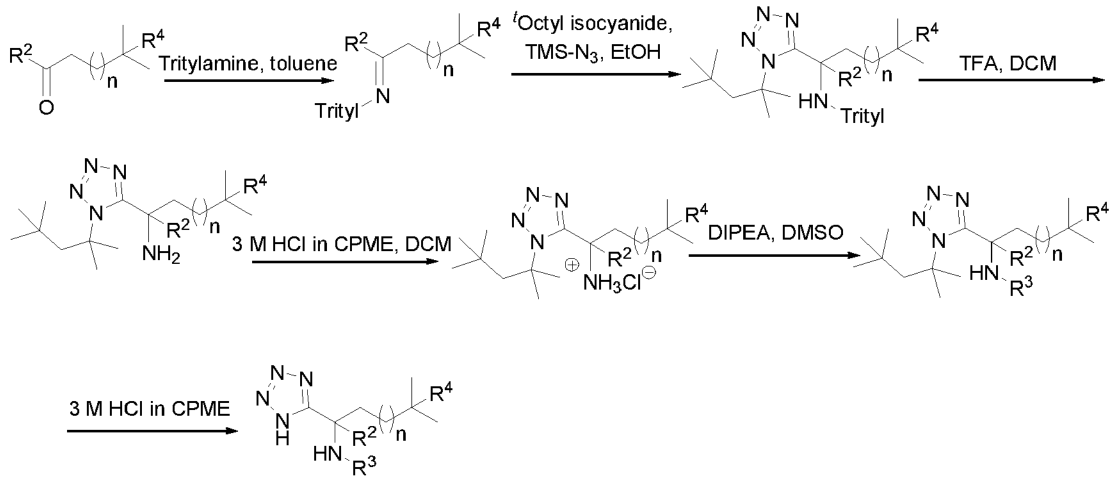


## Scheme 4

The compounds of general formula (I) may be prepared by a variety of procedures, some of which are described below. The products of each step can then be recovered by conventional methods including extraction, evaporation, precipitation, chromatography, filtration, trituration, crystallisation and the like.

Compounds of general formula (I) may contain one or more stereogenic centres. Those can be introduced from available single enantiomers, optically active, starting materials. The integrity of the existing stereogenic centre can be confirmed by analytical techniques well known to those skilled in the art like for example chiral support high pressure chromatography. Alternatively, when racemic starting materials are used, it will be appreciated that if desired, single isomer products can be obtained as single enantiomers or as single diastereoisomers, by known techniques like preparative chiral support high pressure chromatography.

The skilled artisan will also appreciate that not all of the substituents in the compounds of formula (I) will tolerate certain reaction conditions employed to synthesise the compounds. These moieties may be introduced at a convenient point in the synthesis, or may be protected and then deprotected as necessary or desired, as is well known in the art. The skilled artisan will appreciate that the protecting groups may be removed at any convenient point in the synthesis of the compounds of the present invention. Methods for introducing or removing protecting groups used in this invention are well known in the art; see, for example, Greene and Wuts, Protective Groups in Organic Synthesis, 4th Ed., John Wiley and Sons, New York (2006).

## EXAMPLES

### Abbreviations

approx: approximately; aq: aqueous; br: broad; *ca.*: *circa*; CDI: 1,1'-Carbonyldiimidazole; CPME: cyclopentyl methyl ether; d: doublet; DCM: dichloromethane; DIC: N,N'-Diisopropylcarbodiimide; dioxane: 1,4-dioxane; DIPEA: diisopropylethylamine; DMF: dimethylformamide; eq.: equivalent; $Et_3N$: triethylamine; EtOAc: ethyl acetate; EtOH: ethanol; $Et_2O$: diethyl ether; Fmoc: fluorenylmethoxycarbonyl; Boc: tert-butoxycarbonyl; h: hours; min: minutes: HATU:

2-(3*H*-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; MS: mass spectrometry; NMR: nuclear magnetic resonance; PDA: photodiode array; q: quartet; rt: room temperature (ca. 20 °C); $R_T$: retention time; s: singlet, solid; SPPS: solid phase peptide synthesis. t: triplet; TBAF: tetrabutylammonium fluoride; TBME: *tert*-butyl methyl ether; TFA: trifluoroacetic acid; THF: tetrahydrofuran; UPLC: ultra-performance liquid chromatography; UV: ultraviolet.

Other abbreviations are intended to convey their generally accepted meaning.

### General Experimental Conditions

All starting materials and solvents were obtained either from commercial sources or prepared according to the literature citation. Reaction mixtures were magnetically stirred, and reactions performed at room temperature (ca. 20 °C) unless otherwise indicated.

Column chromatography was performed on an automated flash chromatography system, such as a CombiFlash Rf system, using pre-packed silica (40 μm) cartridges, unless otherwise indicated.

### Analytical Methods

$^1$H-NMR spectra were recorded at 400 MHz on a Bruker Avance AV-I-400 or on a BrukerAvance AV-II-400 instrument. Chemical shift values are expressed in ppm-values relative to tetramethylsilane unless noted otherwise. The following abbreviations or their combinations are used for multiplicity of NMR signals: br = broad, d = doublet, m = multiplet, q = quartet, quint = quintet, s = singlet and t = triplet.

Method 1: Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gas pressure 40 psi, drift tube temp: 50°C; column: Waters XSelect CSH C18, 50×2.1mm, 2.5μm, Temp: 25°C, Flow: 0.6 mL/min, Gradient: t0 = 5% B, t2.0min = 98% B, t2.7min = 98% B, Post-time: 0.3 min, Eluent A: 10mM ammonium bicarbonate in water (pH=9.5), Eluent B: acetonitrile.

Method 2: Apparatus: Agilent Technologies G6130B Quadrupole; HPLC instrument type: Agilent Technologies 1290 preparative LC; Column: Waters XSelect CSH (C18, 100x30mm, 10μ); Flow: 55 mL/min; Column temp: ROOM TEMPERATURE; Eluent A: 0.1% formic acid in water; Eluent B: 100% acetonitrile lin. gradient: t=0 min 20% B, t=2 min 20% B, t=8.5 min 60% B, t=10 min 100% B, t=13 min 100% B; Detection: DAD (220-320 nm); Detection: MSD (ESI pos/neg) mass range: 100 - 1000; fraction collection based on MS and DAD.

Method 3: Apparatus: ACQ-SQD2; HPLC instrument type: Waters Modular Preparative HPLC System; column: Waters XSelect (C18, 100x30mm, 10μm); flow: 55 ml/min prep pump; column temp: ROOM TEMPERATURE; eluent A: 10mM ammonium bicarbonate in water pH=9.5, eluent B: 100% acetonitrile; detection: DAD (220-320 nm); detection: MSD (ESI pos/neg) mass range: 100 - 800; fraction collection based on MS and DAD.

Method 4: Instrument: Waters I-Class UPLC, Binary Solvent Manager (BSM), Sample Manager-FTN (SM-FTN) and Sample Organizer (SO), Column Manager (CM-A), PDA 210-320nm, QDa ESI 100-800 (pos) 100-800 (neg), Column: XSelect CSH C18 XP (50×2.1 mm 2.5μm) Flow: 0.6 ml/min; Column temp: 25°C, Eluent A: 10mM ammonium bicarbonate in water (pH 9.5), Eluent B: acetonitrile, Gradient: t=0 min 5% B, t=2 min 98% B, t=2.7 min 98% B, Post-run: 0.3 min.

Method 5: UPLC Acidic Method

Column: Waters ACQUITY UPLC® CSH C18, 1.7 μm, 2.1 × 30 mm at 40 °C
Detection: UV at 210-400 nm unless otherwise indicated, MS by electrospray ionisation. Solvents: A: 0.1% Formic in water, B: MeCN

Gradient:

| Time | %A | %B | Flow rate (ml/min) |
|---|---|---|---|
| 0.00 | 98 | 2 | 0.77 |
| 2.50 | 0 | 100 | 0.77 |
| 3.00 | 0 | 100 | 0.77 |

Method 6: LCMS Acidic Method

Column: Waters Cortecs C18, 30 × 2.1 mm, 2.7μm, at 40 °C
Detection: UV at 260nm +/- 90nm unless otherwise indicated, MS by electrospray ionisation
Solvents: A: 0.1% formic acid in water, B: MeCN

Gradient:

| Time | %A | %B | Flow rate (ml/min) |
|---|---|---|---|
| 0.00 | 98 | 2 | 1.35 |
| 2.50 | 0 | 100 | 1.35 |
| 3.00 | 0 | 100 | 1.35 |

Method Chiral 7: SFC using a Waters UPC2. The column was a Chiralpak IG 4.6X250, 5um, flow rate 4 mL/min-1 eluting with 20% MeOH (0.1 % Ammonia), 80% CO2 at a wavelength 210 - 400nm and BPR 120 Bar.

**Example 1**

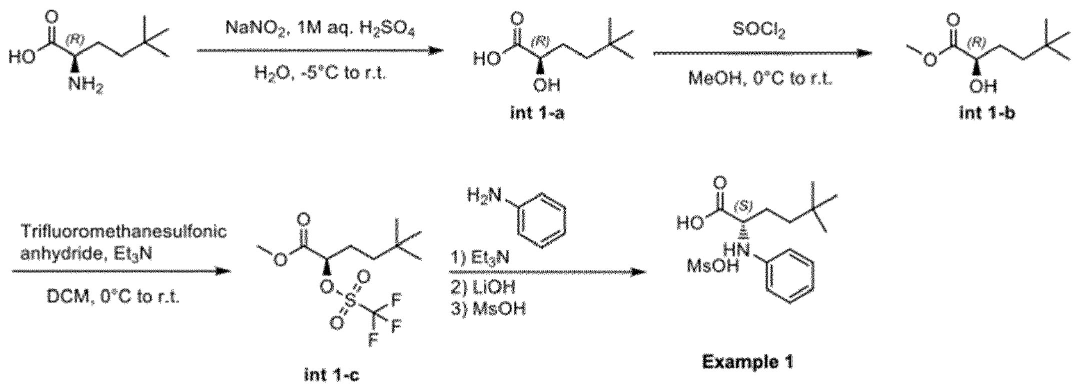

int 1-a
int 1-b
int 1-c
Example 1

### *Synthesis of (R)-2-hydroxy-5,5-dimethylhexanoic acid (Int 1-a)*

1M aq. sulfuric acid (226 mL, 226 mmol, 3.0 equiv.) was added to (R)-2-amino-5,5-dimethylhexanoic acid (12 g, 75 mmol, 1.0 equiv.) in water (220 ml). The mixture was cooled to -5 °C and a solution of sodium nitrite (31.2 g, 452 mmol, 6.0 equiv.) in Water (220 ml) was added dropwise, keeping the temperature below 0 °C. After addition, the mixture was allowed to warm to room temperature and stirred for 16 hours.

The mixture was extracted with $Et_2O$ (4 × 200 mL) and the combined organics were washed with brine (300 mL), dried over $Na_2SO_4$, filtered and concentrated in *vacuo* to afford (*R*)-2-hydroxy-5,5-dimethylhexanoic acid (8.98 g, 56.1 mmol, 74% yield) as a yellow solid. $^1$H-NMR (400 MHz, $CDCl_3$) δ 4.28 (dd, J = 7.2, 4.2 Hz, 1H), 1.92 - 1.80 (m, 1H), 1.75 - 1.62 (m, 1H), 1.41 - 1.27 (m, 2H), 0.90 (s, 9H).

### *Synthesis of methyl (R)-2-hydroxy-5,5-dimethylhexanoate (Int 1-b)*

Thionyl chloride (12 ml, 164 mmol, 2.93 equiv.) was added to (R)-2-hydroxy-5,5-dimethylhexanoic acid (8.98 g, 56.1 mmol, 1 equiv.) in MeOH (120 ml) at 0 °C. After addition, the mixture was allowed to warm to room temperature and stirred for 16 hours. The mixture was basified to pH 9 by addition of saturated aqueous NaHCOs and extracted with $Et_2O$ (2 × 400 mL). Combined organics were dried over $Na_2SO_4$, filtered and concentrated *in vacuo* to afford methyl

(R)-2-hydroxy-5,5-dimethylhexanoate (10.01 g, 55.0 mmol, 98% yield) as yellow oil. Contains 4.2% (w/w) MeOH. $^1$H-NMR (400 MHz, $CDCl_3$) δ 4.18 (dd, J = 7.2, 4.2 Hz, 1H), 3.80 (s, 3H), 1.84 - 1.72 (m, 1H), 1.67 - 1.52 (m, 1H), 1.37 - 1.21 (m, 2H), 0.89 (s, 9H).

***Synthesis of methyl (R)-5,5-dimethyl-2-(((trifluoromethyl)sulfonyl)oxy)hexanoate (Int 1-c)***

Trifluoromethanesulfonic anhydride (15.56 ml, 92 mmol) was added dropwise to a cooled solution (ice salt bath) of methyl (R)-2-hydroxy-5,5-dimethylhexanoate (14.59 g, 84 mmol) and triethylamine (14.01 ml, 100 mmol) in dichloromethane (400 ml). The colour changed from light yellow to dark orange. The mixture was stirred for 2h and diluted with water (50 ml), layers were separated and the aqueous layer was extracted with DCM. The combined organic layers were dried over sodium sulphate and concentrated. The black thick oil was put on a 4 cm silica plug and eluted off with (600mL) Heptane:DCM 1:2 in 2 fractions of 300 mL. Evaporation of the solvent afforded methyl (*R*)-5,5-dimethyl-2-(((trifluoromethyl)sulfonyl)oxy)hexanoate (19.23 g, 60.3 mmol, 72% yield) as a light yellow oil. $^1$H-NMR (400 MHz, $CDCl_3$) δ 5.12 (dd, J = 6.9, 5.0 Hz, 1H), 3.85 (s, 3H), 2.05 - 1.90 (m, 2H), 1.36 - 1.24 (m, 2H), 0.90 (s, 9H).

***Synthesis of (S)-2-anilino-5,5-dimethylhexanoic acid mesylate (Example 1)***

Methyl (R)-5,5-dimethyl-2-(((trifluoromethyl)sulfonyl)oxy)hexanoate (100 mg, 0.326 mmol) in DCM (1mL) was added dropwise to a solution of aniline (30.4 mg, 0.326 mmol) and triethylamine (50.1 μl, 0.359 mmol; 1.1 eq.) in dichloromethane (1mL). DCM was removed by an air flow and the mixture was taken up in MeCN (1mL) and water (1 mL). Lithium hydroxide (39.1 mg, 1.632 mmol; 5 equiv.) was added and the mixture was stirred overnight. The mixture was submitted for acidic prep (Method 2) to afford (*S*)-5,5-dimethyl-2-(phenylamino)hexanoic acid (18.9 mg, 0.080 mmol, 24% yield, 99% purity). The material was taken up in MeCN (1.6 mL) and methanesulfonic acid (800 μl, 0.08 mmol) in water was added. The product was lyophilized to afford (*S*)-5,5-dimethyl-2-(phenylamino)hexanoic acid compound with methanesulfonic acid (23.0 mg; 0.069 mmol; 21% yield, 99% purity). LCMS (Method 1, 0.942 min; M+H- MsOH = 236.4; calcd. 236.2). $^1$H-NMR (400 MHz, DMSO) δ 7.07 (t, J = 7.8 Hz, 2H), 6.60 (d, J = 8.2 Hz, 2H), 6.56 (t, J = 7.3 Hz, 1H), 3.80 (t, J = 6.6 Hz, 2H), 2.34 (s, 3H; MsOH), 1.68 (dqd, J = 18.0, 12.9, 6.3 Hz, 2H), 1.36 (td, J = 12.5, 5.0 Hz, 1H), 1.23 (td, J = 12.5, 5.2 Hz, 1H), 0.87 (s, 9H).

The following **Examples, 2 and 3** were prepared in an analogous manner to Example 1, starting from the corresponding precursors.

| Example | Structure | Yield | LCMS | NMR |
|---|---|---|---|---|
| **2** | 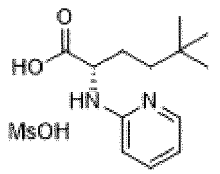 | 4.3 mg (4% yield, 97% purity) | Method 1, 0.860 min; M+H-MsOH= 237.4; calcd. 237.2 | |
| **3** | 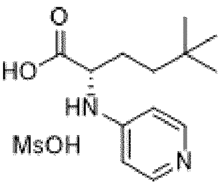 | 54.6 mg (50% yield, 100% purity) | Method 1, 0.839 min; M+H-MsOH= 237.4; calcd. 237.2 | $^1$H-NMR (400 MHz, DMSO) δ 14.27 - 13.47 (br, 1H), 8.28 - 8.11 (m, 4H), 6.84 (d, *J* = 7.5 Hz, 2H), 5.06 (dd, *J* = 10.7, 4.8 Hz, 1H), 2.30 (s, 3H; MsOH), 2.21 - 1.98 (m, 2H), 1.25 (td, *J* = 12.6, 4.7 Hz, 1H), 0.84 (s, 9H), 0.77 (td, *J* = 12.8, 4.5 Hz, 1H). |

**Example 4**

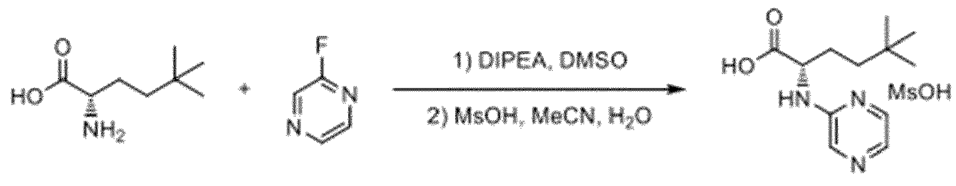


Example 4

*Synthesis of (S)-5,5-dimethyl-2-(2-pyrazinylamino)hexanoic acid (Example 4)*

2-Fluoropyrazine (60.1 mg, 0.612 mmol; 1.5 equiv.) was added to a solution of (S)-2-amino-5,5-dimethylhexanoic acid (65.0 mg, 0.408 mmol) in dimethyl sulfoxide (dry) (1 ml) and DIPEA(0.214 ml, 1.225 mmol; 3.00 equiv.). The reaction mixture was stirred at 110 °C overnight. The mixture was submitted for basic prep (Method 3). Purified fractions were combined, reformatted, redissolved in MeCN after weighing (0.05M) and a 0.1M MsOH in MeCN (1.0 equiv.) solution was added. Little water was added and solvent evaporated, affording (*S*)-5,5-dimethyl-2-(pyrazin-2-ylamino)hexanoic acid compound with methanesulfonic acid (53.8 mg, 0.161 mmol, 39% yield, 100 % purity). LCMS (Method 1, 0.773 min; M+H-MsOH = 238.3; calcd. 238.2). $^1$H-NMR (400 MHz, DMSO) $\delta$ 8.05 (d, J = 1.5 Hz, 1H), 7.91 (dd, J = 2.8, 1.5 Hz, 1H), 7.68 (d, J = 2.8 Hz, 1H), 7.41-7.27 (br, 2H), 4.25 (dd, J = 8.2, 5.0 Hz, 1H), 2.33 (s, 3H, MsOH), 1.85 - 1.63 (m, 2H), 1.39 - 1.21 (m, 2H), 0.87 (s, 9H).

The following **Example 5** was prepared in an analogous manner to Example 4, starting from the corresponding precursors.

| Example | Structure | Yield | LCMS | NMR |
|---|---|---|---|---|
| **5** | 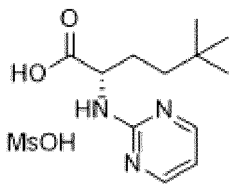 | 65.3 mg (48% yield, 99% purity) | Method 1, 0.786 min; M+H-MsOH= 238.2; calcd. 238.2 | $^1$H-NMR (400 MHz, DMSO) $\delta$ 8.39 (d, *J* = 5.0 Hz, 2H), 7.87 - 7.60 (m, 2H), 6.74 (t, *J* = 4.9 Hz, 1H), 4.29 (dd, *J* = 8.5, 5.0 Hz, 1H), 2.36 (s, 3H, MsOH), 1.85 - 1.63 (m, 2H), 1.39 - 1.12 (m, 2H), 0.86 (s, 9H). |

**Example 6**

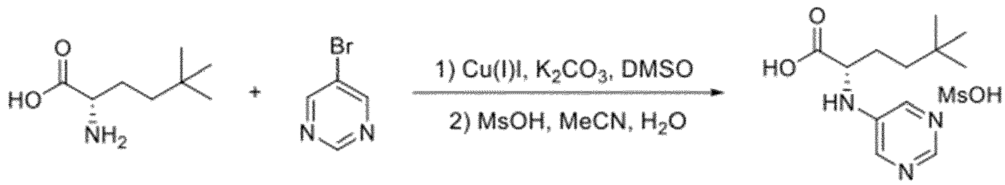


Example 6

*Synthesis of (S)-5,5-dimethyl-2-(5-pyrimidinylamino)hexanoic acid (Example 6)*

5-Bromopyrimidine (150 mg, 0.942 mmol), potassium carbonate (132 mg, 0.956 mmol) and Copper(I) iodide (9.87 mg, 0.052 mmol) were added to a solution of (S)-2-amino-5,5-dimethylhexanoic acid (75 mg, 0.471 mmol) in Dimethyl sulfoxide (dry) (2 ml). The reaction mixture was stirred at 100 °C overnight. The mixture was purified by basic prep (Method 3). Purified fractions were combined and concentrated *in vacuo.* The filtrates showed residual copper (blue-green solid). Solids were suspended in water with little MeOH and filtered over an SCX cation resin. The column wash flushed with water, then MeOH, then 7M ammonia in MeOH to flush off the target, affording 12.2 mg of an off-white solid. Redissolved in MeCN and a 0.1M MsOH in MeCN (1.0 equiv.) solution was added. Little water was added and solvent evaporated, affording (S)-5,5-dimethyl-2-(pyrimidin-5-ylamino)hexanoic acid compound with methanesulfonic acid (16.6 mg, 0.050 mmol, 10% yield, 93% purity). LCMS (Method 1, 0.773 min; M+H- MsOH = 238.2; calcd. 238.2). $^1$H-NMR (400 MHz, DMSO) $\delta$ 12.96 - 12.63 (br, 1H), 8.41 (s, 1H), 8.14 (s, 2H), 6.38 - 6.29 (m, 2H), 4.06 - 3.96

(m, 1H), 2.30 (s, 3H, MsOH), 1.85 - 1.61 (m, 2H), 1.39 - 1.17 (m, 2H), 0.87 (s, 9H).

**Example 7**

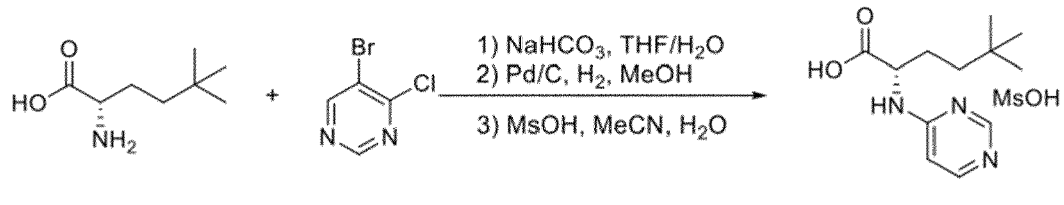


Example 7

***Synthesis of (S)-5,5-dimethyl-2-(4-pyrimidinylamino)hexanoic acid (Example 7)***

5-bromo-4-chloropyrimidine (134 mg, 0.691 mmol) and sodium bicarbonate (264 mg, 3.14 mmol) were added to a solution of (S)-2-amino-5,5-dimethylhexanoic acid (100 mg, 0.628 mmol) in THF (8 mL)/water (2 mL). The reaction mixture was stirred at 70 °C for 3h. The mixture was concentrated, purged with nitrogen, Palladium 5 % on activated carbon (401 mg, 0.188 mmol) was added and the mixture put under hydrogen via 3 vacuum/hydrogen cycles. Stirred overnight at room temperature. The mixture was purged with nitrogen, filtered over celite and filtrate concentrated, affording 185 mg of a white solid. The product was purified by acidic prep (Method 2). Purified fractions were combined, reformatted, redissolved in MeCN after weighing (0.05M) and a 0.1M MsOH in MeCN (1 equiv.) solution was added. Little water was added and solvent evaporated, affording (S)-5,5-dimethyl-2-(pyrimidin-4-ylamino)hexanoic acid compound with methanesulfonic acid (56.7 mg, 0.170 mmol, 27% yield, 100 % purity). LCMS (Method 1, 0.743 min; M+H-MsOH = 238.4; calcd. 238.2). $^1$H-NMR (400 MHz, DMSO) $\delta$ 13.42 - 12.83 (br, 1H), 9.60 (d, J = 7.6 Hz, 1H), 8.84 (s, 1H), 8.22 (dd, J = 7.1, 1.5 Hz, 1H), 6.95 (d, J = 7.8 Hz, 1H), 4.60 (td, J = 8.0, 4.9 Hz, 1H), 2.32 (s, 3H, MsOH), 1.92 - 1.69 (m, 2H), 1.31 - 1.20 (m, 2H), 0.87 (s, 9H).

**Example 8**

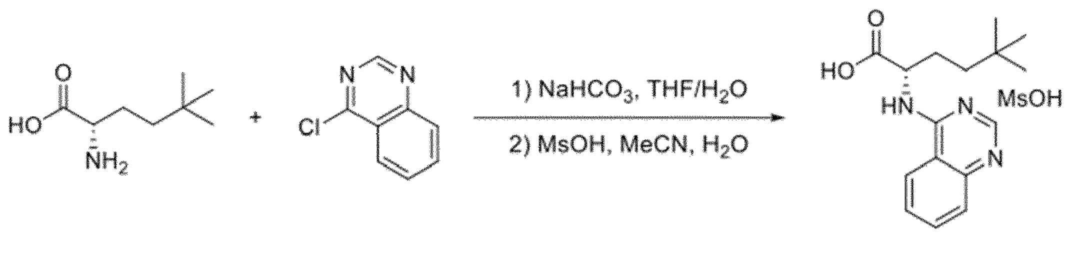


Example 8

***Synthesis of (S)-5,5-dimethyl-2-(4-quinazolinylamino)hexanoic acid (Example 8)***

4-chloroquinazoline (56.9 mg, 0.345 mmol) and sodium bicarbonate (132 mg, 1.57 mmol) were added to a solution of (S)-2-amino-5,5-dimethylhexanoic acid (50 mg, 0.314 mmol) in Tetrahydrofuran (4 ml) Water (1 ml). The reaction mixture was stirred at 70 °C overnight. The mixture was concentrated and purified by acidic prep (Method 2). Purified fractions were combined, reformatted, redissolved in MeCN after weighing (0.05M) and a 0.1M MsOH in MeCN (1 equiv.) solution was added. Little water was added and solvent evaporated, affording (S)-5,5-dimethyl-2-(quinazolin-4-ylamino)hexanoic acid compound with methanesulfonic acid (74.2 mg, 0.193 mmol, 61% yield, 97% purity). LCMS (Method 1, 0.903 min; M+H-MsOH = 288.4; calcd. 288.2). $^1$H-NMR (400 MHz, DMSO) $\delta$ 13.20 - 12.85 (m, 1H), 9.64 (d, J = 7.3 Hz, 1H), 8.83 (s, 1H), 8.63 (d, J = 8.9 Hz, 1H), 8.03 (t, J = 7.8 Hz, 1H), 7.84 - 7.74 (m, 2H), 4.86 (q, J = 7.4 Hz, 1H), 2.30 (s, 3H), 2.04 - 1.89 (m, 2H), 1.45 - 1.22 (m, 2H), 0.90 (s, 9H).

The following **Examples 9 to 28** were prepared in an analogous manner to Example 8, starting from the

corresponding precursors.

| Ex. | Structure | Yield | LCMS | NMR |
|---|---|---|---|---|
| **9** | 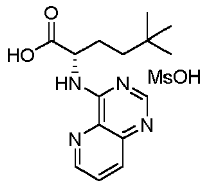 | 46.5 mg (38% yield, 99% purity) | Method 1, 0.849 min; M+H-MsOH= 289.4; calcd. 289.2 | $^1$H-NMR (400 MHz, DMSO) $\delta$ 10.43 (d, J = 8.0 Hz, 1H), 9.08 (dd, J = 4.4, 1.4 Hz, 1H), 8.99 (s, 1H), 8.28 (dd, J = 8.6, 1.5 Hz, 1H), 8.12 (dd, J = 8.6, 4.3 Hz, 1H), 4.91 (td, J = 8.5, 4.9 Hz, 1H), 2.35 (s, 3H), 2.15 - 1.98 (m, 2H), 1.37 - 1.17 (m, 2H), 0.87 (s, 9H). |
| **10** | 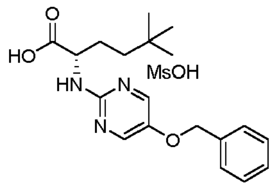 | 16.3 mg (11% yield, 98% purity) | Method 1, 1.073 min; M+H-MsOH= 344.2; calcd. 344.2 | $^1$H-NMR (400 MHz, DMSO) $\delta$ 12.58 - 11.96 (m, 1H), 8.16 (s, 2H), 7.46 - 7.30 (m, 5H), 5.07 (s, 2H), 4.16 - 4.07 (m, 1H), 2.30 (s, 3H, MsOH), 1.81 - 1.61 (m, 2H), 1.36 (td, *J* = 12.4, 4.8 Hz, 1H), 1.21 (td, *J* = 12.3, 5.1 Hz, 1H), 0.86 (s, 9H). |
| **11** | 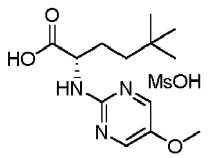 | 6.6 mg (5% yield, 92% purity) | Method 1, 0.857 min; M+H-MsOH= 268.1; calcd. 268.2 | $^1$H-NMR (400 MHz, DMSO) $\delta$ 8.10 (s, 2H), 6.72 (d, *J* = 7.5 Hz, 1H), 4.08 (td, *J* = 7.9, 4.9 Hz, 1H), 3.74 (s, 3H), 2.29 (s, 3H, MsOH), 1.79 - 1.61 (m, 2H), 1.32 (td, *J* = 12.7, 5.0 Hz, 1H), 1.22 (tdz, *J* = 13.1, 6.5 Hz, 1H), 0.85 (s, 9H). |
| **12** | 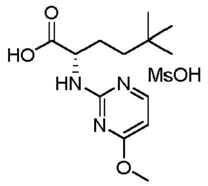 | 42.3 mg (37% yield, 97% purity) | Method 1, 0.893 min; M+H-MsOH= 268.1; calcd. 268.2 | $^1$H-NMR (400 MHz, DMSO) $\delta$ 13.36 - 12.16 (br, 1H), 8.42 (s, 1H), 8.20 - 7.94 (m, 1H), 6.43 - 6.06 (m, 1H), 4.45 - 4.16 (m, 1H), 3.91 (s, 3H), 2.33 (s, 3H, MsOH), 1.88 - 1.64 (m, 2H), 1.45 - 1.16 (m, 2H), 0.87 (s, 9H). |
| **14** | 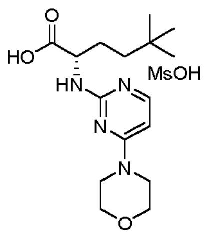 | 19.0 mg (14% yield, 90% purity) | Method 1, 0.877 min; M+H-MsOH= 323.1; calcd. 323.2 | $^1$H-NMR (400 MHz, DMSO) $\delta$ 11.71 - 11.49 (br, 1H), 8.27 (d, *J* = 6.9 Hz, 1H), 7.88 (d, *J* = 7.6 Hz, 1H), 6.55 (d, *J* = 7.6 Hz, 1H), 4.39 - 4.22 (m, 1H), 3.85 - 3.58 (m, 9H), 2.31 (s, 3H, MsOH), 1.88 - 1.63 (m, 2H), 1.25 (dd, *J* = 10.1, 7.0 Hz, 2H), 0.87 (s, 9H). |
| **15**[a] | 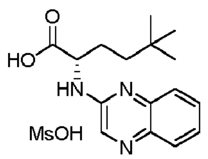 | 15.7 mg (13% yield, 95.31% purity) | Method 1, 0.959 min; M+H-MsOH= 288.1; calcd. 288.2 | $^1$H-NMR (400 MHz, DMSO) $\delta$ 8.46 (s, 1H), 7.95 (s, 1H), 7.78 (d, *J* = 7.6 Hz, 1H), 7.70 - 7.48 (m, 2H), 7.36 (ddd, *J* = 8.3, 6.3, 2.0 Hz, 1H), 4.49 (q, *J* = 6.3 Hz, 1H), 2.32 (s, 3H), 1.91 - 1.70 (m, 2H), 1.42 - 1.27 (m, 2H), 0.89 (s, 9H). |
| **18**[a] | 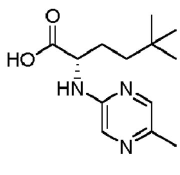 | 14.9 mg (13% yield, 95% purity) | Method 4, 0.827 min; M+H-MsOH= 252.1; calcd. 252.2 | $^1$H-NMR (400 MHz, DMSO) $\delta$ 12.61 - 12.19 (br, 1H), 7.96 (d, *J* = 1.4 Hz, 1H), 7.79 (s, 1H), 7.07 - 6.98 (m, 1H), 4.28 - 4.16 (m, 1H), 2.30 (s, 3H, MsOH), 2.25 (s, 3H), 1.82 - 1.63 (m, 2H), 1.41 - 1.17 (m, 2H), 0.87 (s, 9H). |
| **19**[a] | 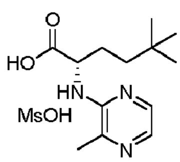 | 26.6 mg (24% yield, 99% purity) | Method 4, 0.827 min; M+H-MsOH= 252.1; calcd. 252.2 | $^1$H-NMR (400 MHz, DMSO) $\delta$ 7.84 (d, *J* = 2.9 Hz, 1H), 7.65 (d, *J* = 2.9 Hz, 1H), 4.32 - 4.24 (m, 1H), 2.38 (s, 3H), 2.31 (s, 3H), 1.88 - 1.74 (m, 2H), 1.44 - 1.33 (m, 1H), 1.28 - 1.18 (m, 1H), 0.88 (s, 9H). |

(continued)

| Ex. | Structure | Yield | LCMS | NMR |
|---|---|---|---|---|
| **21**[a] | 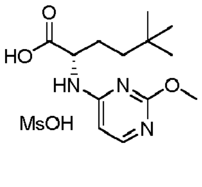 | 18.6 mg (16% yield, 83% purity) | Method 4, 0.713 min; M+H-MsOH= 268.1; calcd. 268.2 | $^{1}$H-NMR (400 MHz, DMSO) δ 12.76 (s, 1H), 8.49 (s, 1H), 7.91 (d, *J* = 6.3 Hz, 1H), 6.39 (d, *J* = 6.3 Hz, 1H), 4.43 (d, *J* = 6.7 Hz, 1H), 3.86 (s, 3H), 2.30 (s, 3H, MsOH), 1.87 - 1.58 (m, 2H), 1.39 - 1.11 (m, 2H), 0.87 (s, 9H). |
| **22** | 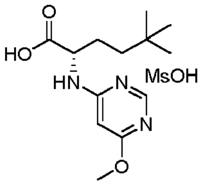 | 13.9 mg (12% yield, 99% purity) | Method 4, 0.836 min; M+H-MsOH= 268.1; calcd. 268.2 | $^{1}$H-NMR (400 MHz, DMSO) δ 12.80 - 12.06 (br, 1H), 8.18 (s, 1H), 7.61 - 7.31 (m, 1H), 5.90 (s, 1H), 4.37 (s, 1H), 3.80 (s, 3H), 2.30 (s, 1H, MsOH), 1.82 - 1.59 (m, 2H), 1.25 (pd, *J* = 13.2, 5.0 Hz, 2H), 0.86 (s, 9H). |
| **23**[a] | 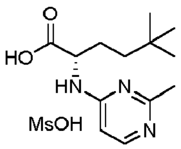 | 81.3 mg (74% yield, 99% purity) | Method 1, 0.767 min; M+H-MsOH= 252.4; calcd. 252.2 | $^{1}$H-NMR (400 MHz, DMSO) δ 13.96 - 13.79 (br, 1H), 9.51 (d, *J* = 7.7 Hz, 1H), 8.15 (d, *J* = 7.2 Hz, 1H), 6.81 (d, *J* = 7.2 Hz, 1H), 4.63 (td, *J* = 8.0, 5.0 Hz, 1H), 2.51 (s, 3H), 2.32 (s, 3H, MsOH), 1.95 - 1.68 (m, 2H), 1.45 - 1.11 (m, 2H), 0.87 (s, 9H). |
| **24** | 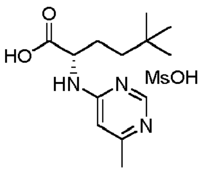 | 63.7 mg (58% yield, 99% purity) | Method 1, 0.772 min; M+H-MsOH= 252.4; calcd. 252.2 | $^{1}$H-NMR (400 MHz, DMSO) δ 14.27 - 13.82 (br, 1H), 13.25 - 12.94 (br, 1H), 9.48 (d, *J* = 7.6 Hz, 1H), 8.78 (s, 1H), 6.73 (s, 1H), 4.59 (td, *J* = 8.1, 4.8 Hz, 1H), 2.39 (s, 3H), 2.30 (s, 3H, MsOH), 1.95-1.62 (m, 2H), 1.41 - 1.16 (m, 2H), 0.87 (s, 9H). |
| **25** | 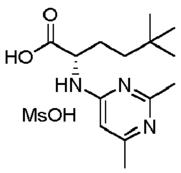 | 56.0 mg (49% yield, 98% purity) | Method 1, 0.790 min; M+H-MsOH=266.5; calcd. 266.2 | $^{1}$H-NMR (400 MHz, DMSO) δ 13.68 (s, 1H), 13.05 (s, 1H), 9.38 (d, *J* = 7.8 Hz, 1H), 6.59 (s, 1H), 4.62 (td, *J* = 8.1, 4.8 Hz, 1H), 2.36 (s, 3H), 2.30 (s, 3H, MsOH), 1.89 - 1.66 (m, 2H), 1.29 - 1.18 (m, 2H), 0.87 (s, 9H). |
| **26** | 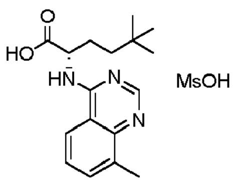 | 70.7 mg (56% yield, 98% purity) | Method 1, 0.941 min; M+H-MsOH= 302.5; calcd. 302.2 | $^{1}$H-NMR (400 MHz, DMSO) δ 13.27 - 12.85 (br, 1H), 9.72 (s, 1H), 8.82 (s, 1H), 8.48 (d, *J* = 8.4 Hz, 1H), 7.90 (d, *J* = 7.2 Hz, 1H), 7.70 (t, *J* = 7.8 Hz, 1H), 4.87 (q, *J* = 7.4 Hz, 1H), 2.59 (s, 3H), 2.31 (s, 3H, MsOH), 2.03 - 1.93 (m, 2H), 1.42 - 1.22 (m, 2H), 0.89 (s, 9H). |
| **27** | 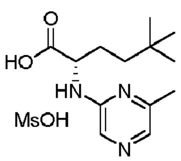 | 15.7 mg (14% yield, 96% purity) | Method 4, 0.841 min; M+H-MsOH= 252.1; calcd. 252.2 | $^{1}$H-NMR (400 MHz, DMSO) δ 7.85 (s, 1H), 7.60 (s, 1H), 7.29 (brz, 1H), 4.33 - 4.27 (m, 1H), 2.32 (s, 3H, MsOH), 2.23 (s, 3H), 1.81 - 1.64 (m, 2H), 1.37 - 1.21 (m, 2H), 0.87 (s, 9H). |
| **28** | 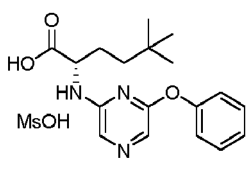 | 33.0 mg (24% yield, 99% purity) | Method 4, 1.076 min; M+H-MsOH= 330.2; calcd. 330.2 | $^{1}$H-NMR (400 MHz, DMSO) δ 7.79 (s, 1H), 7.47 - 7.33 (m, 4H), 7.20 (t, *J* = 7.4 Hz, 1H), 7.14 - 7.11 (m, 1H), 7.11 - 7.09 (m, 1H), 4.08 - 3.97 (m, 1H), 2.31 (s, 3H, MsOH), 1.74 - 1.64 (m, 1H), 1.64 - 1.52 (m, 1H), 1.20 (pd, *J* = 13.1, 5.0 Hz, 2H), 0.83 (s, 9H). |

Starting from the corresponding heteroaryl bromide

**Example 29**

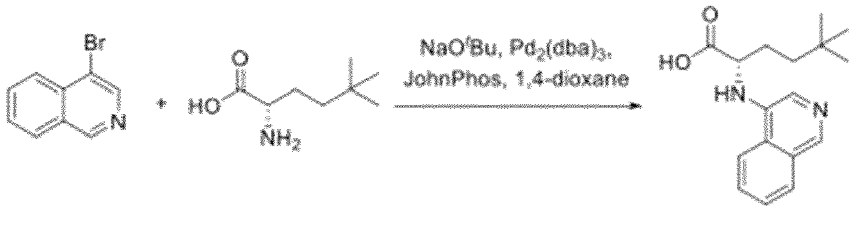


**Example 29**

***Synthesis of (S)-2-(isoquinolin-4-ylamino)-5,5-dimethylhexanoic acid (Example 29)***

4-Bromoisoquinoline (212 mg, 98 % Wt, 1 Eq, 1.00 mmol), (S)-2-amino-5,5-dimethylhexanoic acid (209 mg, 99 % Wt, 1.30 Eq, 1.30 mmol), $Pd_2(dba)_3$ (18.5 mg, 99% Wt, 0.0200 Eq, 20.0 μmol), JohnPhos (15.1 mg, 99% Wt, 0.05 Eq, 50.0 μmol) and sodium tert-butoxide (233 mg, 99% Wt, 2.40 Eq, 2.40 mmol) were combined and flushed with nitrogen for 5 min before 1,4-Dioxane (5.00 mL) was added. The reaction mixture was stirred at room temperature for 1 min, before being heated up to 70 °C for 2 hours. The mixture was cooled to room temperature and was quenched with hydrogen chloride (87.5 mg, 2.40 mL, 1.00 molar, 2.40 Eq, 2.40 mmol). The mixture was diluted with DCM (15 mL) and the layers were separated. The aqueous was extracted with DCM (10 mL). The combined organic layers were washed with brine (20 mL), dried over $MgSO_4$, filtered, and concentrated *in vacuo*. The crude product was purified by chromatography on silica gel (solid load on celite, 12 g cartridge, 0-20% DCM/MeOH) to afford (S)-2-(isoquinolin-4-ylamino)-5,5-dimethylhexanoic acid (78.0 mg, 269 μmol, 26% yield, 98% Purity) as a yellow solid. UPLC (Method 5), 1.23 min; M+H = 287.3. $^1$H-NMR (400 MHz, DMSO) δ 12.72 (bs, 1H), 8.59 (s, 1H), 8.36 (d, J = 8.4 Hz, 1H), 7.96 (dd, J = 8.2, 1.4 Hz, 1H), 7.70 (ddd, J = 8.4, 6.8, 1.5 Hz, 1H), 7.66 - 7.58 (m, 2H), 6.26 (s, 1H), 4.02 (s, 1H), 1.90 (dt, J = 8.6, 6.6 Hz, 2H), 1.55 - 1.45 (m, 1H), 1.33 - 1.26 (m, 1H), 0.90 (s, 9H).

**Example 30**

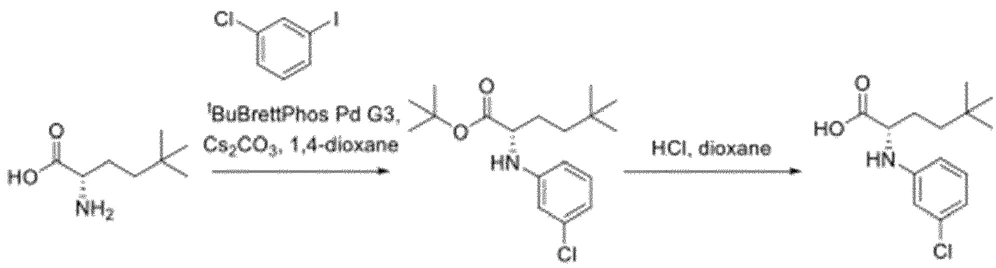


**Int 30-a** **Example 30**

***Synthesis of tert-butyl (S)-2-((3-chlorophenyl)amino)-5,5-dimethylhexanoate (Int 30-a)***

A mixture of *tert*-butyl (S)-2-amino-5,5-dimethylhexanoate (70 mg, 1 Eq, 0.33 mmol), 1-chloro-3-iodobenzene (78 mg, 40 μL, 1 Eq, 0.33 mmol) and tBuBrettPhos Pd G3, 96% (15 mg, 96% Wt, 0.052 Eq, 17 μmol) was sparged with nitrogen before $Cs_2CO_3$ (0.16 g, 1.5 Eq, 0.49 mmol) and 1,4-Dioxane (3.0 mL) were added. The mixture was again sparged with nitrogen and the reaction was heated at 50 °C for 18 h. The reaction mixture was cooled to room temperature before being acidified with formic acid (~150 uL) then filtered and concentrated on to silica (~1 g). The crude product was purified by chromatography on silica gel (12 g cartridge, 0-100% EtOAc/isohexane) to afford tert-butyl (S)-2-((3-chlorophenyl)amino)-5,5-dimethylhexanoate (14 mg, 42 μmol, 13% yield, 98% Purity) as a yellow gum. LCMS (Method 6), 2.70 min; M-$^t$Bu+H = 270.2. $^1$H-NMR (500 MHz, MeOD) δ 7.07 (app. t, J = 8.0 Hz, 1H), 6.67 - 6.60 (m, 2H), 6.56 (ddd, J = 8.2, 2.3, 0.9 Hz, 1H), 3.81 (t, J = 6.6 Hz, 1H), 1.87 - 1.70 (m, 2H), 1.45 (s, 9H), 1.46 - 1.38 (m, 1H), 1.32 (app. td, J = 12.8, 5.0 Hz, 1H), 0.95 (s, 9H).

***Synthesis of (S)-2-((3-chlorophenyl)amino)-5,5-dimethylhexanoic acid (Example 30)***

A solution of *tert*-butyl (S)-2-((3-chlorophenyl)amino)-5,5-dimethylhexanoate (14 mg, 1 Eq, 43 μmol) in 4 M HCl in dioxane (3 mL) was stirred at room temperature for 2 hours before concentrating to afford (S)-2-((3-chlorophenyl)amino)-5,5-dimethylhexanoic acid (10 mg, 36 μmol, 85% yield, 98% Purity) as colourless solid. LCMS (Method 6), 2.14 min, M+H = 270.2. $^1$H-NMR (500 MHz, DMSO) δ 12.67 - 12.51 (m, 1H), 7.06 (app. t, J = 8.0 Hz, 1H), 6.58 (app. t, J = 2.2 Hz, 1H), 6.56 - 6.49 (m, 2H), 6.17 - 6.13 (m, 1H), 3.81 - 3.77 (m, 1H), 1.76 - 1.61 (m, 2H), 1.38 - 1.17 (m, 2H), 0.87 (s, 9H).

The following **Example 31** was prepared in an analogous manner to **Example 30,** starting from the corresponding aryl bromide.

| Ex. | Structure | Yield | LCMS | NMR |
|---|---|---|---|---|
| 31 | 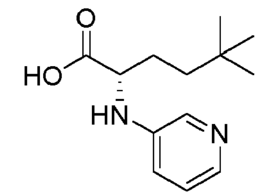 | 8 mg (60% yield, 98% purity) | Method 6, 1.13 min, M+H = 237.2 | $^1$H-NMR (500 MHz, DMSO) δ 12.91 (s, 1H), 8.09 (d, J = 2.7 Hz, 1H), 8.01 (d, J = 5.1 Hz, 1H), 7.67 - 7.48 (m, 2H), 7.10-7.04 (m, 1H), 4.14 - 4.11 (m, 1H), 1.86 - 1.63 (m, 2H), 1.36 - 1.22 (m, 2H), 0.87 (s, 9H). |

**Example 32**

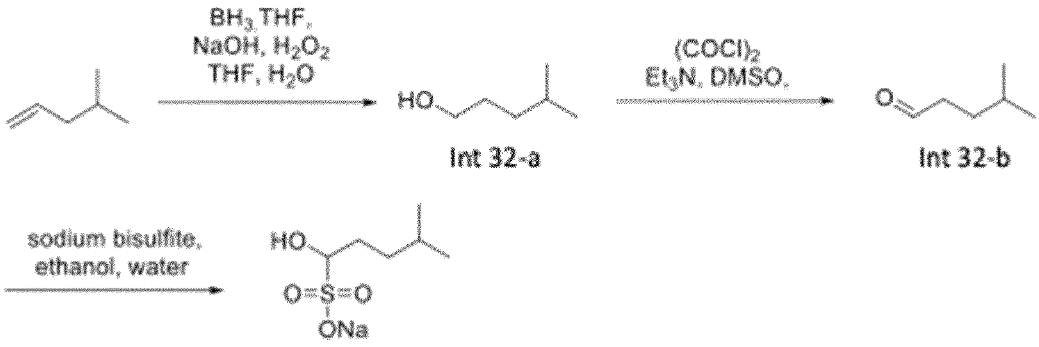


Int 32-a

Int 32-b

Int 32-c

***Synthesis of 4-methylpentan-1-ol (Int 32-a)***

THF (51.0 mL) and $BH_3.Me_2S$ (1.99 g, 13.1 mL, 2.00 molar, 0.44 Eq, 26.1 mmol) were combined and cooled to 0 °C (internal temperature) with an ice bath. 4-Methylpent-1-ene (5.00 g, 7.52 mL, 1 Eq, 59.4 mmol) was added dropwise and the reaction was allowed to stir at 0 °C for 1.5 h. The solution was allowed to warm up to r.t. and stirred over 16 h. Water (8.70 mL), sodium hydroxide (1.05 g, 8.71 mL, 3.00 molar, 0.44 Eq, 26.1 mmol), and hydrogen peroxide (9.63 g, 8.76 mL, 30% Wt, 1.43 Eq, 85.0 mmol) were added dropwise to the reaction flask respectively while cooled to 0 °C, and stirred for another 10 minutes at the same temperature. The reaction was allowed to stir for 2 hours at room temperature. The reaction mixture was diluted with MTBE (300 mL) and washed with brine (3 x 150 mL). The organic layer was dried over $MgSO_4$ and the solvent removed *in vacuo.* 4-Methylpentan-1-ol (5.5 g, 48 mmol, 81% yield, 99% Purity) was afforded as a colorless oil. UPLC (Method 5), n/a min; M+H = n/a. $^1$H-NMR (400 MHz, DMSO) δ 4.32 (t, J = 5.2 Hz, 1H), 3.36 (td, J = 6.7, 5.2 Hz, 2H), 1.50 (dp, J = 13.3, 6.7 Hz, 1H), 1.44 - 1.35 (m, 2H), 1.19 - 1.12 (m, 2H), 0.86 (s, 3H), 0.84 (s, 3H).

***Synthesis of 4-methylpentanal (Int 32-b)***

DMSO (8.78 g, 7.98 mL, 2.9 Eq, 112 mmol) and oxalyl chloride (7.38 g, 5.09 mL, 1.5 Eq, 58.1 mmol) were combined in DCM (200 mL) at -65 °C. After 10 min stirring, 4-methylpentan-1-ol (4.00 g, 4.92 mL, 99% Wt, 1 Eq, 38.8

mmol) was added and the mixture was further stirred over 1 h. $Et_3N$ (11.8 g, 16.2 mL, 3 Eq, 116 mmol) was added dropwise and stirred over 10 min, before being allowed to warm to room temperature and stirred over 2.5 h. The reaction was quenched with water followed by the addition of aqueous 1 M HCl (200 mL) and the layers were separated. The aqueous layer was extracted with DCM (2x100 mL) and the combined organic layers were washed with water (150 mL), 1 M aqueous HCl (150 mL), saturated aqueous NaHCOs (150mL) and brine (3x150 mL). After drying over $MgSO_4$, the mixture was filtered and concentrated *in vacuo* to afford 4-methylpentanal (6.14 g, 27 mmol, 70% yield, 44% Purity) as a yellow liquid. UPLC (Method 5), n/a min; M+H = n/a. $^1$H-NMR (400 MHz, DMSO) δ 9.71 (t, J = 1.7 Hz, 1H), 2.48 - 2.41 (m, 2H), 1.55 (ddd, J = 13.8, 12.8, 6.5 Hz, 1H), 1.46 (qd, J = 7.1, 0.6 Hz, 2H), 0.90 (d, J = 6.5 Hz, 6H).

***Synthesis of sodium 1-hydroxy-4-methylpentane-1-sulfonate (Int 32-c)***

4-methylpentanal (2.14 g) was diluted in Ethanol (7.00 mL). To this mixture was added, dropwise over 5 min, a solution of sodium bisulfite (999 mg, 0.248 Eq, 9.60 mmol) in water (1.60 mL) at 45 °C and stirred over 2 hours. The mixture was cooled to room temperature and filtered before being washed with ethanol (2x5 mL) to give sodium 1-hydroxy-4-methylpentane-1-sulfonate (1.28 g, 6.2 mmol, 65% yield, 99% Purity) as a white solid. UPLC (Method 5), n/a min; M+H = n/a. $^1$H-NMR (400 MHz, DMSO) δ 5.06 (s, 1H), 3.73 (d, J = 9.5 Hz, 1H), 1.75 (ddq, J = 13.1, 8.1, 2.7 Hz, 1H), 1.56 - 1.27 (m, 3H), 1.21 - 1.10 (m, 1H), 0.85 (dd, J = 6.6, 3.1 Hz, 6H).

The following **Intermediate 32-c-2 (Int 32-c-2)** was prepared in an analogous manner to **Int 32-c,** starting from the corresponding alkene.

| Intermediate | Structure | Yield | NMR |
|---|---|---|---|
| **Int 32-c-2** | 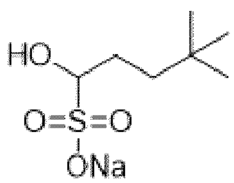 | 4.2 g (56% yield, 99% purity) | $^1$H-NMR (400 MHz, DMSO) δ 5.04 (dd, J = 5.6, 0.9 Hz, 1H), 3.73 - 3.64 (m, 1H), 1.79 - 1.66 (m, 1H), 1.46 - 1.29 (m, 2H), 1.09 (td, J = 13.3, 4.5 Hz, 1H), 0.85 (s, 9H). |

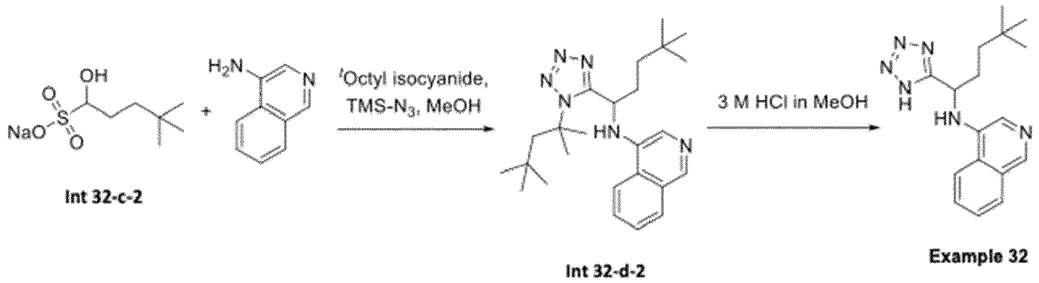

Int 32-c-2

Int 32-d-2

Example 32

***Synthesis of N-(4,4-dimethyl-1-(1-(2,4,4-trimethylpentan-2-yl)-1H-tetrazol-5-yl)pentyl)isoquinolin-4-amine (Int 32-d-2)***

A mixture of isoquinolin-4-amine (147 mg, 98% Wt, 1 Eq, 1.00 mmol) and sodium 1-hydroxy-4,4-dimethylpentane-1-sulfonate (220 mg, 99% Wt, 1 Eq, 1.00 mmol) in MeOH (4.00 mL) was stirred at room temperature for 10 min before methylidyne(2,4,4-trimethylpentan-2-yl)-l4-azane (140 mg, 177 μL, 1 Eq, 1.00 mmol) and TMS-$N_3$ (121 mg, 140 μL, 95% Wt, 1 Eq, 1.00 mmol) were added. The mixture was stirred at 25 °C for 16 h. The reaction mixture was quenched with saturated aqueous NaHCOs (15 mL) and extracted with EtOAc (3 x 15 mL). The combined organic layers were washed with brine, dried over $MgSO_4$, filtered and concentrated *in vacuo.* The crude product was purified by chromatography on silica gel (12 g cartridge, 0-100% EtOAc/isohexane) to give *N*-(4,4-dimethyl-1-(1-(2,4,4-trimethylpentan-2-yl)-1H-tetrazol-5-yl)pentyl)isoquinolin-4-amine (272 mg, 0.53 mmol, 53% yield, 82% Purity) as a white solid. UPLC (Method 5), 1.71 min; M+H = 423.6. $^1$H-NMR (400 MHz, DMSO) δ 8.63 (s, 1H), 8.28 (d, J = 8.3 Hz, 1H), 8.04 (s, 1H), 7.96 (dd, J = 7.8, 1.5 Hz, 1H), 7.69 - 7.57 (m, 2H), 6.51 (d, J = 9.0 Hz, 1H), 5.27 (q, J = 7.5 Hz, 1H), 2.28 - 2.11 (m, 2H), 1.99 (d, J = 15.1 Hz, 1H), 1.92 (d, J = 15.1 Hz, 1H), 1.75 (d, J = 7.4 Hz, 6H), 1.53 (td, J = 12.5, 4.7 Hz, 1H), 1.20 (td, J = 12.5, 4.6 Hz, 1H), 0.88 (s, 9H), 0.60 (s, 9H).

***Synthesis of N-(4,4-dimethyl-1-(1H-tetrazol-5-yl)pentyl)isoquinolin-4-amine (Example 32)***

A mixture of *N*-(4,4-dimethyl-1-(1-(2,4,4-trimethylpentan-2-yl)-1H-tetrazol-5-yl)pentyl)isoquinolin-4-amine (170 mg, 82% Wt, 1 Eq, 330 μmol) was stirred in 3 M HCl in MeOH (722 mg, 6.60 mL, 3.00 molar, 60 Eq, 19.8 mmol) at 65 °C over 16 h. The crude was concentrated *in vacuo* and co-evaporated several times with MeOH. The product was loaded onto celite then purified by chromatography on silica gel (12 g cartridge, 0-20% MeOH/DCM), the pure fractions were collected and evaporated. The crude product was loaded onto a column of SCX (1 g) in MeOH. The column was washed with MeOH and then the product was eluted with 0.7 M ammonia in MeOH. The resultant mixture was concentrated *in vacuo* to afford *N*-(4,4-dimethyl-1-(1H-tetrazol-5-yl)pentyl)isoquinolin-4-amine (74.0 mg, 0.24 mmol, 72% yield, 99% Purity) as a yellow solid. UPLC (Method 5), 1.18 min; M+H = 311.4. $^1$H-NMR (400 MHz, DMSO) δ 8.54 (s, 1H), 8.38 (d, J = 8.5 Hz, 1H), 7.94 (dd, J = 8.1, 1.3 Hz, 1H), 7.73 - 7.66 (m, 2H), 7.61 (t, J = 7.4 Hz, 1H), 6.52 (d, J = 7.7 Hz, 1H), 4.95 (q, J = 7.2 Hz, 1H), 2.19 - 2.07 (m, 1H), 2.07 - 1.96 (m, 1H), 1.44 (td, J = 12.8, 4.6 Hz, 1H), 1.10 (td, J = 12.8, 4.4 Hz, 1H), 0.86 (s, 9H).

The following examples were prepared in an analogous manner to **Example 32,** starting from the corresponding bisulfite and amine.

| Ex. | Structure | Yield | LCMS | NMR |
|---|---|---|---|---|
| **33** | 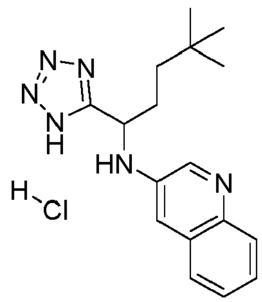 | 62 mg (53% yield, 99% purity) | Method 5, 1.40 min, M+H = 311.4 | $^1$H-NMR (400 MHz, DMSO) δ 8.75 (d, J = 2.8 Hz, 1H), 7.99 - 7.88 (m, 1H), 7.81 (d, J = 7.5 Hz, 1H), 7.68 - 7.49 (m, 3H), 7.40 (s, 1H), 5.16 - 4.99 (m, 1H), 2.12 - 1.93 (m, 2H), 1.49 - 1.32 (m, 1H), 1.25-1.11 (m, 1H), 0.88 (s, 9H). |
| **34** | 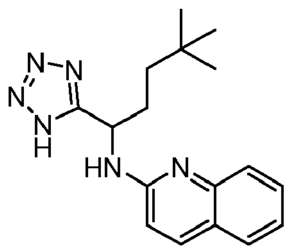 | 74 mg (63% yield, 93% purity) | Method 5, 1.31 min, M+H = 311.4 | $^1$H-NMR (400 MHz, DMSO) δ 7.90 (d, J = 8.9 Hz, 1H), 7.69 (d, J = 7.2 Hz, 1H), 7.63 (d, J = 7.8 Hz, 1H), 7.45 (dd, J = 5.7, 1.6 Hz, 2H), 7.16 (ddd, J = 8.0, 5.8, 2.3 Hz, 1H), 6.89 (d, J = 8.9 Hz, 1H), 5.49 (q, J = 7.0 Hz, 1H), 2.03 - 1.92 (m, 2H), 1.41 - 1.30 (m, 1H), 1.20 - 1.10 (m, 1H), 0.86 (s, 9H). |
| **35** | 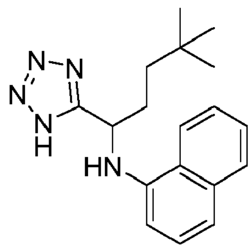 | 28 mg (27% yield, 99% purity) | Method 5, 2.10 min, M+H = 310.2 | $^1$H-NMR (400 MHz, DMSO) δ 8.27 - 8.19 (m, 1H), 7.77 - 7.69 (m, 1H), 7.46 - 7.37 (m, 2H), 7.18 (t, J = 7.9 Hz, 1H), 7.07 (d, J = 8.0 Hz, 1H), 6.54 (d, J = 7.6 Hz, 1H), 6.21 (d, J = 7.3 Hz, 1H), 4.79 (q, J = 6.9 Hz, 1H), 2.14 - 2.01 (m, 1H), 1.95 (td, J = 12.6, 6.2 Hz, 1H), 1.37 (td, J = 12.8, 4.5 Hz, 1H), 1.10 (td, J = 13.0, 4.6 Hz, 1H), 0.84 (s, 9H). |
| **36** | 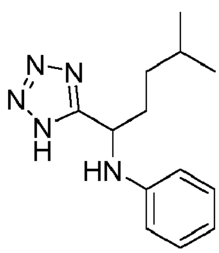 | 23 mg (47% yield, 97% purity) | Method 5, 1.73 min, M+H = 246.2 | $^1$H-NMR (400 MHz, DMSO) δ 7.10 - 6.96 (m, 2H), 6.60 - 6.48 (m, 3H), 6.17 (d, J = 6.8 Hz, 1H), 4.74 (q, J = 6.9 Hz, 1H), 2.02 - 1.82 (m, 2H), 1.52 (hept, J = 6.6 Hz, 1H), 1.34 - 1.22 (m, 1H), 1.16-1.04 (m, 1H), 0.85 (d, J = 5.2 Hz, 3H), 0.83 (d, J = 5.3 Hz, 3H). |

(continued)

| Ex. | Structure | Yield | LCMS | NMR |
|---|---|---|---|---|
| **37** | 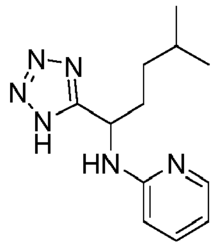 | 98 mg (75% yield, 98% purity) | Method 5, 0.93 min, M+H = 247.3 | $^1$H-NMR (400 MHz, DMSO) δ 8.87 (bs, 1H), 7.96 (d, J = 6.0 Hz, 1H), 7.87 (s, 1H), 7.08 (s, 1H), 6.86 (d, J = 7.4 Hz, 1H), 5.76 (s, 0H), 5.41 (d, J = 6.9 Hz, 1H), 2.08 (d, J = 5.7 Hz, 1H), 1.97 (td, J = 15.1, 8.6 Hz, 1H), 1.56 (dp, J = 13.3, 6.7 Hz, 1H), 1.27 (dtd, J = 9.3, 6.5, 2.4 Hz, 2H), 0.88 (d, J = 1.8 Hz, 3H), 0.86 (d, J = 1.8 Hz, 3H). |
| **38** | 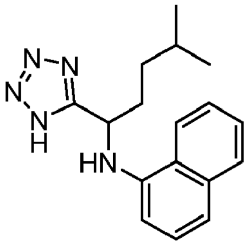 | 17 mg (20% yield, 99% purity) | Method 5, 2.02 min, M+H = 296.2 | $^1$H-NMR (400 MHz, DMSO) δ 8.32 - 8.21 (m, 1H), 7.79 - 7.68 (m, 1H), 7.50 - 7.36 (m, 2H), 7.17 (t, J = 7.9 Hz, 1H), 7.09 (d, J = 8.1 Hz, 1H), 6.51 (d, J = 7.5 Hz, 1H), 6.27 (d, J = 7.2 Hz, 1H), 4.87 (q, J = 7.0 Hz, 1H), 2.12 (tdd, J = 12.1, 7.0, 5.1 Hz, 1H), 1.98 (tdd, J = 12.6, 6.9, 5.2 Hz, 1H), 1.61 - 1.44 (m, J = 6.7 Hz, 1H), 1.40 - 1.24 (m, 1H), 1.14 (tdd, J = 12.4, 6.8, 5.1 Hz, 1H), 0.85 (d, J = 6.6 Hz, 3H), 0.83 (d, J = 6.6 Hz, 3H). |
| **39** | 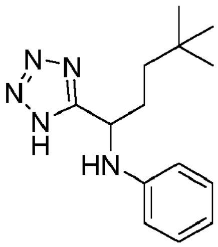 | 50 mg (72% yield, 94% purity) | Method 6, 1.86 min, M+H =360.2 | $^1$H-NMR (400 MHz, DMSO) δ 7.09 - 6.97 (m, 2H), 6.60 - 6.51 (m, 3H), 6.18 (d, J = 6.8 Hz, 1H), 4.71 (q, J = 6.9 Hz, 1H), 1.97 - 1.81 (m, 2H), 1.36 (td, J = 12.7, 4.7 Hz, 1H), 1.07 - 0.99 (m, 1H), 0.84 (s, 9H). |

The following examples were prepared in an analogous manner to **Example 32,** starting from the corresponding aldehyde and amine.

| Ex. | Structure | Yield | LCMS | NMR |
|---|---|---|---|---|
| **40** | 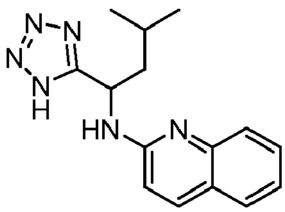 | 95 mg (61% yield, 98% purity) | Method 5, 1.11 min, M+H = 283.3 | $^1$H-NMR (400 MHz, DMSO) δ 7.90 (d, J = 8.9 Hz, 1H), 7.65 (d, J = 7.4 Hz, 1H), 7.63 (dt, J = 8.0, 1.2 Hz, 1H), 7.49 - 7.42 (m, 2H), 7.20 - 7.13 (m, 1H), 6.86 (d, J = 8.9 Hz, 1H), 5.67 - 5.59 (m, 1H), 1.91 (ddd, J = 13.4, 9.2, 6.1 Hz, 1H), 1.86 - 1.77 (m, 1H), 1.73 - 1.61 (m, 1H), 0.96 (d, J = 6.6 Hz, 3H), 0.91 (d, J = 6.5 Hz, 3H). |
| **41** | 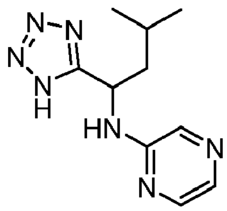 | 35 mg (61% yield, 99% purity) | Method 5, 1.20 min, M+H = 234.2 | $^1$H-NMR (400 MHz, DMSO) δ 7.99 (d, J = 1.5 Hz, 1H), 7.86 (dd, J = 2.9, 1.5 Hz, 1H), 7.62 (d, J = 2.8 Hz, 1H), 7.43 (d, J = 8.0 Hz, 1H), 5.37 - 5.27 (m, 1H), 1.83 - 1.67 (m, 2H), 1.63 - 1.50 (m, 1H), 0.91 (d, J = 6.6 Hz, 3H), 0.85 (d, J = 6.6 Hz, 3H). |

(continued)

| Ex. | Structure | Yield | LCMS | NMR |
|---|---|---|---|---|
| **42** | 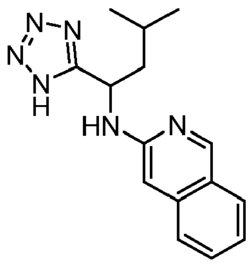 | 17 mg (38% yield, 99% purity) | Method 5, 1.51 min, M+H = 283.3 | $^1$H-NMR (400 MHz, DMSO) δ 16.14 (bs, 1H), 8.83 (s, 1H), 7.79 (d, J = 8.2 Hz, 1H), 7.55 (d, J = 8.4 Hz, 1H), 7.47 (ddd, J = 8.3, 6.6, 1.3 Hz, 1H), 7.18 (ddd, J = 8.1, 6.6, 1.2 Hz, 1H), 7.02 (d, J = 7.7 Hz, 1H), 6.74 (s, 1H), 5.42 (q, J = 7.8 Hz, 1H), 1.93 (ddd, J = 13.3, 9.0, 6.2 Hz, 1H), 1.80 (ddd, J = 13.6, 7.9, 6.2 Hz, 1H), 1.73 - 1.63 (m, 1H), 0.95 (d, J = 6.6 Hz, 3H), 0.89 (d, J = 6.5 Hz, 3H). |
| **43** | 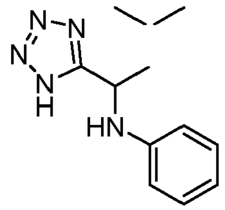 | 14 mg (16% yield, 90% purity) | Method 5, 1.38 min, M+H = 246.2 | $^1$H-NMR (500 MHz, DMSO) δ 0.87 (s, 9H), 1.86 (dd, J = 5.9, 14.1 Hz, 1H), 1.96 (dd, J = 6.8, 14.1 Hz, 1H), 4.81 (q, J = 6.6 Hz, 1H), 6.03 (d, J = 7.1 Hz, 1H), 6.52 - 6.56 (m, 1H), 6.57 - 6.61 (m, 2H), 7.04 (dd, J = 7.1, 8.5 Hz, 2H), 16.14 (s, 1H). |

**Example 44**

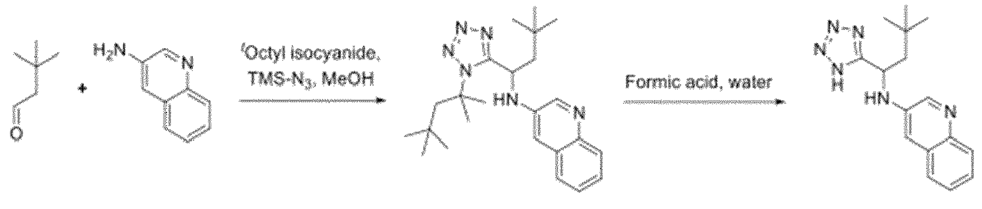


**Int 44-a** **Example 44**

***Synthesis of N-(3,3-dimethyl-1-(1-(2,4,4-trimethylpentan-2-yl)-1H-tetrazol-5-yl)butyl)quinolin-3-amine (Int 44-a)***

A mixture of quinolin-3-amine (123 mg, 1 Eq, 850 μmol) and 3,3-dimethylbutanal (87.8 mg, 110 μL, 97% Wt, 1 Eq, 850 μmol) in MeOH (3.00 mL) was stirred at room temperature for 10 min before methylidyne(2,4,4-trimethylpentan-2-yl)-l4-azane (126 mg, 158 μL, 95% Wt, 1 Eq, 850 μmol) was added. After a further 5 min stirring, TMS-$N_3$ (100 mg, 115 μL, 94% Wt, 0.96 Eq, 816 μmol) was added. The mixture was stirred at 25 °C for 16 h. The reaction mixture was treated with saturated aqueous NaHCOs (10 mL) and ethyl acetate (10 mL). The layers were separated and the aqueous was extracted with ethyl acetate (2x10 mL). The pooled organic layers were dried ($MgSO_4$) filtered and concentrated *in vacuo.* The crude product was purified by chromatography on silica gel (12 g cartridge, 0-100% EtOAc/isohexane) to afford N-(3,3-dimethyl-1-(1-(2,4,4-trimethylpentan-2-yl)-1H-tetrazol-5-yl)butyl)quinolin-3-amine (289 mg, 0.67 mmol, 79% yield, 95% Purity) as a white solid. UPLC (Method 5), 2.19 min; M+H = 409.5. $^1$H-NMR (400 MHz, DMSO) δ 0.62 (s, 9H), 0.93 (s, 9H), 1.85 (d, J = 9.6 Hz, 6H), 1.90 - 2.10 (m, 3H), 2.26 - 2.36 (m, 1H), 5.11 - 5.21 (m, 1H), 6.89 (d, J = 9.4 Hz, 1H), 7.24 (d, J = 2.8 Hz, 1H), 7.34 - 7.47 (m, 2H), 7.68 - 7.75 (m, 1H), 7.79 (d, J = 8.1 Hz, 1H), 8.56 (d, J = 2.7 Hz, 1H).

***Synthesis of N-(3,3-dimethyl-1-(1H-tetrazol-5-yl)butyl)quinolin-3-amine (Example 44)***

A solution of *N*-(3,3-dimethyl-1-(1-(2,4,4-trimethylpentan-2-yl)-1H-tetrazol-5-yl)butyl)quinolin-3-amine (146 mg, 1 Eq, 357 μmol) in formic acid (0.5 mL) and water (1 drop) was heated at 50 °C for 18 h. The solvent was removed *in vacuo.* The crude product was purified by chromatography on silica gel (12 g Redisep Gold cartridge, 0-10% MeOH/DCM) to afford the product which was dried in the vacuum desiccator at 45 °C for 18 hours. *N*-(3,3-dimethyl-1-(1H-tetrazol-5-yl)butyl)quinolin-3-amine (45.0 mg, 0.14 mmol, 40% yield, 94% Purity) was afforded as a white solid. UPLC (Method 5), 1.24 min; M+H = 297.4. $^1$H-NMR (400 MHz, DMSO) δ 0.93 (s, 9H), 1.94 - 2.09 (m, 2H), 4.94 - 5.03 (m, 1H), 6.83 (d, J = 7.4 Hz, 1H), 7.06 (d, J = 2.8 Hz, 1H), 7.30 - 7.43 (m, 2H), 7.56 - 7.63 (m, 1H), 7.77 (dd, J = 1.3, 7.7 Hz, 1H), 8.53 (d,

J = 2.8 Hz, 1H), 16.29 (s, 1H).

The following examples were prepared in an analogous manner to **Example 44,** starting from the corresponding aldehyde and amine.

| Ex. | Structure | Yield | LCMS | NMR |
|---|---|---|---|---|
| **45** | 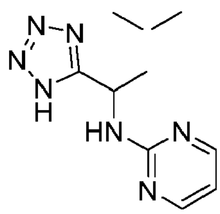 | 50 mg (91% yield, 97% purity) | Method 6, 0.99 min, M+H = 248.3 | 1H-NMR (500 MHz, DMSO) δ 8.33 (d, J = 4.8 Hz, 2H), 7.82 (d, J = 8.1 Hz, 1H), 6.67 (t, J = 4.8 Hz, 1H), 5.47 (td, J = 8.1, 4.0 Hz, 1H), 2.04 (dd, J = 14.3, 8.7 Hz, 1H), 1.93 (dd, J = 14.3, 4.2 Hz, 1H), 0.91 (s, 9H). |
| **46** | 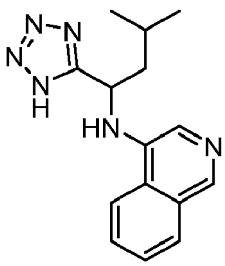 | 64 mg (31% yield, 95% purity) | Method 5, 1.06 min, M+H = 283.3 | 1H-NMR (400 MHz, DMSO) δ 0.91 (d, J = 6.6 Hz, 3H), 0.99 (d, J = 6.6 Hz, 3H), 1.65 - 1.80 (m, 1H), 1.83 - 1.94 (m, 1H), 2.08 - 2.20 (m, 1H), 5.10 - 5.20 (m, 1H), 6.59 (d, J = 7.7 Hz, 1H), 7.60 - 7.68 (m, 2H), 7.69 - 7.77 (m, 1H), 7.93 - 8.00 (m, 1H), 8.37 - 8.45 (m, 1H), 8.59 (s, 1H), 16.25 (s, 1H). |
| **47** | 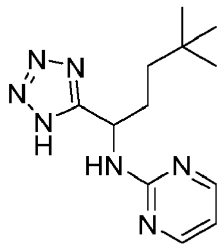 | 14 mg (20% yield, 96% purity) | Method 5, 1.45 min, M+H = 262.3 | 1H-NMR (400 MHz, DMSO) δ 8.28 (d, J = 4.8 Hz, 2H), 7.68 (d, J = 7.6 Hz, 1H), 6.63 (t, J = 4.8 Hz, 1H), 5.22 (q, J = 7.4 Hz, 1H), 1.98 - 1.88 (m, 2H), 1.40 - 1.27 (m, 1H), 1.19 - 1.11 (m, 1H), 0.85 (s, 9H). |
| **48** | 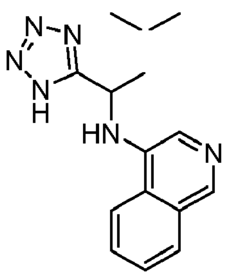 | 35 mg (38% yield, 95% purity) | Method 5, 1.13 min, M+H = 297.4 | 1H-NMR (400 MHz, DMSO) δ 0.94 (s, 9H), 1.98 (dd, J = 4.6, 14.2 Hz, 1H), 2.30 (dd, J = 8.0, 14.2 Hz, 1H), 5.10 - 5.20 (m, 1H), 6.54 (d, J = 7.9 Hz, 1H), 7.64 (ddd, J = 1.1, 6.8, 8.0 Hz, 1H), 7.69 - 7.77 (m, 2H), 7.93 - 8.00 (m, 1H), 8.38 (d, J = 8.5 Hz, 1H), 8.59 (s, 1H), 16.24 (s, 1H). |
| **49** | 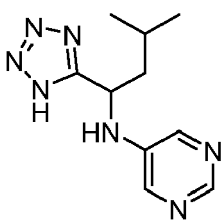 | 20 mg (57% yield, 95% purity) | Method 5, 1.16 min, M+H = 234.0 | 1H-NMR (400 MHz, DMSO) δ 0.87 (d, J = 6.6 Hz, 3H), 0.93 (d, J = 6.6 Hz, 3H), 1.55 - 1.67 (m, 1H), 1.74-1.92 (m, 2H), 4.89 (q, J = 7.6 Hz, 1H), 6.60 (d, J = 7.7 Hz, 1H), 8.14 (s, 2H), 8.37 (s, 1H). |
| **50** | 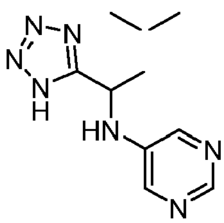 | 29 mg (42 yield, 99% purity) | Method 5, 0.88 min, M+H = 248.2 | 1H-NMR (500 MHz, DMSO) δ 16.05, (bs, 1H), 8.39 (s, 1H), 8.16 (s, 2H), 6.62 (d, J = 7.9 Hz, 1H), 4.99 - 4.90 (m, 1H), 2.01 - 1.89 (m, 2H), 0.89 (s, 9H). |

**Example 51**

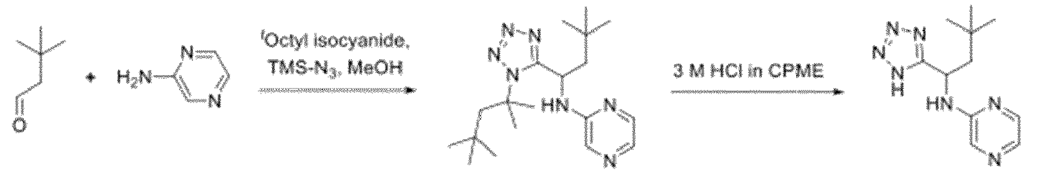


**Int 51-a**

**Example 51**

***Synthesis of N-(3,3-dimethyl-1-(1-(2,4,4-trimethylpentan-2-yl)-1H-tetrazol-5-yl)butyl)pyrazin-2-amine (Int 51-a)***

A mixture of pyrazin-2-amine (97.0 mg, 98% Wt, 1 Eq, 1.00 mmol) and 3,3-dimethylbutanal (101 mg, 127 μL, 99% Wt, 1 Eq, 1.00 mmol) in MeOH (4.00 mL) was stirred at room temperature for 10 min before methylidyne(2,4,4-trimethylpentan-2-yl)-l4-azane (140 mg, 177 μL, 1 Eq, 1.00 mmol) and TMS-$N_3$ (121 mg, 140 μL, 95% Wt, 1 Eq, 1.00 mmol) were added. The mixture was stirred at 25 °C for 16 h. The reaction mixture was quenched with saturated aqueous NaHCOs (15 mL) and extracted with EtOAc (3 x 15 mL). The combined organic layers were washed with brine, dried over $MgSO_4$, filtered and concentrated *in vacuo.* The crude product was purified by chromatography on silica gel (12 g cartridge, 0-100% EtOAc/isohexane) before the product was loaded onto a column of SCX (1.5 g) in MeOH. The column was washed with MeOH and then the product was eluted with 0.7 M ammonia in MeOH. The resultant mixture was concentrated *in vacuo* to afford N-(3,3-dimethyl-1-(1-(2,4,4-trimethylpentan-2-yl)-1H-tetrazol-5-yl)butyl)pyrazin-2-amine (84.0 mg, 0.23 mmol, 23% yield, 97% Purity) as a white solid. UPLC (Method 5), 2.25 min; M+H = 360.6. $^1$H-NMR (400 MHz, DMSO) δ 8.02 (d, J = 1.5 Hz, 1H), 7.97 (dd, J = 2.9, 1.5 Hz, 1H), 7.86 (d, J = 9.2 Hz, 1H), 7.69 (d, J = 2.8 Hz, 1H), 5.98 (td, J = 9.0, 4.3 Hz, 1H), 2.12 (d, J = 15.1 Hz, 1H), 1.96 (dd, J = 14.4, 8.9 Hz, 1H), 1.88 (d, J = 13.9 Hz, 5H), 1.81 (s, 3H), 0.92 (s, 9H), 0.61 (s, 9H).

***Synthesis of N-(3,3-dimethyl-1-(1H-tetrazol-5-yl)butyl)pyrazin-2-amine (Example 51)***

*N*-(3,3-dimethyl-1-(1-(2,4,4-trimethylpentan-2-yl)-1H-tetrazol-5-yl)butyl)pyrazin-2-amine (84.0 mg, 98% Wt, 1 Eq, 229 μmol) was stirred in 3 M HCl in CPME (501 mg, 4.58 mL, 3.00 molar, 60 Eq, 13.7 mmol) at 65 °C for 4 h. The crude was concentrated *in vacuo* before being loaded onto a column of SCX (1.5 g) in MeOH. The column was washed with MeOH and then the product was eluted with 0.7 M ammonia in MeOH. The resultant mixture was concentrated on celite *in vacuo* and purified by chromatography on silica gel (12 g cartridge, 0-20% MeOH/DCM) to afford N-(3,3-dimethyl-1-(1H-tetrazol-5-yl)butyl)pyrazin-2-amine (21.0 mg, 84 μmol, 37% yield, 99% Purity) as a yellow solid. UPLC (Method 5), 1.38 min; M+H = 348.2. $^1$H-NMR (400 MHz, DMSO) δ 8.01 (d, J = 1.5 Hz, 1H), 7.90 (dd, J = 2.9, 1.5 Hz, 1H), 7.74 (d, J = 7.7 Hz, 1H), 7.70 (d, J = 2.9 Hz, 1H), 5.41 (q, J = 6.7 Hz, 1H), 1.93 (d, J = 6.4 Hz, 2H), 0.90 (s, 9H).

The following **Example 52** was prepared in an analogous manner to **Example 51,** starting from the corresponding aldehyde and amine.

| Ex. | Structure | Yield | LCMS | NMR |
|---|---|---|---|---|
| 52 | 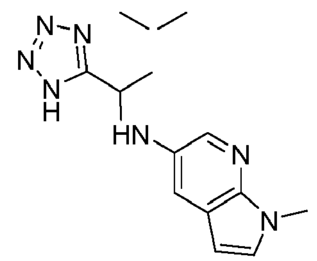 | 25 mg (24% yield, 95% purity) | Method 5, 1.41 min, M+H = 300.4 | $^1$H-NMR (400 MHz, DMSO) δ 0.88 (s, 9H), 1.86 - 2.04 (m, 2H), 3.69 (s, 3H), 4.83 (q, J = 6.6 Hz, 1H), 5.73 (d, J = 7.6 Hz, 1H), 6.18 (d, J = 3.4 Hz, 1H), 7.06 (d, J = 2.5 Hz, 1H), 7.30 (d, J = 3.3 Hz, 1H), 7.84 (d, J = 2.5 Hz, 1H), 16.17 (s, 1H). |

The following **Example 53** was prepared in an analogous manner to **Example 51,** starting from the corresponding bisulfite and amine.

| Ex. | Structure | Yield | LCMS | NMR |
|---|---|---|---|---|
| 53 | 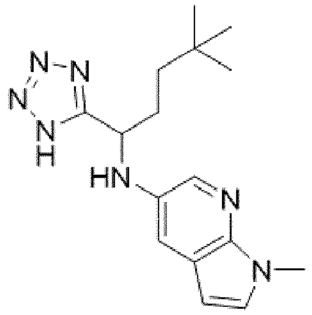 | 9 mg (12% yield, 95% purity) | Method 5, 1.56 min, M+H = 314.5 | $^1$H-NMR (400 MHz, DMSO) δ 0.84 (s, 9H), 1.01 - 1.17 (m, 1H), 1.31 - 1.43 (m, 1H), 1.81 - 2.05 (m, 2H), 3.69 (s, 3H), 4.75 (q, J = 6.8 Hz, 1H), 5.82 (d, J = 7.2 Hz, 1H), 6.17 (d, J = 3.3 Hz, 1H), 7.00 (d, J = 2.5 Hz, 1H), 7.29 (d, J = 3.3 Hz, 1H), 7.86 (d, J = 2.5 Hz, 1H). |

**Example 54**

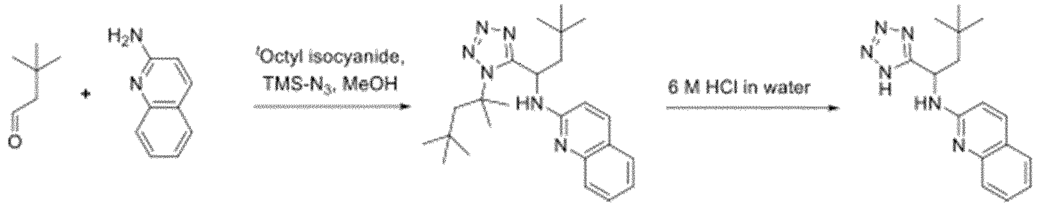


**Int 54-a** **Example 54**

***Synthesis of N-(3,3-dimethyl-1-(1-(2,4,4-trimethylpentan-2-yl)-1H-tetrazol-5-yl)butyl)quinolin-2-amine (Int 54-a)***

A mixture of quinolin-2-amine (169 mg, 98% Wt, 1 Eq, 1.15 mmol) and 3,3-dimethylbutanal (115 mg, 144 μL, 1 Eq, 1.15 mmol) in MeOH (3.00 mL) was stirred at room temperature for 10 min before methylidyne(2,4,4-trimethylpentan-2-yl)-l4-azane (170 mg, 213 μL, 95% Wt, 1 Eq, 1.15 mmol) and TMS-$N_3$ (139 mg, 160 μL, 95% Wt, 1 Eq, 1.15 mmol) were added. The mixture was stirred at 25 °C for 16 h. The reaction mixture was quenched with aq. NaHCOs (15 mL) and extracted with EtOAc (3 x 15 mL). The combined organic layers were washed with brine (15 mL), dried over $MgSO_4$, filtered and concentrated *in vacuo.* The crude product was purified by chromatography on silica gel (12 g cartridge, 0-100% EtOAc/isohexane) to give *N*-(3,3-dimethyl-1-(1-(2,4,4-trimethylpentan-2-yl)-1H-tetrazol-5-yl)butyl)quinolin-2-amine (275 mg, 0.64 mmol, 56% yield, 95% Purity) as a white solid. UPLC (Method 5), 2.60 min; M+H = 409.6. $^1$H-NMR (400 MHz, DMSO) δ 7.87 (d, J = 8.9 Hz, 1H), 7.76 (d, J = 9.2 Hz, 1H), 7.65 - 7.57 (m, 1H), 7.56 - 7.44 (m, 2H), 7.17 (ddd, J = 8.1, 6.3, 1.9 Hz, 1H), 6.84 (d, J = 8.9 Hz, 1H), 6.44 (td, J = 9.1, 4.0 Hz, 1H), 2.30 (d, J = 15.1 Hz, 1H), 2.07 - 1.99 (m, 1H), 1.92 (s, 3H), 1.91 (s, 3H), 1.88 - 1.84 (m, 1H), 1.80 (d, J = 9.2 Hz, 1H), 0.96 (s, 9H), 0.54 (s, 9H).

***Synthesis of N-(3,3-dimethyl-1-(1H-tetrazol-5-yl)butyl)quinolin-2-amine (Example 54)***

A mixture of *N*-(3,3-dimethyl-1-(1-(2,4,4-trimethylpentan-2-yl)-1H-tetrazol-5-yl)butyl)quinolin-2-amine (175 mg, 99% Wt, 1 Eq, 424 μmol) was stirred in HCl in water (928 mg, 4.24 mL, 6.00 molar, 60 Eq, 25.4 mmol) at 75 °C for 5 h. The crude was concentrated *in vacuo* before being diluted with MeCN and evaporated again *in vacuo.* The residue was loaded onto celite and purified by chromatography on silica gel (12 g cartridge, 0-10% MeOH/DCM) to afford N-(3,3-dimethyl-1-(1H-tetrazol-5-yl)butyl)quinolin-2-amine (42 mg, 0.13 mmol, 31% yield, 94% Purity) as a green solid. UPLC (Method 5), 1.20 min; M+H = 297.4. $^1$H-NMR (500 MHz, DMSO) δ 8.09 (bs, 1H), 7.80 - 7.53 (m, 3H), 7.31 (bs, 1H), 7.00 (bs, 1H), 5.83 (bs, 1H), 2.12 - 2.01 (m, 2H), 0.96 (s, 9H).

The following Examples were prepared in an analogous manner to **Example 54,** starting from the corresponding aldehyde and amine.

| Ex. | Structure | Yield | LCMS | NMR |
|---|---|---|---|---|
| **55** | 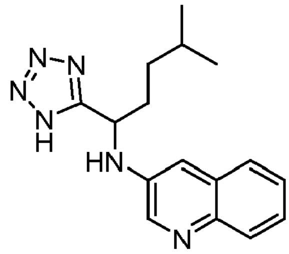 | 12 mg (34% yield, 90% purity) | Method 5, 1.36 min, M+H = 297.4 | $^1$H-NMR (400 MHz, DMSO) δ 0.83 - 0.92 (m, 6H), 1.13 - 1.27 (m, 1H), 1.27 - 1.39 (m, 1H), 1.50 - 1.62 (m, 1H), 1.95 - 2.06 (m, 2H), 4.91 (q, J = 7.1 Hz, 1H), 6.85 (d, J = 7.1 Hz, 1H), 6.97 (d, J = 2.8 Hz, 1H), 7.29 - 7.42 (m, 2H), 7.56 (dd, J = 1.7, 7.7 Hz, 1H), 7.77 (dd, J = 1.6, 7.8 Hz, 1H), 8.57 (d, J = 2.8 Hz, 1H). |
| **56** | 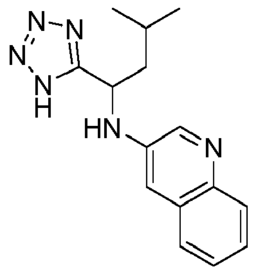 | 48 mg (56% yield, 95% purity) | Method 5, 1.22 min, M+H = 283.3 | $^1$H-NMR (400 MHz, DMSO) δ 0.88 (d, J = 6.6 Hz, 3H), 0.96 (d, J = 6.6 Hz, 3H), 1.64 (hept, J = 6.7 Hz, 1H), 1.73 - 1.97 (m, 2H), 4.89 (q, J = 7.5 Hz, 1H), 6.73 (d, J = 7.6 Hz, 1H), 7.01 (d, J = 2.8 Hz, 1H), 7.26 - 7.40 (m, 2H), 7.55 (dd, J = 1.6, 7.9 Hz, 1H), 7.71 - 7.78 (m, 1H), 8.54 (d, J = 2.8 Hz, 1H). |
| **57** | 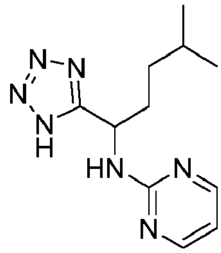 | 25 mg (43% yield, 95% purity) | Method 6, 1.41 min, M+H = 248.2 | $^1$H-NMR (400 MHz, DMSO) δ 0.84 (dd, J = 3.8, 6.6 Hz, 6H), 1.12-1.30 (m, 2H), 1.45 - 1.60 (m, 1H), 1.85 - 2.03 (m, 2H), 5.24 (q, J = 7.4 Hz, 1H), 6.56 - 6.65 (m, 1H), 7.52 (d, J = 7.7 Hz, 1H), 8.27 (d, J = 4.8 Hz, 2H). |
| **58** | 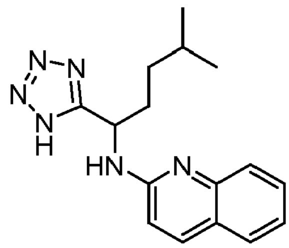 | 19 mg (19% yield, 94% purity) | Method 6, 1.35 min, M+H = 297.1 | $^1$H-NMR (400 MHz, DMSO) δ 0.86 (dd, J = 3.5, 6.6 Hz, 6H), 1.13-1.36 (m, 2H), 1.51 - 1.63 (m, 1H), 1.99 (q, J = 7.7 Hz, 2H), 5.52 (q, J = 7.1 Hz, 1H), 6.89 (d, J = 8.9 Hz, 1H), 7.12 - 7.21 (m, 1H), 7.40 - 7.50 (m, 2H), 7.59 - 7.66 (m, 2H), 7.90 (d, J = 8.9 Hz, 1H). |
| **59** | 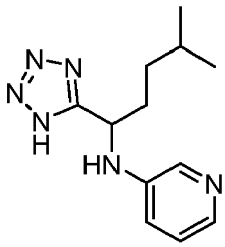 | 31 mg (71% yield, 95% purity) | Method 6, 0.89 min, M+H = 247.3 | $^1$H-NMR (400 MHz, DMSO) δ 0.80 - 0.88 (m, 6H), 1.04 - 1.17 (m, 1H), 1.21 - 1.35 (m, 1H), 1.45 - 1.59 (m, 1H), 1.84 - 2.01 (m, 2H), 4.76 (q, J = 7.1 Hz, 1H), 6.35 (d, J = 7.3 Hz, 1H), 6.85 - 6.92 (m, 1H), 7.02 (dd, J = 4.6, 8.3 Hz, 1H), 7.75 (dd, J = 1.3, 4.7 Hz, 1H), 7.99 (d, J = 2.8 Hz, 1H). |
| **60** | 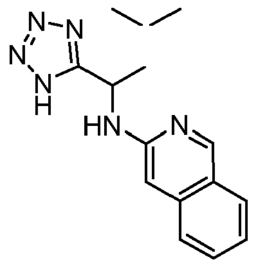 | 18 mg (15% yield, 99% purity) | Method 5, 1.63 min, M+H = 297.4 | $^1$H-NMR (400 MHz, DMSO) δ 16.12 (s, 1H), 8.85 (s, 1H), 7.79 (dd, J = 8.4, 1.1 Hz, 1H), 7.58 - 7.52 (m, 1H), 7.47 (ddd, J = 8.3, 6.6, 1.3 Hz, 1H), 7.18 (ddd, J = 8.0, 6.7, 1.2 Hz, 1H), 6.97 (d, J = 8.0 Hz, 1H), 6.73 (s, 1H), 5.50 (td, J = 7.9, 4.9 Hz, 1H), 2.06 - 1.92 (m, 2H), 0.92 (s, 9H). |

(continued)

| Ex. | Structure | Yield | LCMS | NMR |
|---|---|---|---|---|
| **61** | 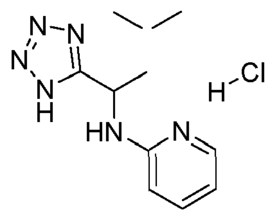 | 71 mg (74% yield, 99% purity) | Method 6, 0.63 min, M+H = 247.2 | $^1$H-NMR (500 MHz, DMSO) δ 8.99, (bs, 1H), 7.98 (d, J = 6.1 Hz, 1H), 7.90 (s, 1H), 7.11 (s, 1H), 6.89 (s, 1H), 5.59 (s, 1H), 2.08 (dd, J = 14.4, 3.4 Hz, 1H), 1.99 (dd, J = 14.4, 8.8 Hz, 1H), 0.96 (s, 9H). |
| **62** | 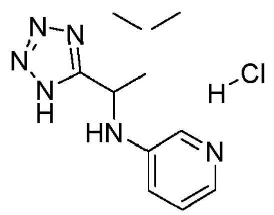 | 98 mg (85% yield, 99% purity) | Method 6, 0.57 min, M+H = 247.3 | $^1$H-NMR (500 MHz, DMSO) δ 16.26 (bs, 1H), 8.22 (d, J = 2.6 Hz, 1H), 8.06 (d, J = 5.2 Hz, 1H), 7.81 - 7.74 (m, 1H), 7.71 (dd, J = 8.8, 5.2 Hz, 1H), 7.67 (d, J = 8.0 Hz, 1H), 5.14 (td, J = 7.6, 4.8 Hz, 1H), 2.04 (dd, J = 14.3, 4.9 Hz, 1H), 1.97 (dd, J = 14.3, 7.6 Hz, 1H), 0.92 (s, 9H). |
| **63** | 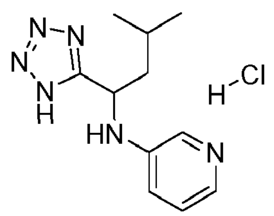 | 123 mg (65% yield, 96% purity) | Method 6, 0.48 min, M+H = 231.2 | $^1$H-NMR (500 MHz, D2O) δ 8.00 (t, J = 3.3 Hz, 1H), 7.97 (d, J = 2.1 Hz, 1H), 7.75 - 7.62 (m, 2H), 5.14 (dd, J = 8.9, 6.0 Hz, 1H), 2.02 (ddd, J = 14.8, 9.0, 6.3 Hz, 1H), 1.90 (ddd, J = 13.9, 8.0, 6.0 Hz, 1H), 1.79 - 1.66 (m, 1H), 0.98 (d, J = 6.7 Hz, 3H), 0.94 (d, J = 6.6 Hz, 3H). |
| **64** | 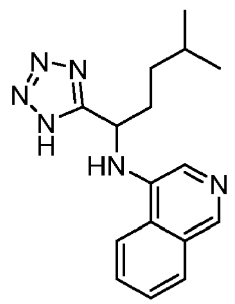 | 8 mg (57% yield, 95% purity) | Method 5, 1.10 min, M+H = 297.4 | $^1$H-NMR (400 MHz, DMSO) δ 0.87 (dd, J = 6.6, 7.8 Hz, 6H), 1.12-1.30 (m, 1H), 1.33 - 1.46 (m, 1H), 1.58 (hept, J = 6.7 Hz, 1H), 1.99 - 2.13 (m, 1H), 2.13 - 2.23 (m, 1H), 5.06 (q, J = 7.2 Hz, 1H), 6.56 (d, J = 7.4 Hz, 1H), 7.59 - 7.68 (m, 2H), 7.68 - 7.77 (m, 1H), 7.93 - 8.00 (m, 1H), 8.40 (dd, J = 1.0, 8.6 Hz, 1H), 8.58 (s, 1H). |
| **65** | 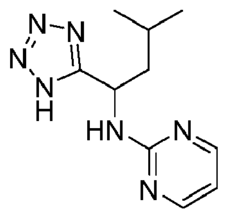 | 21 mg (91% yield, 98% purity) | Method 5, 0.88 min, M+H = 234.2 | $^1$H-NMR (500 MHz, DMSO) δ 15.77 (bs, 1H), 8.28 (d, J = 4.8 Hz, 2H), 7.74 (d, J = 7.8 Hz, 1H), 6.64 (t, J = 4.8 Hz, 1H), 5.38 (ddd, J = 9.7, 7.7, 5.6 Hz, 1H), 1.97 - 1.89 (m, 1H), 1.80 - 1.72 (m, 1H), 1.71 - 1.61 (m, 1H), 0.92 (d, J = 6.6 Hz, 3H), 0.88 (d, J = 6.5 Hz, 3H). |
| **66** | 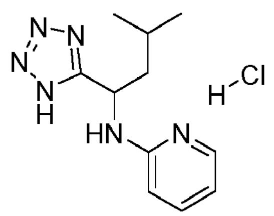 | 62 mg (72% yield, 96% purity) | Method 6, 0.53 min, M+H = 233.1 | $^1$H-NMR (500 MHz, DMSO) δ 8.72 (bs, 1H), 7.95 (d, J = 5.7 Hz, 1H), 7.83 (s, 1H), 7.04 (s, 1H), 6.84 (s, 1H), 5.50 (d, J = 7.5 Hz, 1H), 1.99 - 1.81 (m, 2H), 1.73 (dt, J = 13.2, 6.6 Hz, 1H), 0.95 (d, J = 6.6 Hz, 4H), 0.92 (d, J = 6.6 Hz, 4H). |

(continued)

| Ex. | Structure | Yield | LCMS | NMR |
|---|---|---|---|---|
| **67** | 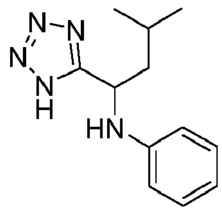 | 36 mg (57% yield, 99% purity) | Method 6, 0.52 min, M+H = 232.2 | $^1$H-NMR (400 MHz, DMSO) δ 16.19 (bs, 1H), 7.10 - 6.95 (m, 2H), 6.61 - 6.52 (m, 3H), 6.13 (d, J = 7.0 Hz, 1H), 4.81 (q, J = 7.3 Hz, 1H), 1.93 - 1.81 (m, 1H), 1.78 - 1.67 (m, 1H), 1.60 (hept, J = 6.7 Hz, 1H), 0.94 (d, J = 6.5 Hz, 3H), 0.86 (d, J = 6.6 Hz, 3H). |
| **68** | 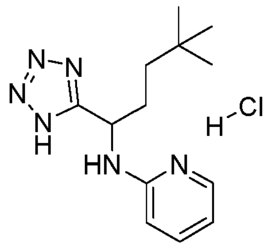 | 13 mg (35% yield, 99% purity) | Method 6, 1.06 min, M+H = 261.3 | $^1$H-NMR (400 MHz, DMSO) δ 7.89 (ddd, J = 5.1, 1.9, 0.8 Hz, 1H), 7.38 (ddd, J = 8.7, 7.1, 1.9 Hz, 1H), 7.07 (d, J = 7.3 Hz, 1H), 6.60 (dt, J = 8.4, 1.0 Hz, 1H), 6.50 (ddd, J = 7.0, 5.1, 1.0 Hz, 1H), 5.25 (q, J = 7.1 Hz, 1H), 1.94 - 1.83 (m, 2H), 1.38 -1.26 (m, 1H), 1.15 - 1.04 (m, 1H), 0.84 (s, 9H) |
| **69** | 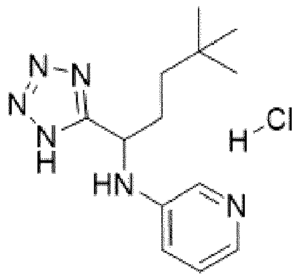 | 82 mg (72% yield, 99% purity) | Method 6, 0.99 min, M+H = 261.3 | $^1$H-NMR (400 MHz, DMSO) δ 8.17 - 8.09 (m, 1H), 8.05 (dd, J = 4.1, 2.4 Hz, 1H), 7.71 - 7.63 (m, 2H), 7.58 (d, J = 7.8 Hz, 1H), 5.06 (q, J = 7.3 Hz, 1H), 2.06 - 1.91 (m, 2H), 1.36 (td, J = 12.3, 5.0 Hz, 1H), 1.20 - 1.07 (m, 1H), 0.87 (s, 9H) |

## Intermediates 70a to d (Int 70 a-d)

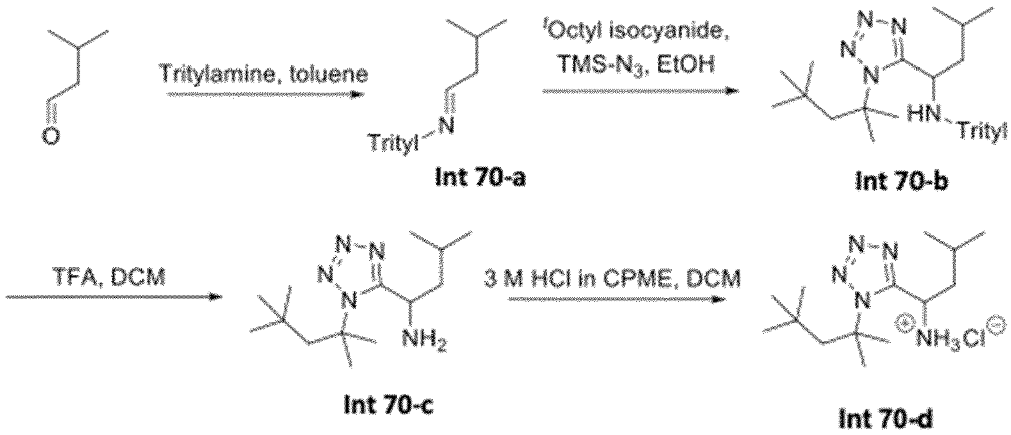


***Synthesis of (E)-3-methyl-N-tritylbutan-1-imine (Int 70-a)***

A mixture of tritylamine (6.48 g, 1 Eq, 25.0 mmol) and isovalderaldehyde (3.23 g, 4.11 mL, 1.5 Eq, 37.5 mmol) in Toluene (25.0 mL) was refluxed for 18 hours using a Dean-Stark distillation device. The reaction was cooled to room temperature, $MgSO_4$ was added, the mixture was stirred over 15 min before being filtered and concentrated *in vacuo* to give (E)-3-methyl-N-tritylbutan-1-imine (8.7 g, 25.0 mmol, 100% yield, 94% Purity) as a yellow oil. UPLC (Method 5), 1.20 min; M+H = 243.2. $^1$H-NMR (400 MHz, $CDCl_3$) δ 7.30 - 7.20 (m, 16H), 2.38 - 2.31 (m, 2H), 1.96 (dp, J = 13.5, 6.7 Hz, 1H), 0.93 (d, J = 6.7 Hz, 6H).

***Synthesis of 3-methyl-1-(1-(2,4,4-trimethylpentan-2-yl)-1H-tetrazol-5-yl)-N-tritylbutan-1-amine (70-b)***

(E)-3-methyl-N-tritylbutan-1-imine (945 mg, 90% Wt, 1 Eq, 2.60 mmol) dissolved in EtOH (8.00 mL) was stirred at room temperature before methylidyne(2,4,4-trimethylpentan-2-yl)-l4-azane (0.37 g, 0.47 mL, 98% Wt, 1.0 Eq, 2.6 mmol) was introduced. After stirring at room temperature for 10 min, TMS-N3 (0.31 g, 0.36 mL, 94% Wt, 0.98 Eq, 2.5 mmol) was added. The mixture was stirred at 25 °C for 3 days. The reaction was diluted with ethyl acetate (20 mL) and washed with saturated solution of NaHCOs (30 mL) followed by brine (50 mL). The organic layer was dried over $MgSO_4$ before being filtered and concentrated *in vacuo.* The crude product was purified by chromatography on silica gel (40 g cartridge, 0-20% EtOAc/isohexane) to afford 3-methyl-1-(1-(2,4,4-trimethylpentan-2-yl)-1H-tetrazol-5-yl)-N-tritylbutan-1-amine (1.17 g, 2.0 mmol, 79% yield) as a colourless gum. UPLC (Method 5), 2.82 min; M+H = n/a. $^1$H-NMR (400 MHz, DMSO) δ 0.53 - 0.61 (m, 3H), 0.63 - 0.90 (m, 12H), 1.27 - 1.69 (m, 11H), 3.39 (d, J = 7.9 Hz, 1H), 4.18 (s, 1H), 7.14 - 7.20 (m, 3H), 7.20 - 7.28 (m, 6H), 7.34 - 7.41 (m, 6H).

***Synthesis of 3-methyl-1-(1-(2,4,4-trimethylpentan-2-yl)-1H-tetrazol-5-yl)butan-1-amine (Int 70-c)***

3-Methyl-1-(1-(2,4,4-trimethylpentan-2-yl)-1H-tetrazol-5-yl)-N-tritylbutan-1-amine (1.16 g, 89% Wt, 1 Eq, 2.03 mmol) was dissolved in DCM (10.0 mL) and stirred at 25 °C before TFA (577 mg, 390 μL, 2.5 Eq, 5.06 mmol) was added dropwise. The mixture was stirred over 1 h 15 min, before being quenched with saturated NaHCOs (30 mL). The layers were separated and the aqueous was further extracted with DCM (25 mL). The pooled organics were washed with brine (25 mL) before being passed through a hydrophobic frit and concentrated *in vacuo.* The crude product was loaded onto a column of SCX (~6 g) in MeOH. The column was washed with MeOH (200 mL) and then the product was eluted with 7 M ammonia in MeOH (150 mL). The resultant mixture was concentrated *in vacuo* to afford 3-methyl-1-(1-(2,4,4-trimethylpentan-2-yl)-1H-tetrazol-5-yl)butan-1-amine (499 mg, 1.8 mmol, 88 % yield, 95% Purity) as a colorless oil. UPLC (Method 5), n/a min; M+H = 268.7. $^1$H-NMR (400 MHz, DMSO) δ 0.72 (s, 9H), 0.91 (dd, J = 6.3, 8.8 Hz, 6H), 1.50 - 1.62 (m, 1H), 1.73 -1.86 (m, 8H), 1.90 (d, J = 15.2 Hz, 1H), 2.06 (d, J = 15.2 Hz, 2H), 4.29 (dd, J = 5.0, 8.7 Hz, 1H).

***Synthesis of 3-methyl-1-(1H-tetrazol-5-yl)butan-1-amine, hydrochloride salt (Int 70-d)***

3-Methyl-1-(1-(2,4,4-trimethylpentan-2-yl)-1H-tetrazol-5-yl)butan-1-amine (499 mg, 1 Eq, 1.87 mmol) was dissolved in dichloromethane (1.00 mL) before being treated with 3 M HCl in CPME (273 mg, 2.50 mL, 3.00 molar, 4.02 Eq, 7.50 mmol). The reaction mixture was stirred at room temperature for 15 min. The solvent was removed *in vacuo* to afford 3-methyl-1-(1-(2,4,4-trimethylpentan-2-yl)-1H-tetrazol-5-yl)butan-1-amine, HCl (559 mg, 1.7 mmol, 94 % yield, 95% Purity) as a white solid. UPLC (Method 5), n/a min; M+H = 268.7. $^1$H-NMR (400 MHz, DMSO) δ 0.79 (s, 9H), 0.93 (d, J = 6.5 Hz, 3H), 0.98 (d, J = 6.4 Hz, 3H), 1.63 - 1.71 (m, 1H), 1.77 - 1.85 (m, 7H), 1.88 - 2.04 (m, 3H), 5.01 (d, J = 10.1 Hz, 1H), 8.75 (s, 3H).

The following Intermediates were prepared in an analogous manner to Intermediate 70-d, starting from the corresponding aldehyde.

| Int. | Structure | Yield | LCMS | NMR |
|---|---|---|---|---|
| **71d** | 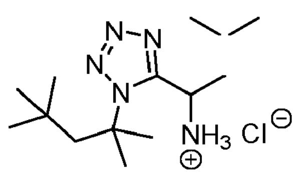 | 480 mg (90% yield, 90% purity) | Method 5, 1.34 min, M+H = 282.5 | $^1$H-NMR (400 MHz, DMSO) δ 0.83 (s, 9H), 0.99 (s, 9H), 1.81 - 2.01 (m, 9H), 2.16 (dd, J = 8.8, 14.9 Hz, 1H), 4.99 (d, J = 7.8 Hz, 1H), 8.80 (s, 3H). |
| **72d** | 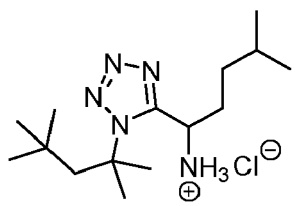 | 1.3 g (100% yield, 93% purity) | Method 5, n/a min, M+H = 282.5 | $^1$H-NMR (400 MHz, DMSO) δ 0.78 (s, 9H), 0.85 (dd, J = 2.3, 6.6 Hz, 6H), 1.16 - 1.37 (m, 2H), 1.42 - 1.66 (m, 1H), 1.81 (d, J = 8.1 Hz, 6H), 1.94 - 2.04 (m, 4H), 5.06 (s, 1H), 8.92 (s, 3H). |

(continued)

| Int. | Structure | Yield | LCMS | NMR |
|---|---|---|---|---|
| **73d** | 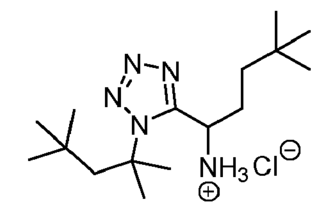 | 422 mg (85% yield, 95% purity) | Method 5, 1.2 min, M+H = 296.5 | $^1$H-NMR (400 MHz, DMSO) δ 0.73 (s, 9H), 0.85 (s, 9H), 0.92 - 1.05 (m, 1H), 1.33 - 1.45 (m, 1H), 1.69 - 1.81 (m, 7H), 1.83 - 1.94 (m, 2H), 1.99 - 2.10 (m, 2H), 4.22 (t, J = 6.9 Hz, 1H). |

**Examples 74, 75 and 76**

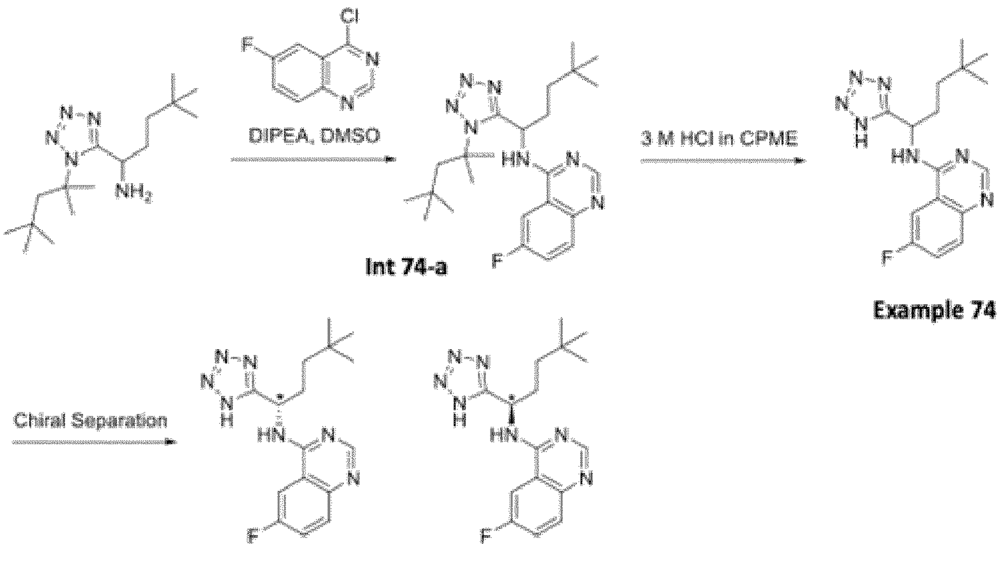


Int 74-a

Example 74

Example 75

Example 76

***Synthesis of N-(4,4-dimethyl-1-(1-(2,4,4-trimethylpentan-2-yl)-1H-tetrazol-5-yl)pentyl)-6-fluoroquinazolin-4-amine (Int 74-a)***

4-Chloro-6-fluoroquinazoline (255 mg, 1.5 Eq, 1.40 mmol) and 4,4-dimethyl-1-(1-(2,4,4-trimethylpentan-2-yl)-1H-tetrazol-5-yl)pentan-1-amine (275 mg, 1 Eq, 931 μmol) were dissolved in DMSO (3.00 mL). DIPEA (301 mg, 405 μL, 2.5 Eq, 2.33 mmol) was introduced and the reaction mixture was stirred at 80 °C for 18 hours. The reaction mixture was diluted with DCM (20 mL) before being washed with 1M HCl (20 mL), saturated aqueous NaHCOs (20 mL) followed by brine (3x25 mL). The organic layer was dried (MgSO4), filtered and concentrated *in vacuo.* The crude product was purified by chromatography on silica gel (12 g cartridge, 0-50% EtOAc/isohexane) to afford *N*-(4,4-dimethyl-1-(1-(2,4,4-trimethylpentan-2-yl)-1H-tetrazol-5-yl)pentyl)-6-fluoroquinazolin-4-amine (377 mg, 0.81 mmol, 87% yield, 95% Purity) as an orange solid. UPLC (Method 5), 2.19 min; M+H = 442.2. $^1$H-NMR (400 MHz, DMSO) δ 0.63 (s, 9H), 0.86 (s, 9H), 1.10 - 1.21 (m, 1H), 1.35- 1.47 (m, 1H), 1.83 (s, 6H), 1.92 -2.17 (m, 4H), 6.11 (q, J = 7.6 Hz, 1H), 7.72 (td, J = 2.7, 8.7 Hz, 1H), 7.80 (dd, J = 5.5, 9.2 Hz, 1H), 8.41 (dd, J = 2.8, 10.2 Hz, 1H), 8.50 (s, 1H), 8.80 (d, J = 8.1 Hz, 1H). 19F NMR (376 MHz, DMSO) δ -113.01.

***Synthesis of N-(4,4-dimethyl-1-(1H-tetrazol-5-yl)pentyl)-6-fluoroquinazolin-4-amine (Example 74)***

*N*-(4,4-dimethyl-1-(1-(2,4,4-trimethylpentan-2-yl)-1H-tetrazol-5-yl)pentyl)-6-fluoroquinazolin-4-amine (375 mg, 1 Eq, 849 μmol) was dissolved in DCM (2 mL) before the addition of 3 M hydrogen chloride in CPME (1.31 g, 12.0 mL, 3.00 molar, 42.4 Eq, 36.0 mmol). The reaction was heated at 70 °C for 3 h before being cooled to room temperature. The solvent was decanted from the residue and the resultant solid was concentrated *in vacuo.* The crude product was

loaded onto a column of SCX (0.5 g) in MeOH. The column was washed with MeOH (100 mL) and then the product was eluted with 0.7 M ammonia in MeOH (150 mL). The resultant mixture was concentrated *in vacuo* to afford the product. The crude product was purified by chromatography on silica gel (12 g cartridge, 0-20% MeOH/DCM) to afford *N*-(4,4-dimethyl-1-(1H-tetrazol-5-yl)pentyl)-6-fluoroquinazolin-4-amine (90.0 mg, 0.26 mmol, 31 %, 95% Purity) as a yellow solid. UPLC (Method 5), 1.27 min; M+H = 330.4. $^1$H-NMR (400 MHz, DMSO) δ 0.85 (s, 9H), 1.18 (td, J = 5.1, 12.7 Hz, 1H), 1.34 (td, J = 4.9, 12.6 Hz, 1H), 1.94 - 2.11 (m, 2H), 5.66 - 5.73 (m, 1H), 7.64 - 7.71 (m, 1H), 7.72 - 7.79 (m, 1H), 8.34 (dd, J = 2.8, 10.2 Hz, 1H), 8.41 (s, 1H), 8.50 (d, J = 8.0 Hz, 1H). 19F NMR (376 MHz, DMSO) δ -113.81.

***Enantiomers resolution of Example 74, N-(4,4-dimethyl-1-(1H-tetrazol-5-yl)pentyl)-6-fluoroquinazolin-4-amine (Examples 75 and 76)***

*N*-(4,4-dimethyl-1-(1-(2,4,4-trimethylpentan-2-yl)-1H-tetrazol-5-yl)pentyl)-6-fluoroquinazolin-4-amine (140 mg, 0.43 mmol) was dissolved to 6 mg/mL in MeOH with sonication and heating. This was filtered and was then purified by chiral SFC on a Waters prep 100 with a PDA and a QDA detectors, 40 °C, 120 bar. The column was a Chiralpak IH 5 μm, 21 mm X 250 mm; flow rate 65 mL/ min of 20% MeOH (no buffer), 80% $CO_2$. The clean fractions were pooled, rinsed with MeOH and concentrated to dryness using a rotary evaporator. The residues were re-dissolved in MeOH transferred into final vials and evaporated on a Biotage V10. The samples were then further dried in a vacuum oven at 30 °C/ 5 mbar for 18 h to afford the first eluting isomer E1 *N*-(4,4-dimethyl-1-(1H-tetrazol-5-yl)pentyl)-6-fluoroquinazolin-4-amine (46 mg, 137 μmol, 30% yield, 98% purity) as a yellow film. LCMS (Method 6), 1.38 min; M+H = 330.2. $^1$H-NMR (400 MHz, DMSO) δ 8.54 (d, J = 7.7 Hz, 1H), 8.43 (s, 1H), 8.32 (dd, J = 10.2, 2.8 Hz, 1H), 7.79 (dd, J = 9.2, 5.6 Hz, 1H), 7.71 (td, J = 8.8, 2.7 Hz, 1H), 5.70 (q, J = 7.8 Hz, 1H), 2.09 (qd, J = 12.5, 6.2 Hz, 2H), 1.37 (td, J = 12.3, 5.1 Hz, 1H), 1.22 (dd, J = 11.9, 4.9 Hz, 1H), 0.87 (s, 9H). 19F NMR (376 MHz, DMSO) δ -113.81.

The second eluting isomer was E2, *N*-(4,4-dimethyl-1-(1H-tetrazol-5-yl)pentyl)-6-fluoroquinazolin-4-amine (43 mg, 129 μmol, 28% yield, 99% purity) as a yellow film. was also afforded as a yellow film. LCMS (Method 6), 1.38 min; M+H = 330.2. $^1$H-NMR (400 MHz, DMSO) δ 8.54 (d, J = 7.7 Hz, 1H), 8.43 (s, 1H), 8.32 (dd, J = 10.2, 2.8 Hz, 1H), 7.79 (dd, J = 9.2, 5.6 Hz, 1H), 7.71 (td, J = 8.8, 2.7 Hz, 1H), 5.70 (q, J = 7.8 Hz, 1H), 2.09 (qd, J = 12.5, 6.2 Hz, 2H), 1.37 (td, J = 12.3, 5.1 Hz, 1H), 1.22 (dd, J = 11.9, 4.9 Hz, 1H), 0.87 (s, 9H). 19F NMR (376 MHz, DMSO) δ -113.81. Chiral UPLC (Method 7), E2, *N*-(4,4-dimethyl-1-(1H-tetrazol-5-yl)pentyl)-6-fluoroquinazolin-4-amine, at 1.2 min, 99.32% EE. z, *N*-(4,4-dimethyl-1-(1H-tetrazol-5-yl)pentyl)-6-fluoroquinazolin-4-amine, at 1.6 min, 97.4% EE. *Predicted stereochemistry not yet confirmed.

The following **Examples 80 to 96** were prepared in an analogous manner to **Examples 74, 75 and 76,** starting from the corresponding amine intermediate ***(Int* 71d** to **73d**) and aryl chloride and using the same resolution method for enantiomer pairs **Examples 93** and **94, 95** and **96** (predicted stereochemistry not yet confirmed).

| Ex. | Structure | Yield | LCMS | NMR |
|---|---|---|---|---|
| **77** | 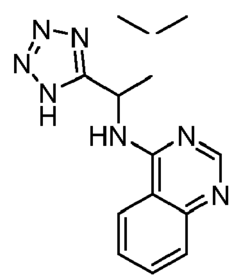 | 56 mg (53% yield, 95% purity) | Method 5, 1.03 min, M+H = 298.4 | $^1$H-NMR (400 MHz, DMSO) δ 0.94 (s, 9H), 2.02 - 2.11 (m, 1H), 2.16 - 226 (m, 1H), 5.97 (t, J = 7.5 Hz, 1H), 7.51 - 7.59 (m, 1H), 7.70 (d, J = 8.3 Hz, 1H), 7.75 - 7.83 (m, 1H), 8.40 (d, J = 8.3 Hz, 1H), 8.46 (s, 1H), 8.60 (d, J = 8.2 Hz, 1H). |
| **78** | 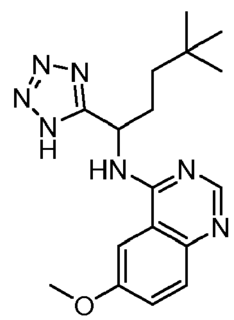 | 30 mg (38% yield, 95% purity) | Method 5, 1.23 min, M+H = 342.5 | $^1$H-NMR (400 MHz, DMSO) δ 0.88 (s, 9H), 1.13 - 1.24 (m, 1H), 1.31 - 1.43 (m, 1H), 1.98 - 2.23 (m, 2H), 3.90 (s, 3H), 5.74 (q, J = 7.6 Hz, 1H), 7.43 (dd, J = 2.7, 9.1 Hz, 1H), 7.65 (d, J = 9.0 Hz, 1H), 7.81 (d, J = 2.7 Hz, 1H), 8.35 (s, 1H), 8.48 (d, J = 7.8 Hz, 1H). |

(continued)

| Ex. | Structure | Yield | LCMS | NMR |
|---|---|---|---|---|
| **79** | 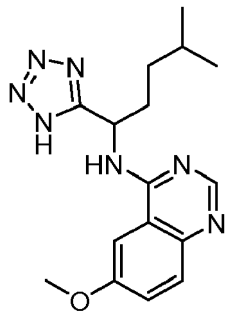 | 37 mg (47% yield, 95% purity) | Method 5, 1.14 min, M+H = 328.4 | $^1$H-NMR (400 MHz, DMSO) δ 0.87 (dd, J = 5.6, 6.6 Hz, 6H), 1.17 - 1.41 (m, 2H), 1.52 - 1.66 (m, 1H), 2.02 - 2.24 (m, 2H), 3.90 (s, 3H), 5.77 (q, J = 7.8 Hz, 1H), 7.43 (dd, J = 2.7, 9.1 Hz, 1H), 7.65 (d, J = 9.1 Hz, 1H), 7.80 (d, J = 2.8 Hz, 1H), 8.35 (s, 1H), 8.44 (d, J = 7.8 Hz, 1H). |
| **80** | 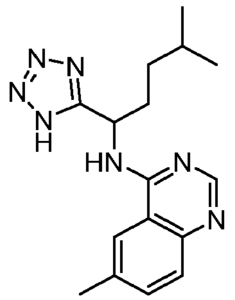 | 46 mg (59% yield, 99% purity) | Method 5, 1.13 min, M+H = 312.4 | $^1$H-NMR (400 MHz, DMSO) δ 8.37 (d, J = 7.5 Hz, 2H), 8.22 (s, 1H), 7.66 - 7.56 (m, 2H), 5.78 - 5.66 (m, 1H), 2.48 (s, 3H), 2.16 - 2.01 (m, 2H), 1.57 (dt, J = 13.3, 6.6 Hz, 1H), 1.37 - 1.16 (m, 2H), 0.87 (d, J = 5.0 Hz, 3H), 0.85 (d, J = 5.1 Hz, 3H). |
| **81** | 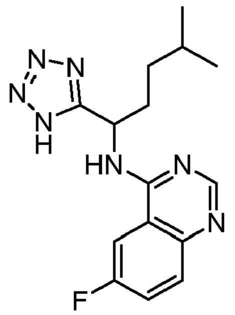 | 72 mg (39% yield, 95% purity) | Method 5, 1.17 min, M+H = 316.4 | $^1$H-NMR (400 MHz, DMSO) δ 0.87 (t, J = 6.3 Hz, 6H), 1.18 - 1.43 (m, 2H), 1.52 - 1.64 (m, 1H), 2.04 - 2.20 (m, 2H), 5.71 (q, J = 7.5 Hz, 1H), 7.74 (td, J = 2.7, 8.7 Hz, 1H), 7.78 - 7.85 (m, 1H), 8.31 (dd, J = 2.8, 10.1 Hz, 1H), 8.44 (s, 1H), 8.63 (d, J = 7.5 Hz, 1H). 19F NMR (376 MHz, DMSO) δ -113.43. |
| **82** | 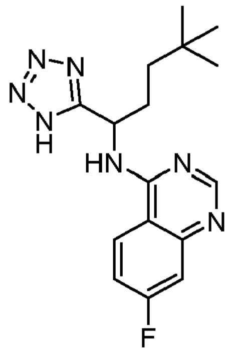 | 43 mg (30% yield, 100% purity) | Method 5, 1.26 min, M+H = 330.4 | $^1$H-NMR (400 MHz, DMSO) δ 16.23 (bs, 1H), 8.75 (d, J = 7.6 Hz, 1H), 8.53 (dd, J = 9.1, 6.0 Hz, 1H), 8.44 (s, 1H), 7.56 - 7.42 (m, 2H), 5.71 (q, J = 7.8 Hz, 1H), 2.11 (ddd, J = 14.2, 10.1, 5.4 Hz, 2H), 1.39 (td, J = 12.2, 5.4 Hz, 1H), 1.22 (td, J = 11.7, 5.3 Hz, 1H), 0.88 (s, 9H). 19F NMR (376 MHz, DMSO) δ -106.02. |
| **83** | 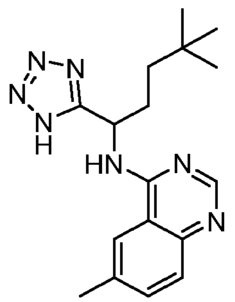 | 32 mg (23% yield, 94% purity) | Method 5, 1.21 min, M+H = 326.4 | $^1$H-NMR (400 MHz, DMSO) δ 8.55 (d, J = 7.6 Hz, 1H), 8.40 (s, 1H), 8.31 (d, J = 8.5 Hz, 1H), 7.52 (s, 1H), 7.40 (dd, J = 8.4, 1.8 Hz, 1H), 5.70 (q, J = 7.5 Hz, 1H), 2.48 (s, 3H), 2.11 (td, J = 11.2, 5.3 Hz, 2H), 1.38 (td, J = 11.7, 5.7 Hz, 1H), 1.25 - 1.19 (m, 1H), 0.87 (s, 9H). |

(continued)

| Ex. | Structure | Yield | LCMS | NMR |
|---|---|---|---|---|
| **84** | 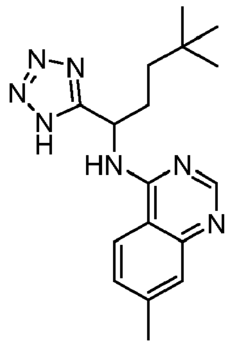 | 25 mg (18% yield, 100% purity) | Method 5, 1.23 min, M+H = 326.4 | $^1$H-NMR (400 MHz, DMSO) δ 8.50 (d, J = 7.7 Hz, 1H), 8.38 (s, 1H), 8.23 (s, 1H), 7.74, - 7.56 (m, 2H), 5.71 (q, J = 7.6 Hz, 1H), 2.49 (s, 3H), 2.17 - 2.03 (m, 2H), 1.38 (td, J =, 11.9, 5.6 Hz, 1H), 1.23 - 1.15 (m, 1H), 0.88 (s, 9H). |
| **85** | 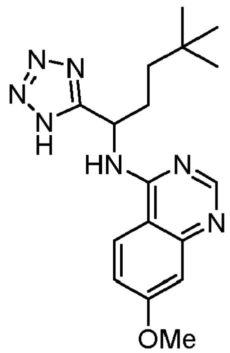 | 28 mg (20% yield, 100% purity) | Method 5, 1.21 min, M+H = 340.3 | $^1$H-NMR (400 MHz, DMSO) δ 8.46 (d, J = 7.7 Hz, 1H), 8.40 - 8.29 (m, 2H), 7.17 (dd, J = 9.1, 2.6 Hz, 1H), 7.11 (d, J = 2.6 Hz, 1H), 5.69 (q, J = 7.7 Hz, 1H), 3.89 (s, 3H), 2.09 (dq, J = 13.3, 6.4 Hz, 2H), 1.37 (td, J = 11.9, 5.5 Hz, 1H), 1.25 - 1.14 (m, 1H), 0.87 (s, 9H). |
| **86** | 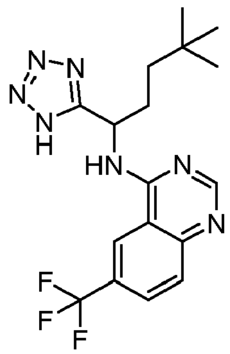 | 39 mg (36% yield, 99% purity) | Method 5, 1.62 min, M+H = 380.4 | $^1$H-NMR (400 MHz, DMSO) δ 9.10 (d, J = 7.6 Hz, 1H), 8.96 (s, 1H), 8.56 (s, 1H), 8.08 (dd, J = 8.9, 2.0 Hz, 1H), 7.90 (d, J = 8.7 Hz, 1H), 5.76 (q, J = 7.5 Hz, 1H), 2.13 (tt, J = 13.6, 5.6 Hz, 2H), 1.40 (td, J = 12.4, 5.0 Hz, 1H), 1.22 (td, J = 12.2, 4.9 Hz, 1H), 0.88 (s, 9H). 19F NMR (376 MHz, DMSO) δ -60.24. |
| **87** | 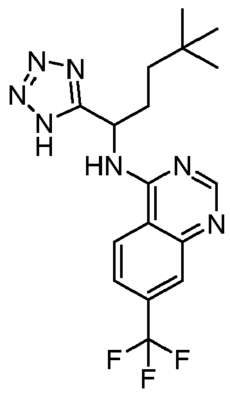 | 40 mg (23% yield, 99% purity) | Method 5, 1.67 min, M+H = 378.3 | $^1$H-NMR (400 MHz, DMSO) δ 9.00 (d, J = 7.7 Hz, 1H), 8.68 (d, J = 8.7 Hz, 1H), 8.55 (s, 1H), 8.05 (s, 1H), 7.91 (d, J = 8.7 Hz, 1H), 5.73 (q, J = 7.5 Hz, 1H), 2.22 - 2.05 (m, 2H), 1.40 (td, J = 12.1, 5.4 Hz, 1H), 1.23 (q, J = 7.1 Hz, 1H), 0.88 (s, 9H). 19F NMR (376 MHz, DMSO) δ - 61.63. |

(continued)

| Ex. | Structure | Yield | LCMS | NMR |
|---|---|---|---|---|
| **88** | 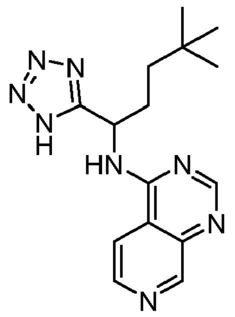 | 62 mg (43% yield, 98% purity) | Method 5, 1.35 min, M+H = 313.3 | $^1$H-NMR (400 MHz, DMSO) δ 9.12 (d, J = 0.8 Hz, 1H), 9.01 (d, J = 7.7 Hz, 1H), 8.67 (d, J = 5.6 Hz, 1H), 8.58 (s, 1H), 8.31 (dd, J = 5.7, 1.0 Hz, 1H), 5.80 - 5.67 (m, 1H), 2.14 (qd, J = 13.1, 6.1 Hz, 2H), 1.39 (td, J = 12.4, 4.9 Hz, 1H), 1.23 (td, J = 12.3, 5.0 Hz, 1H), 0.88 (s, 9H). |
| **89** | 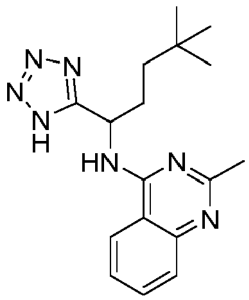 | 44 mg (36% yield, 98% purity) | Method 6, 1.31 min, M+H = 324.3 | $^1$H-NMR (400 MHz, DMSO) δ 8.59 (s, 1H), 8.37 (dd, J = 8.4, 1.4 Hz, 1H), 7.76 (ddd, J = 8.3, 6.9, 1.3 Hz, 1H), 7.62 (dd, J = 8.4, 1.2 Hz, 1H), 7.48 (ddd, J = 8.2, 6.9, 1.3 Hz, 1H), 5.74 (d, J = 9.1 Hz, 1H), 2.40 (s, 3H), 2.19-2.02 (m, 2H), 1.45 - 1.31 (m, 1H), 1.27 - 1.12 (m, 1H), 0.88 (s, 9H). |
| **90** | 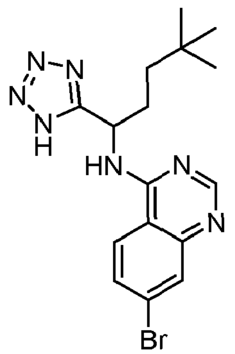 | 103 mg (48% yield, 99% purity) | Method 6, 1.44 min, M+H = 392.4/394.3 | $^1$H-NMR (400 MHz, DMSO) δ 16.25 (s, 1H), 8.82 (d, J = 7.5 Hz, 1H), 8.45 (s, 1H), 8.39 (d, J = 8.9 Hz, 1H), 7.93 (d, J = 2.0 Hz, 1H), 7.77 (dd, J = 8.9, 2.1 Hz, 1H), 5.70 (q, J = 7.5 Hz, 1H), 2.11 (dq, J = 13.9, 7.1 Hz, 2H), 1.39 (td, J = 12.0, 5.4 Hz, 1H), 1.21 (td, J = 11.9, 5.4 Hz, 1H), 0.88 (s, 9H). |
| **91** | 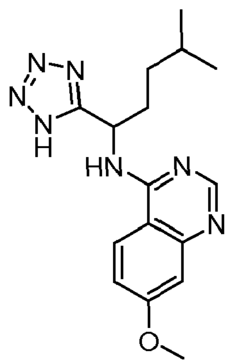 | 15 mg (29% yield, 95% purity) | Method 5, 1.13 min, M+H = 328.4 | $^1$H-NMR (400 MHz, DMSO) δ 0.86 (dd, J = 5.5, 6.6 Hz, 6H), 1.12 - 1.40 (m, 2H), 1.51 - 1.63 (m, 1H), 2.03 - 2.19 (m, 2H), 3.90 (s, 3H), 5.72 (q, J = 7.8 Hz, 1H), 7.11 (d, J = 2.6 Hz, 1H), 7.17 (dd, J = 2.6, 9.1 Hz, 1H), 8.33 (d, J = 9.2 Hz, 1H), 8.37 (s, 1H), 8.47 (d, J = 7.6 Hz, 1H), 16.19 (s, 1H). |

(continued)

| Ex. | Structure | Yield | LCMS | NMR |
|---|---|---|---|---|
| **92** | 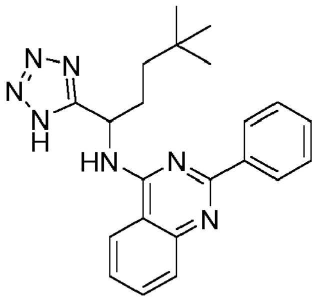 | 73 mg (43% yield, 99% purity) | Method 6, 1.52 min, M+H = 388.4 | $^1$H-NMR (400 MHz, DMSO) δ 16.25 (s, 1H), 8.71 (d, J = 7.2 Hz, 1H), 8.46 (d, J = 8.3 Hz, 1H), 8.38 - 8.31 (m, 2H), 7.86 - 7.76 (m, 2H), 7.55 (ddd, J = 8.2, 5.8, 2.4 Hz, 1H), 7.48 - 7.42 (m, 3H), 5.82 (q, J = 7.2 Hz, 1H), 2.18 (dq, J = 11.2, 5.5 Hz, 2H), 1.52 - 1.41 (m, 1H), 1.33-1.21 (m, 1H), 0.89 (s, 9H). |
| **93** | 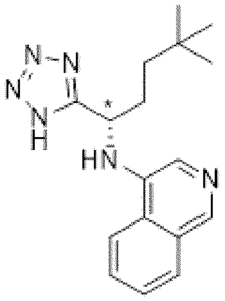 | 48 mg (25% yield, 100% purity. | Method 6, 1.33 min, M+H = 311.3. Chiral method 7, 1.64 min, 99% EE | $^1$H-NMR (400 MHz, DMSO) δ 16.25 (bs, 1H), 8.58 (s, 1H), 8.40 (d, J = 8.5 Hz, 1H), 7.97 (dd, J = 8.1, 1.3 Hz, 1H), 7.73 (ddd, J = 8.4, 6.8, 1.4 Hz, 1H), 7.67 - 7.61 (m, 2H), 6.60 (d, J = 7.4 Hz, 1H), 5.05 (q, J = 7.2 Hz, 1H), 2.17 (tdd, J = 12.5, 7.4, 4.7 Hz, 1H), 2.11 - 1.99 (m, 1H), 1.47 (td, J = 12.8, 4.6 Hz, 1H), 1.12 (td, J = 12.9, 4.4 Hz, 1H), 0.87 (s, 9H). |
| **94** | 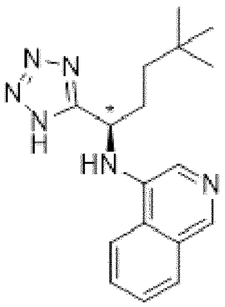 | 67 mg (35% yield, 98% purity) | Method 6, 1.33 min, M+H = 311.2 Chiral method 7, 2.22 min, 96% EE | $^1$H-NMR (400 MHz, DMSO) δ 16.25 (bs, 1H), 8.58 (s, 1H), 8.40 (d, J = 8.5 Hz, 1H), 7.97 (dd, J = 8.1, 1.3 Hz, 1H), 7.73 (ddd, J = 8.4, 6.8, 1.4 Hz, 1H), 7.67 - 7.61 (m, 2H), 6.60 (d, J = 7.4 Hz, 1H), 5.05 (q, J = 7.2 Hz, 1H), 2.17 (tdd, J = 12.5, 7.4, 4.7 Hz, 1H), 2.11 - 1.99 (m, 1H), 1.47 (td, J = 12.8, 4.6 Hz, 1H), 1.12 (td, J = 12.9, 4.4 Hz, 1H), 0.87 (s, 9H). |
| **95** | 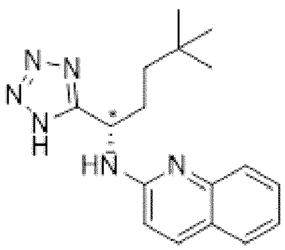 | 25 mg (34% yield, 89% purity) | Method 6, 1.29 min, M+H = 311.4 Chiral method 7, 2.31 min, 98% EE | $^1$H-NMR (400 MHz, DMSO) δ 7.89 (d, J = 8.9 Hz, 1H), 7.61 (t, J = 6.4 Hz, 2H), 7.49 - 7.40 (m, 2H), 7.16 (ddd, J = 8.1, 5.2, 3.0 Hz, 1H), 6.89 (d, J = 8.9 Hz, 1H), 5.50 (q, J = 7.2 Hz, 1H), 2.54 (s, 3H), 2.01 - 1.91 (m, 2H), 1.35 (dt, J = 13.2, 8.0 Hz, 1H), 1.15 (dt, J = 13.1, 7.9 Hz, 1H), 0.86 (s, 9H). |
| **96** | 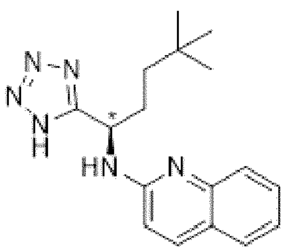 | 27 mg (37% yield, 90% purity) | Method 6, 1.29 min, M+H = 311.4 Chiral method 7, 2.88 min, 94% EE | $^1$H-NMR (400 MHz, DMSO) δ 7.89 (d, J = 8.9 Hz, 1H), 7.61 (t, J = 6.4 Hz, 2H), 7.49 - 7.40 (m, 2H), 7.16 (ddd, J = 8.1, 5.2, 3.0 Hz, 1H), 6.89 (d, J = 8.9 Hz, 1H), 5.50 (q, J = 7.2 Hz, 1H), 2.54 (s, 3H), 2.01 - 1.91 (m, 2H), 1.35 (dt, J = 13.2, 8.0 Hz, 1H), 1.15 (dt, J = 13.1, 7.9 Hz, 1H), 0.86 (s, 9H). |

**Examples 97 and 98**

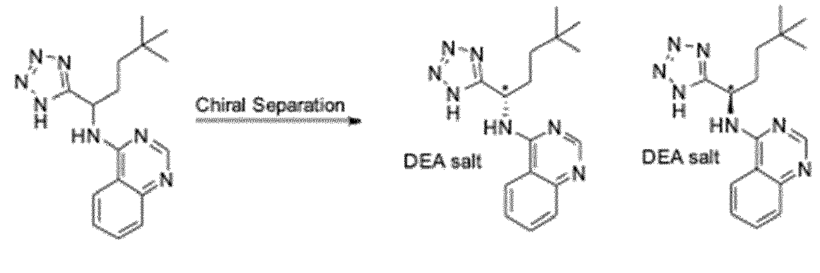


Example 102 Example 97 Example 98

***Enantiomers resolution of Example 102, N-(4,4-dimethyl-1-(1H-tetrazol-5-yl)pentyl)quinazolin-4-amine (Examples* 97 *and 98)***

*N*-(4,4-dimethyl-1-(1H-tetrazol-5-yl)pentyl)quinazolin-4-amine was dissolved to 10 mg/mL in MeOH with sonication. This was filtered and purified by chiral SFC on a Waters prep 100 with a PDA and a QDA detectors, 40 °C, 120 bar. The column was a Chiralpak IG 5 μm, 21 mm X 250 mm; flow rate 65 mL/ min of 25% MeOH (0.1 % DEA), 75% $CO_2$. The clean fractions were pooled, rinsed with MeoH and concentrated to dryness using a Genevac. The residues were re-dissolved in MeOH transferred into final vials and evaporated on a Biotage V10. The isolated pure enantiomers were further dried *in-vacuo,* and in the desiccator to remove the excess of diethylamine to afford the first eluting isomer, E1, *N*-(4,4-dimethyl-1-(1H-tetrazol-5-yl)pentyl)quinazolin-4-amine, diethylamine (78.0 mg, 203 μmol, 29% yield, 100% Purity) as a white solid. UPLC (Method 5), 1.13 min; M+H = 312.4. Chiral method 7, 1.12 min, 99% EE. $^1$H-NMR (400 MHz, DMSO) δ 8.41 (d, J = 7.3 Hz, 2H), 8.24 (d, J = 8.5 Hz, 1H), 7.73 (ddd, J = 8.2, 6.9, 1.3 Hz, 1H), 7.64 (dd, J = 8.4, 1.3 Hz, 1H), 7.46 (ddd, J = 8.3, 6.9, 1.4 Hz, 1H), 5.72 (q, J = 7.9 Hz, 1H), 2.88 (q, J = 7.3 Hz, 4H), 2.09 - 1.88 (m, 2H), 1.31 - 1.17 (m, 2H), 1.14 (t, J = 7.2 Hz, 6H), 0.84 (s, 9H).

Second eluting isomer, E2, *N*-(4,4-dimethyl-1-(1H-tetrazol-5-yl)pentyl)quinazolin-4-amine, diethylamine (82.0 mg, 0.21 mmol, 30% yield, 99% Purity) (isomer 2) was also afforded as a white solid. UPLC (Method 5), 1.13 min; M+H = 312.4. Chiral method 7, 1.35 min, 97% EE. $^1$H-NMR (400 MHz, DMSO) δ 8.42 (d, J = 8.1 Hz, 2H), 8.25 (d, J = 8.4 Hz, 1H), 7.73 (ddd, J = 8.2, 6.9, 1.3 Hz, 1H), 7.64 (dd, J = 8.3, 1.3 Hz, 1H), 7.46 (ddd, J = 8.3, 6.9, 1.4 Hz, 1H), 5.72 (q, J = 7.7 Hz, 1H), 2.90 (q, J = 7.3 Hz, 4H), 2.09 - 1.88 (m, 2H), 1.32 - 1.17 (m, 2H), 1.14 (t, J = 7.3 Hz, 6H), 0.84 (s, 9H).

The following single enantiomers, **Examples 99 and 100,** were obtained in an analogous manner to **Examples 97** and **98.** Predicted stereochemistry not yet confirmed.

| Ex. | Structure | Yield | LCMS | NMR |
|---|---|---|---|---|
| 99 | 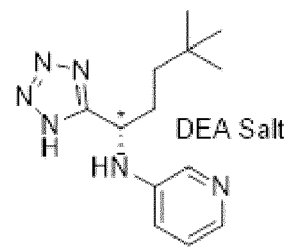 DEA Salt | 6 mg (17% yield, 95% purity) | Method 6, 1.00 min, M+H = 261.3 Chiral method 7, 1.28 min, 93% EE | $^1$H-NMR (400 MHz, DMSO) δ 0.83 (s, 9H), 0.97 - 1.09 (m, 1H), 1.16 (t, J = 7.3 Hz, 6H), 1.27 - 1.38 (m, 1H), 1.79 - 1.93 (m, 2H), 2.92 (q, J = 7.2 Hz, 4H), 4.66 (q, J = 7.2 Hz, 1H), 6.23 (d, J = 7.5 Hz, 1H), 6.86 - 6.94 (m, 1H), 7.00 (dd, J = 4.5, 8.3 Hz, 1H), 7.71 (d, J = 4.6 Hz, 1H), 7.99 (d, J = 2.8 Hz, 1H). |
| 100 | 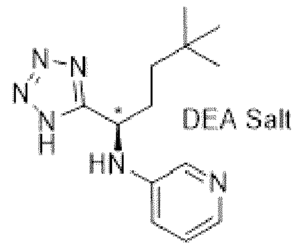 DEA Salt | 6 mg (16% yield, 95% purity) | Method 6, 0.99 min, M+H = 261.3 Chiral method 7, 1.63 min, 88% EE | $^1$H-NMR (400 MHz, DMSO) δ 0.83 (s, 9H), 0.96 - 1.08 (m, 1H), 1.16 (t, J = 7.2 Hz, 6H), 1.28 - 1.37 (m, 1H), 1.81 - 1.93 (m, 2H), 2.92 (q, J = 7.3 Hz, 4H), 4.65 (q, J = 7.1 Hz, 1H), 6.22 (d, J = 7.5 Hz, 1H), 6.90 (d, J = 8.4 Hz, 1H), 7.00 (dd, J = 4.6, 8.3 Hz, 1H), 7.68 - 7.74 (m, 1H), 7.99 (d, J = 2.8 Hz, 1H). |

**Example 101**

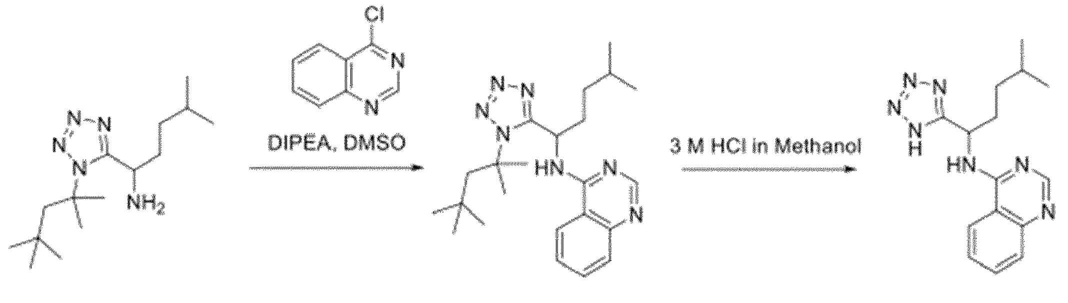


Int 72-a    Int 101-b    Example 101

***Synthesis of N-(4-methyl-1-(1-(2,4,4-trimethylpentan-2-yl)-1H-tetrazol-5-yl)pentyl)quinazolin-4-amine (Int 101-b)***

4-Methyl-1-(1-(2,4,4-trimethylpentan-2-yl)-1H-tetrazol-5-yl)pentan-1-amine, Trifluoroacetic acid (273 mg, 87% Wt, 1 Eq, 600 μmol) was dissolved in DMSO (2.80 mL), then DIPEA (233 mg, 314 μL, 3 Eq, 1.80 mmol) was added, followed by 4-chloroquinazoline (250 mg, 97% Wt, 2.46 Eq, 1.47 mmol). The reaction mixture was stirred over 60 h at 85 °C. The reaction mixture was diluted with DCM (50 mL) before being washed with 1 M HCl (30 mL), saturated aqueous of NaHCOs (30 mL) followed by brine (3x50 mL). The organic layer was dried ($MgSO_4$), filtered and concentrated *in vacuo.* The crude product was purified by chromatography on silica gel (12 g cartridge, 0-100% EtOAc/isohexane) to afford *N*-(4-methyl-1-(1-(2,4,4-trimethylpentan-2-yl)-1H-tetrazol-5-yl)pentyl)quinazolin-4-amine (158 mg, 323 μmol, 53% yield, 83% Purity) as a brown gum. UPLC (Method 5), 1.73 min; M+H = 410.5. $^1$H-NMR (400 MHz, DMSO) δ 8.79 (d, J = 8.1 Hz, 1H), 8.49 (d, J = 8.6 Hz, 2H), 7.80 (ddd, J = 8.3, 6.9, 1.3 Hz, 1H), 7.71 (dd, J = 8.4, 1.3 Hz, 1H), 7.54 (ddd, J = 8.3, 6.9, 1.4 Hz, 1H), 6.21 - 6.12 (m, 1H), 2.23 - 2.03 (m, 3H), 1.95 (d, J = 15.1 Hz, 1H), 1.84 (d, J = 4.0 Hz, 6H), 1.58 (hept, J = 6.6 Hz, 1H), 1.42 - 1.31 (m, 1H), 1.25 - 1.18 (m, 1H), 0.86 (d, J = 3.7 Hz, 3H), 0.84 (d, J = 3.7 Hz, 3H), 0.63 (s, 9H).

**Synthesis of *N*-(4-methyl-1-(1H-tetrazol-5-yl)pentyl)quinazolin-4-amine (Example 101)**

A mixture of *N*-(4-methyl-1-(1-(2,4,4-trimethylpentan-2-yl)-1H-tetrazol-5-yl)pentyl)quinazolin-4-amine (158 mg, 83% Wt, 1 Eq, 320 μmol) was stirred in HCl in MeOH (969 mg, 8.86 mL, 3.00 molar, 83 Eq, 26.6 mmol) at 65 °C over 16 hours. The crude was concentrated *in vacuo,* co-evaporated several times with MeOH and loaded onto celite. The crude was purified by chromatography on silica gel (12 g cartridge, 0-20% MeOH/DCM) to afford N-(4-methyl-1-(1H-tetrazol-5-yl)pentyl)quinazolin-4-amine, HCl (125 mg, 0.37 mmol, 83 %, 98% Purity) as an off-white solid. This was loaded onto a column of SCX (750 mg) in MeOH. The column was washed with MeOH and then the product was eluted with 0.7 M ammonia in MeOH. The resultant mixture was concentrated *in vacuo* to afford *N*-(4-methyl-1-(1H-tetrazol-5-yl)pentyl)quinazolin-4-amine (21.0 mg, 70 μmol, 58% yield, 99% Purity) as an off-white solid. UPLC (Method 5), 1.04 min; M+H = 298.4. $^1$H-NMR (400 MHz, DMSO) δ 8.49 - 8.38 (m, 3H), 7.77 (ddd, J = 8.2, 6.9, 1.3 Hz, 1H), 7.68 (dd, J = 8.5, 1.3 Hz, 1H), 7.52 (ddd, J = 8.3, 6.9, 1.4 Hz, 1H), 5.79 - 5.69 (m, 1H), 2.16 - 1.95 (m, 2H), 1.56 (dt, J = 13.3, 6.6 Hz, 1H), 1.36 - 1.17 (m, 2H), 0.86 (d, J = 4.5 Hz, 3H), 0.84 (d, J = 4.5 Hz, 3H).

The following Examples were prepared in an analogous manner to **Example 101,** starting from the corresponding amine intermediate **Int 73d** and heteroaryl chloride.

| Ex. | Structure | Yield | LCMS | NMR |
|---|---|---|---|---|
| **102** | 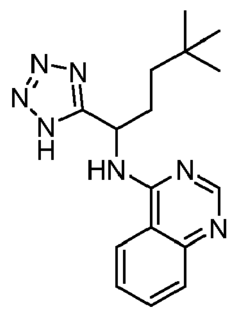 | 40 mg (53% yield, 99% purity) | Method 5, 1.13 min, M+H = 312.4 | $^1$H-NMR (400 MHz, DMSO) δ 8.66 (d, J = 7.7 Hz, 1H), 8.52 - 8.34 (m, 2H), 7.81 (ddd, J = 8.3, 6.9, 1.3 Hz, 1H), 7.72 (dd, J = 8.4, 1.3 Hz, 1H), 7.57 (ddd, J = 8.2, 6.9, 1.4 Hz, 1H), 5.72 (q, J = 7.6 Hz, 1H), 2.22 - 1.99 (m, 2H), 1.48 - 1.31 (m, 1H), 1.30 - 1.15 (m, 1H), 0.87 (s, 9H). |

(continued)

| Ex. | Structure | Yield | LCMS | NMR |
|---|---|---|---|---|
| 103 | 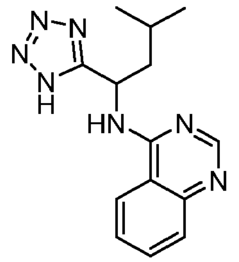 | 29 mg (46% yield, 100% purity) | Method 5, 0.92 min, M+H = 284.3 | $^1$H-NMR (400 MHz, DMSO) δ 8.54 - 8.45 (m, 1H), 8.46 - 8.37 (m, 2H), 7.77 (ddd, J = 8.3, 6.9, 1.3 Hz, 1H), 7.68 (dd, J = 8.5, 1.3 Hz, 1H), 7.52 (ddd, J = 8.3, 6.9, 1.4 Hz, 1H), 5.90 (td, J = 8.4, 5.5 Hz, 1H), 2.11 - 1.96 (m, 1H), 1.96 - 1.84 (m, 1H), 1.78 - 1.60 (m, 1H), 0.94 (d, J = 6.6 Hz, 3H), 0.91 (d, J = 6.6 Hz, 3H). |

**Example 104**

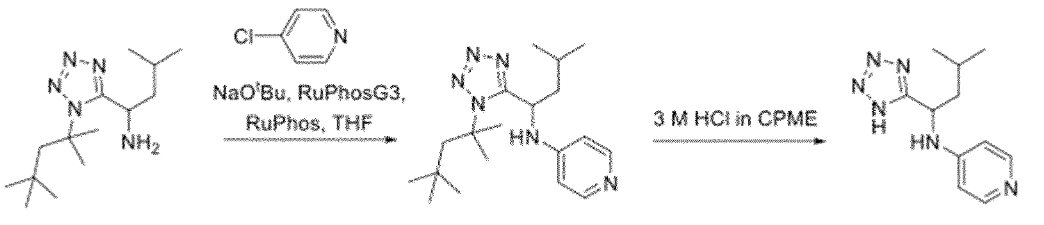


**Int 70-c** **Int 104-b** **Example 104**

***Synthesis of N-(3-methyl-1-(1-(2,4,4-trimethylpentan-2-yl)-1H-tetrazol-5-yl)butyl)pyridin-4-amine (Int 104-b)***

3-Methyl-1-(1-(2,4,4-trimethylpentan-2-yl)-1H-tetrazol-5-yl)butan-1-amine, **Int 70-c,** (150 mg, 1 Eq, 561 μmol), 4-bromopyridine hydrochloride (131 mg, 1.2 Eq, 673 μmol), sodium tert-butoxide (172 mg, 3.2 Eq, 1.79 mmol) and RuPhos (11.0 mg, 0.196 Eq, 23.6 umol) were taken up in THF (3.00 mL). After sparing with $N_2$ for 2 min, RuPhos G3 Precatalyst (47.0 mg, 0.100 Eq, 56.2 μmol) was added. The mixture was sparged with $N_2$ for 2 min before being heated to 75 °C for 15 h. The reaction mixture was cooled to room temperature and diluted with EtOAc (10 mL) and water (10 mL) was added. The layers were separated, and the organic layer was washed with saturated aqueous NaHCOs (15 mL) and brine (15 mL). The organic layer was dried over Magnesium sulfate, filtered and concentrated *in vacuo.* The crude product was purified by chromatography on silica gel (12 g cartridge, 0-10% MeOH/DCM) to afford N-(3-methyl-1-(1-(2,4,4-trimethylpentan-2-yl)-1H-tetrazol-5-yl)butyl)pyridin-4-amine (68.0 mg, 0.19 mmol, 33% yield, 94% Purity) as a yellow gum. UPLC (Method 5), 1.34 min; M+H = 345.5. $^1$H-NMR (400 MHz, DMSO) δ 0.65 (s, 9H), 0.89 (d, J = 6.6 Hz, 3H), 0.95 (d, J = 6.6 Hz, 3H), 1.53 - 1.64 (m, 1H), 1.75 - 1.80 (m, 6H), 1.80 - 2.04 (m, 4H), 5.09 (q, J = 7.6 Hz, 1H), 6.61 - 6.67 (m, 2H), 7.09 (d, J = 9.0 Hz, 1H), 8.04 - 8.10 (m, 2H).

***Synthesis of N-(3-methyl-1-(1H-tetrazol-5-yl)butyl)pyridin-4-amine (Example 104)***

*N*-(3-methyl-1-(1-(2,4,4-trimethylpentan-2-yl)-1H-tetrazol-5-yl)butyl)pyridin-4-amine (68.0 mg, 1 Eq, 197 μmol) was dissolved in DCM (0.5 mL) before the addition of 3 M HCl in CPME (273 mg, 2.50 mL, 3.00 molar, 38.0 Eq, 7.50 mmol). The reaction was heated at 70 °C for 3 h before being cooled to room temperature. The solid was collected by filtration and was washed with MTBE (10 mL). The crude product was loaded onto a column of SCX (0.5 g) in MeOH. The column was washed with MeOH (50 mL) and then the product was eluted with 0.7 M ammonia in MeOH (30 mL). The resultant mixture was concentrated *in vacuo* to afford the product which was treated with MTBE (3 mL) and the solvent was removed in vacuo. The resultant solid was dried in the vacuum desiccator at 45 °C for 15 h. *N*-(3-methyl-1-(1H-tetrazol-5-yl)butyl)pyridin-4-amine (31.0 mg, 0.13 mmol, 64% yield, 95% Purity) was afforded as a white solid. UPLC (Method 5), 0.75 min; M+H = 233.3. $^1$H-NMR (400 MHz, DMSO) δ 0.85 (dd, J = 1.6, 6.6 Hz, 3H), 0.92 (dd, J = 1.6, 6.6 Hz, 3H), 1.46 - 1.59 (m, 1H), 1.78 - 1.92 (m, 2H), 4.89 (q, J = 7.6 Hz, 1H), 6.86 (d, J = 6.6 Hz, 2H), 8.06 (d, J = 6.6 Hz, 2H), 8.62 (d, J = 7.5 Hz, 1H).

The following **Example 105** was prepared in an analogous manner to **Example 104,** starting from the amine Int **70-c** or **d** and aryl chloride.

| Ex. | Structure | Yield | LCMS | NMR |
|---|---|---|---|---|
| **105** | 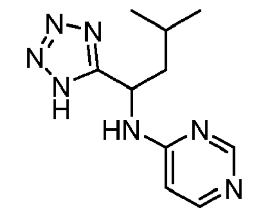 | 15 mg (27% yield, 95% purity) | Method 5, 0.66 min, M+H = 234.3 | $^1$H-NMR (400 MHz, DMSO) δ 0.88 (d, J = 6.6 Hz, 3H), 0.93 (d, J = 6.6 Hz, 3H), 1.54 - 1.69 (m, 1H), 1.71 - 1.92 (m, 2H), 5.48 (s, 1H), 6.58 (dd, J = 1.3, 6.0 Hz, 1H), 7.98 (d, J = 7.7 Hz, 1H), 8.07 (d, J = 5.9 Hz, 1H), 8.39 (d, J = 1.2 Hz, 1H). |

**Example 106**

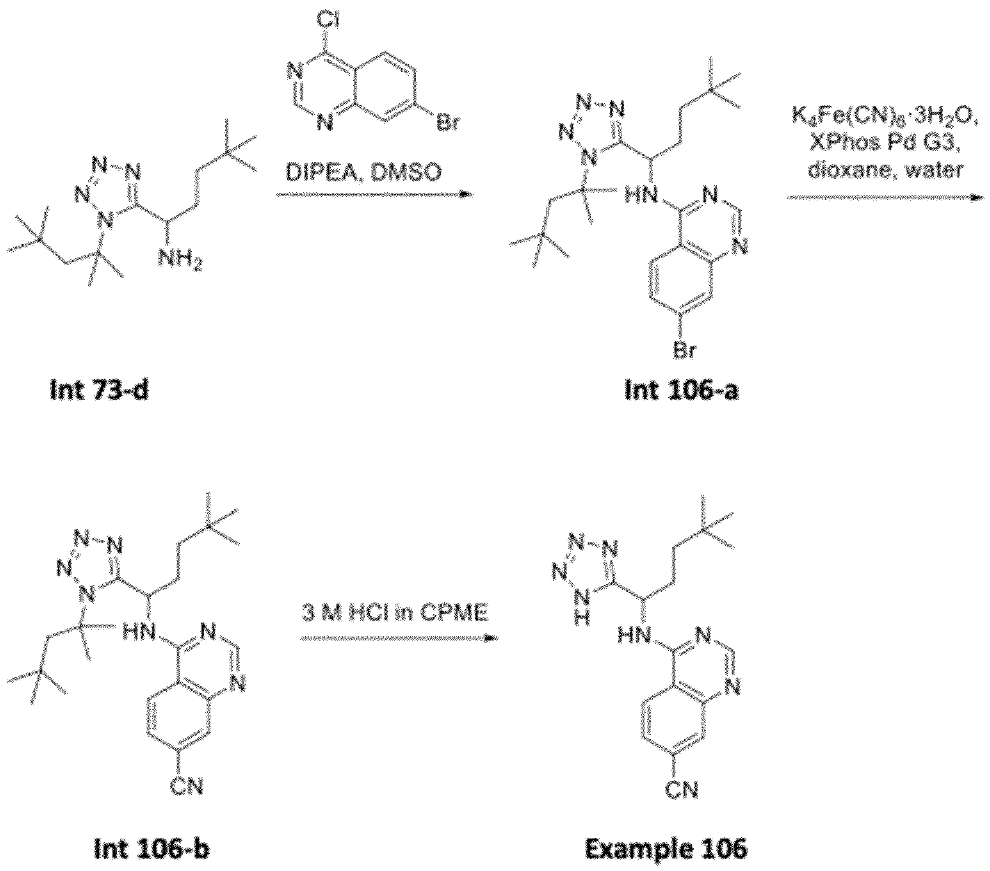


Int 73-d

Int 106-a

Int 106-b

Example 106

***Synthesis of 7-bromo-N-(4,4-dimethyl-1-(1-(2,4,4-trimethylpentan-2-yl)-1H-tetrazol-5-yl)pentyl)quinazolin-4-amine (Int 106-a)***

4,4-Dimethyl-1-(1-(2,4,4-trimethylpentan-2-yl)-1H-tetrazol-5-yl)pentan-1-amine, Trifluoroacetic acid (500 mg, 99% Wt, 1 Eq, 1.21 mmol) was dissolved in DMSO (2.90 mL) and DIPEA (469 mg, 632 μL, 3 Eq, 3.63 mmol) was added. 7-Bromo-4-chloroquinazoline (601 mg, 98% Wt, 2 Eq, 2.42 mmol) was introduced and the reaction mixture was stirred at 85 °C for 16 h. The mixture was diluted with DCM (25 mL), and the solid was filtered. The mother liquor was diluted with DCM (50 mL) before being washed with 1M HCl (30 mL), a saturated solution of NaHCOs (30 mL) followed by brine (3x50 mL). The organic layer was dried ($MgSO_4$), filtered and concentrated *in vacuo.* The crude product was purified by chromatography on silica gel (solid load on Celite, 0-40% then 40-100% EtOAc/n-heptane) to afford the product which was triturated in the minimum amount of n-heptane to afford 7-bromo-N-(4,4-dimethyl-1-(1-(2,4,4-trimethylpentan-2-yl)-1H-tetrazol-5-yl)pentyl)quinazolin-4-amine (426 mg, 0.84 mmol, 69% yield, 99% Purity) as a white solid.

UPLC (Method 5), 2.45 min; M+H = 500.0/502.1. $^1$H-NMR (400 MHz, DMSO) δ 8.99 (d, J = 8.0 Hz, 1H), 8.56

- 8.42 (m, 2H), 7.92 (d, J = 2.0 Hz, 1H), 7.73 (dd, J = 8.9, 2.1 Hz, 1H), 6.10 (td, J = 8.2, 5.8 Hz, 1H), 2.21 - 2.01 (m, 3H), 1.96 (d, J = 15.0 Hz, 1H), 1.83 (s, 6H), 1.41 (td, J = 12.7, 4.8 Hz, 1H), 1.15 (td, J = 12.6, 4.1 Hz, 1H), 0.86 (s, 9H), 0.64 (s, 9H).

***Synthesis of 4-((4,4-dimethyl-1-(1-(2,4,4-trimethylpentan-2-yl)-1H-tetrazol-5-yl)pentyl)amino)quinazoline-7-carbonitrile (Int 106-b)***

7-Bromo-N-(4,4-dimethyl-1-(1-(2,4,4-trimethylpentan-2-yl)-1H-tetrazol-5-yl)pentyl)quinazolin-4-amine (120.0 mg, 1 Eq, 238.8 μmol), XPhos Pd G3 (12.13 mg, 0.06 Eq, 14.33 μmol) and potassium ferrocyanide trihydrate (50.43 mg, 0.5 Eq, 119.4 μmol) were combined under an atmosphere of nitrogen. A mixture of 1,4-Dioxane (1600 μL) Water (800.0 μL) was introduced and the solvent was sparged with nitrogen for 2 min. The reaction mixture was stirred at 80 °C for 18 hours. Upon cooling, the reaction mixture was filtered through a pad of Celite, washing through with ethyl acetate (10 mL). The filtrate was washed with brine (5 mL), the organic layer was dried over $MgSO_4$, filtered, and concentrated *in vacuo*. The crude product was purified by chromatography on silica gel (dry load on Celite, 12 g cartridge, 0-40 EtOAc/heptane) to afford 4-((4,4-dimethyl-1-(1-(2,4,4-trimethylpentan-2-yl)-1H-tetrazol-5-yl)pentyl)amino)quinazoline-7-carbonitrile (61.0 mg, 0.13 mmol, 56% yield, 99% Purity) as an off-white solid. UPLC (Method 5), 2.40 min; M+H = 449.5. $^1$H-NMR (400 MHz, DMSO) δ 9.18 (d, J = 7.9 Hz, 1H), 8.70 (d, J = 8.6 Hz, 1H), 8.60 (s, 1H), 8.26 (d, J = 1.7 Hz, 1H), 7.92 (dd, J = 8.5, 1.7 Hz, 1H), 6.11 (q, J = 7.6 Hz, 1H), 2.10 (t, J = 15.7 Hz, 3H), 2.01 -1.94 (m, 1H), 1.84 (s, 6H), 1.42 (td, J = 12.7, 4.8 Hz, 1H), 1.17 (dt, J = 12.8, 6.3 Hz, 1H), 0.86 (s, 9H), 0.65 (s, 9H).

***Synthesis of 4-((4,4-dimethyl-1-(1H-tetrazol-5-yl)pentyl)amino)quinazoline-7-carbonitrile (Example 106)***

A mixture of 4-((4,4-dimethyl-1-(1-(2,4,4-trimethylpentan-2-yl)-1H-tetrazol-5-yl)pentyl)amino)quinazoline-7-carbonitrile, **Int 105-b,** (60.0 mg, 99% Wt, 1 Eq, 132 μmol) was stirred in HCl in CPME (290 mg, 2.65 mL, 3.00 molar, 60 Eq, 7.94 mmol) at 70 °C for 1 h. The crude was concentrated *in vacuo* and co-evaporated with iPrOH. The crude product was loaded onto a column of SCX (0.5 g) in MeOH. The column was washed with MeOH and then the product was eluted with 0.7 M ammonia in MeOH. The resultant mixture was *concentrated in vacuo.* The crude product was purified by chromatography on silica gel (solid load on Celite, 4 g cartridge, 0-20% MeOH/DCM) to afford 4-((4,4-dimethyl-1-(1H-tetrazol-5-yl)pentyl)amino)quinazoline-7-carbonitrile (18.0 mg, 52.7 μmol, 39 % yield, 98% Purity) as a yellow solid.

UPLC (Method 6), 1.49 min; M+H = 335.3. $^1$H-NMR (400 MHz, DMSO) δ 8.95 (d, J = 7.7 Hz, 1H), 8.62 (d, J = 8.6 Hz, 1H), 8.54 (s, 1H), 8.25 (d, J = 1.6 Hz, 1H), 7.93 (dd, J = 8.5, 1.7 Hz, 1H), 5.72 (q, J = 7.7 Hz, 1H), 2.12 (qd, J = 12.6, 6.2 Hz, 2H), 1.38 (td, J = 12.4, 5.0 Hz, 1H), 1.22 (td, J = 12.2, 5.0 Hz, 1H), 0.87 (s, 9H).

The following **Example 107** was prepared in an analogous manner to **Example 106,** starting from the amine Int **73-a** and the corresponding heteroaryl chloride.

| Ex. | Structure | Yield | LCMS | NMR |
|---|---|---|---|---|
| **107** | 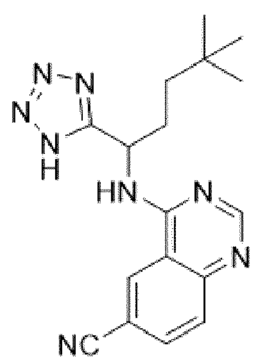 | 21 mg (46% yield, 95% purity) | Method 5, 1.44 min, M+H = 337.4 | $^1$H-NMR (400 MHz, DMSO) δ 9.08 (d, J = 1.8 Hz, 1H), 8.95 (d, J = 7.7 Hz, 1H), 8.55 (s, 1H), 8.12 (dd, J = 8.7, 1.7 Hz, 1H), 7.83 (d, J = 8.7 Hz, 1H), 5.77 - 5.67 (m, 1H), 2.17 - 1.99 (m, 2H), 1.39 (td, J = 12.5, 5.0 Hz, 1H), 1.24 - 1.17 (m, 1H), 0.88 (s, 9H). |

If the stereochemistry is not specified in the structures of the examples above, the compounds were prepared as a racemic mixture.

The following examples can be prepared in an analogous manner to the previous examples.

| Example | Structure |
|---|---|
| 108 | 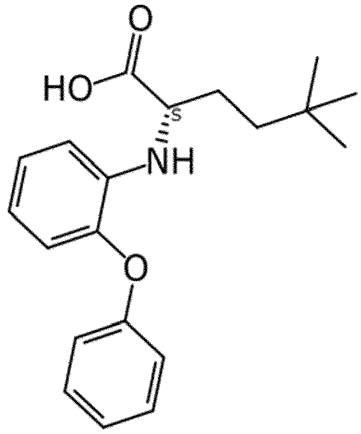 |
| 109 | 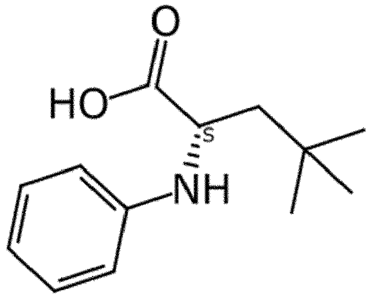 |
| 110 | 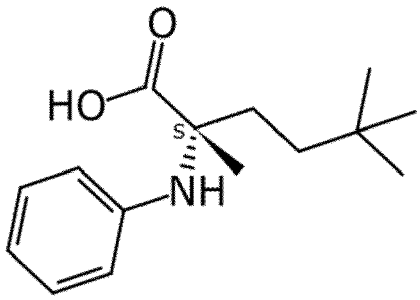 |
| 111 | 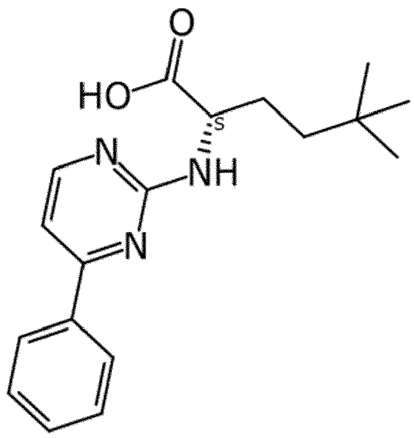 |

(continued)

| Example | Structure |
|---|---|
| 112 | 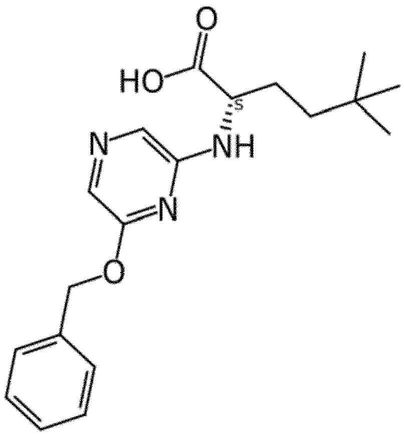 |
| 113 | 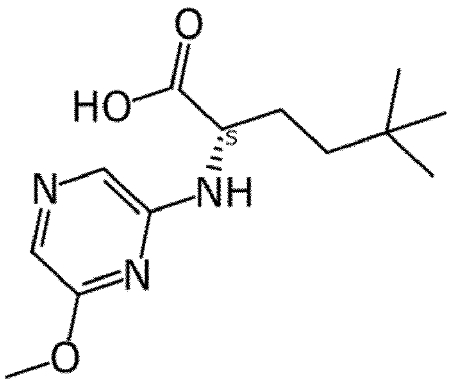 |
| 114 | 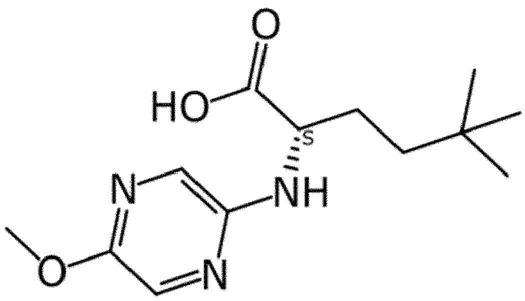 |
| 115 | 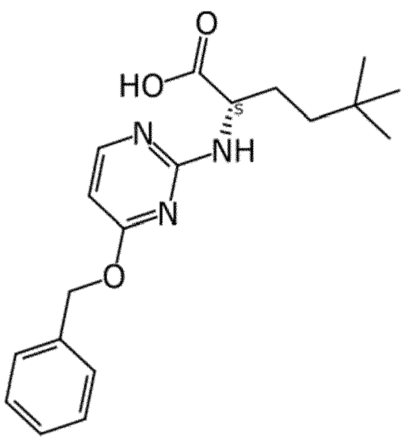 |

(continued)

| Example | Structure |
| --- | --- |
| 116 | 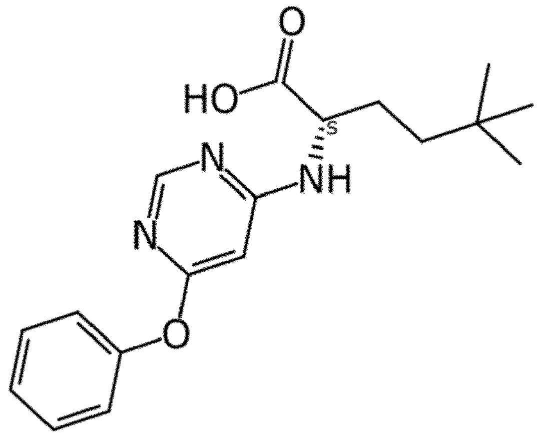 |
| 117 | 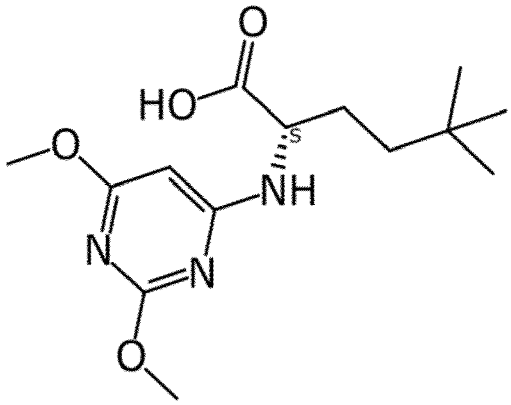 |

**BIOLOGICAL DATA**

**Neurotensin scintillation proximity assay**

The exemplified compounds of the invention were tested in a Neurotensin (NTS) scintillation proximity assay (SPA). The $IC_{50}$ data is shown in the table below. NTS, which is a 13 amino acid neuropeptide, is a sortilin ligand. The $IC_{50}$ is a measure of the amount of the compound required to inhibit the binding of NTS to sortilin by 50%. The skilled person will recognise that the lower the $IC_{50}$ value, the less of the compound needed to achieve the desired effect, and as a result, the chances of undesirable off-target effects are reduced.

Compound affinity was determined by measuring the displacement of [$^3$H]-neurotensin binding to *h*-Sortilin in SPA format. Total volume of 40 μl in 50 mM HEPES pH 7.4 assay buffer containing 100 mM NaCl, 2.0 mM CaCl2, 0.1% BSA and 0.1% Tween-20. Compound pre-incubation for 30 minutes at room temperature with 150 nM of 6his-Sortilin before 5 nM [3H]-Neurotensin and Ni chelate imaging beads (Perkin Elmer) were added, after 6 hours the plate was read on a ViewLux with 360 s exposure time. Dose-response evaluation of compounds was performed with 8 concentrations of drugs (covering 3 decades). $IC_{50}$ values were calculated by nonlinear regression using the sigmoid concentration-response (variable slope) using CDD Vault software. All values reported are average of at least 2 determinations.

**Human Sortilin GCI binding assay**

The exemplified compounds of the invention were tested in a Grating-Coupled Interferometry (GCI) Sortilin binding assay. The GCI data is shown in Table 1 below. The Equilibrium dissociation constant ($K_D$) is a measure of the propensity of a complex (i.e: a ligand and receptor) to dissociate and is used to describe the affinity of an interaction between the constituents of the complex (i.e: a ligand and receptor). The skilled person will recognise that the lower the $K_D$ value, the less of the compound needed to achieve the desired effect, and as a result, the chances of undesirable off-target effects are reduced.

Compound affinity was determined by measuring the association and dissociation of compounds to Human (6-His)Sortilin (RnD Systems) using a Creoptix Wave instrument (Creoptix - Malvern Panalytical). Both Human (6-His)Sortilin immobilisation and compound evaluation were performed in HBS-N running buffer: 10 mM HEPES pH 7.4, 150 mM NaCl, 1 % DMSO - Cytiva which was filtered and degassed for 15 minutes before use.

A 4PCH WAVEchip (Creoptix - Malvern Panalytical) was conditioned prior to immobilisiation using 0.2x HBS-N running buffer with 0.1 M Borate pH 9.0, 1 M NaCl (Xantec Bioanalytics) injected across all flow cells. A buffer exchange was performed to 1x HBS-N running buffer and Human (6-His)Sortilin was immobililised in pH 5.0 Acetate solution (Cytiva) via amine coupling following EDC/NHS (Cytiva) surface activation and then passivated via injection of 50 mM Tris pH 7.4.

Compounds were tested in 'WAVE-RAPID' assays at a single concentration (usually: 1 μM, 10 μM or 100 μM) in time-response format (short pulses of increasing duration) using either 'Weak-Binders' (Settings: 400 μl/min flow rate, 40 Hz acquisiton rate, 5 seconds association time and 20 seconds dissociation time) or 'Intermediate Binders' (Settings: 100 μl/min flow rate, 10 Hz acquisiton rate, 25 seconds association time and 300 seconds dissociation time) settings. Which was deemed most appropriate was based upon the affinity and kinetics of the compound tested.

DMSO calibration was performed as per manufacturers requirements by injection of running buffer containing DMSO 0.5% above that of the running buffer DMSO (1.5 % in that instance) every 20 cycles. All data was double referenced (blank and reference-channel subtracted) with $K_D$ values calculated by fitting to a '1:1 kinetic' binding model using 'WAVEcontrol' software (Creoptix - Malvern Panalytical).

The data in Table 1 below shows that the compounds disclosed herein bind to sortilin.

**Table 1**

| Example | KD (M) Sortilin | $IC_{50}$ [3H]Neurotensin SPA (nM) |
|---|---|---|
| 2 | 2.23E-06 | 2200 |
| 4 | 5.57E-06 | 1075 |
| 5 | 1.32E-05 | 1185 |
| 6 | 4.05E-06 | 1235 |
| 7 | 8.28E-06 | 1135 |
| 8 | 1.16E-07 | 325 |
| 23 | 1.08E-07 | 1310 |
| 24 | 4.29E-07 | 2870 |
| 25 | 4.99E-07 | 1560 |
| 26 | 5.41E-08 | 1830 |
| 27 | 6.64E-07 | 510 |
| 28 | 5.25E-07 | 670 |
| 29 | 5.78E-08 | |
| 31 | 1.71E-06 | 1805 |
| 32 | 2.62E-07 | |
| 33 | 3.43E-06 | |
| 34 | 1.83E-06 | |
| 35 | 1.07E-06 | |
| 38 | 8.75E-06 | |
| 39 | 2.84E-06 | |
| 40 | 2.20E-05 | |
| 42 | 3.17E-06 | |
| 43 | 1.77E-05 | |
| 44 | 6.21E-06 | |

(continued)

| Example | KD (M) Sortilin | $IC_{50}$ [3H]Neurotensin SPA (nM) |
|---|---|---|
| 45 | 9.11E-05 | |
| 46 | 3.78E-06 | |
| 47 | 7.82E-06 | |
| 48 | 2.47E-06 | |
| 49 | 9.80E-05 | |
| 50 | 2.80E-05 | |
| 52 | 3.25E-06 | |
| 53 | 7.35E-07 | |
| 54 | 7.38E-06 | |
| 55 | 1.05E-05 | |
| 56 | 4.49E-05 | |
| 57 | 1.23E-04 | |
| 60 | 5.90E-06 | |
| 61 | 6.00E-05 | |
| 62 | 3.21E-05 | |
| 63 | 4.73E-05 | |
| 64 | 8.71E-06 | |
| 65 | 9.86E-05 | |
| 66 | 1.79E-04 | |
| 67 | 1.11E-04 | |
| 68 | 7.57E-06 | 10000 |
| 69 | 1.80E-06 | 10000 |
| 74 | 3.88E-07 | |
| 75 | 1.34E-07 | |
| 76 | 2.82E-05 | |
| 77 | 5.41E-06 | |
| 78 | 1.49E-06 | |
| 79 | 1.95E-05 | |
| 80 | 3.88E-06 | |
| 81 | 2.22E-06 | |
| 82 | 3.16E-07 | |
| 83 | 2.51E-07 | |
| 84 | 4.61E-07 | |
| 85 | 9.13E-08 | |
| 86 | 1.49E-06 | |
| 87 | 3.51E-07 | |
| 88 | 4.26E-07 | |
| 89 | 8.57E-08 | |

(continued)

| Example | KD (M) Sortilin | $IC_{50}$ [3H]Neurotensin SPA (nM) |
|---|---|---|
| 90 | 2.84E-07 | |
| 93 | 8.41E-08 | |
| 94 | 1.30E-06 | |
| 95 | 6.07E-06 | |
| 96 | 9.12E-07 | |
| 97 | 2.24E-07 | |
| 98 | 1.63E-06 | |
| 99 | 1.56E-06 | |
| 100 | 6.22E-06 | |
| 101 | 1.83E-05 | |
| 102 | 8.53E-07 | |
| 103 | 2.55E-05 | |
| 104 | 7.42E-05 | |
| 105 | 1.87E-04 | |

For compounds of the invention it is advantageous to have a $K_d$ lower than around 1.00 E-4. The $K_d$ data generated demonstrate that examples of the invention bind directly to the sortilin protein.

**X-ray Crystallography**

sSortilin, the luminal domain of sortilin, was obtained as previously described (Andersen et al., Acta Cryst. D, 2017). For crystallization 2 µl 5 mg/ml sSortilin in 50 mM Tris-HCl pH 7.3, 150 mM NaCl was mixed with 0.2 µl of Example 8 dissolved in 50% DMSO at a concentration of 14.86 mM. This drop was mixed with 2 µl of reservoir solution composed of 100 mM Hepes pH 7.3, 400 mM malonate pH 7.3, 7% v/v glycerol, 24% w/v PEG3350. The sitting drop were left to equilibrate by vapor diffusion with 500 µl reservoir solution. Crystals were mounted in litho-loops without further cryoprotection and were flash cooled in liquid nitrogen. Diffraction data were collected at beamline P13 EMBL/DESY, Hamburg, and processed using the XDS package (Kabsch, W., Acta Cryst. D, 2010).

Phases for the structure factors were obtained by molecular replacement using the known structure of sortilin (PDB entry: 3F6K) as search model and the program Phaser implemented in the Phenix software package (Afonine et al., Acta Cryst. D, 2012). A refined model was obtained by several cycles of model building in Coot (Emsley P. et al., Acta Cryst. D, 2010) and maximum likelihood refinement using Phenix.

Details of the diffraction data are provided in the Table 2 below (statistics for the highest-resolution shell are shown in parentheses).

**Table 2**

| Diffraction Data | Example 8 |
|---|---|
| Wavelength | 0.97626 Å |
| Resolution range | 47.04 - 2.7 (2.797 - 2.7) |
| Space group | C 1 2 1 |
| Unit cell | 161.7 78.56 111.65 90 126.97 90 |
| Total reflections | 414893 (41351) |
| Unique reflections | 30913 (3089) |
| Multiplicity | 13.4 (13.4) |
| Completeness (%) | 99.64 (98.84) |

(continued)

| Diffraction Data | Example 8 | |
|---|---|---|
| Mean I/sigma(I) | 16.13 (1.42) | |
| Wilson B-factor | 89.32 | |
| R-merge | 0.09228 (2.045) | |
| R-meas | 0.096 (2.126) | |
| R-pim | 0.02624 (0.5781) | |
| CC1/2 | 0.999 (0.847) | |
| CC* | | 1 (0.958) |
| **Refinement and model quality** | | |
| Reflections (work) | | 30815 (3055) |
| Reflections (Free) | | 1545 (154) |
| R-work | | 0.2139 (0.4077) |
| R-free | | 0.2447 (0.4709) |
| CC(work) | | 0.949 (0.786) |
| CC(free) | | 0.939 (0.688) |
| No. of non-hydrogen atoms | | 5373 |
| | macromolecules | 5176 |
| | ligands | 163 |
| | solvent | 34 |
| Protein residues | | 658 |
| RMS(bonds (Å) / angles (°)) | | 0.002/0.41 |
| Ramachandran favored (%) | | 95.72 |
| Ramachandran outliers (%) | | 0.15 |
| Rotamer outliers (%) | | 0.7 |
| Clash score | | 4.03 |
| Average B-factor | | 119.22 |
| | macromolecules | 118.91 |
| | ligands | 133.81 |
| | solvent | 96.46 |
| Number of TLS groups | | 6 |

The X-ray derived images of Example 8 bound to h-Sortilin are shown in Figures 1 to 6. Figure 6 also depicts an estimation of the atomic contribution to binding affinity.

Figures 7A to 7C depict electron density maps showing the electron density observed during x-ray crystallography fitted to the structure of the molecules to provide a representation of the crystal structure.

Details of the electron density maps are as follows:

Figure 7A - 2fo-fc map. Contouring level = 1.5σ.

Figure 7B - omit map. Contouring level = 4.0σ

Figure 7C - polder map. Contouring level = 6.0σ

3 glycerol (GOL) molecules are included in the models. The polder and omit maps were calculated for GOL and Example 8 separately.

**REFERENCES**

Andersen, J et al., Identification of the first small-molecule ligand of the neuronal receptor sortilin and structure determination of the receptor-ligand complex. Acta Crystallogr D Biol Crystallogr (2014), 70(Pt 2), pp.451-460;

Baker, M.et al., Mutations in progranulin cause tau-negative frontotemporal dementia linked to chromosome 17. Nature (2006), 442(7105), pp. 916-919;

Brouwers, N.et al., Genetic variability in progranulin contributes to risk for clinically diagnosed Alzheimer disease. Neurology, (2008), 71(9), pp. 656-664;

Buttenshon, H.N. et al., Increased serum levels of sortilin are associated with depression and correlated with BDNF and VEGF, Nature Translational Psychiatry (2015), 5(e677), pp. 1-7;

Carecchio, M.,et al., Cerebrospinal fluid biomarkers in Progranulin mutations carriers. J Alzheimers Dis (2011), 27(4), pp. 781-790;

Carrasquillo, M. et al.,. Genome-wide screen identifies rs646776 near sortilin as a regulator of progranulin levels in human plasma. Am J Hum Genet (2010), 87(6), pp. 890-897;

Chen, Z. Y.et al., Sortilin controls intracellular sorting of brain-derived neurotrophic factor to the regulated secretory pathway. J Neurosci (2005), 25(26), pp. 6156-6166;

Cruts, M. et al., Loss of progranulin function in frontotemporal lobar degeneration. Trends Genet (2008), 24(4), pp. 186-194;

De Muynck, L. et al., The neurotrophic properties of progranulin depend on the granulin E domain but do not require sortilin binding. Neurobiol Aging (2013), 34(11), pp. 2541-2547;

Egashira, Y. et al.,The growth factor progranulin attenuates neuronal injury induced by cerebral ischemia-reperfusion through the suppression of neutrophil recruitment. J Neuroinflammation (2013), 10, pp. 105;

Galimberti, D. et al.,. GRN variability contributes to sporadic frontotemporal lobar degeneration. J Alzheimers Dis (2010), 19(1), pp. 171-177;

Galimberti, D.et al.,. Progranulin as a therapeutic target for dementia. Expert Opin Ther Targets (2018), 22(7), pp. 579-585. doi:10.1080/14728222.2018.1487951

Gao, A. et al., Implications of Sortilin in Lipid Metabolism and Lipid Disorder Diseases. DNA and Cell Biology (2017), 36(12), pp.1050-1061;

Gass, J. et al., Progranulin regulates neuronal outgrowth independent of sortilin. Mol Neurodegener (2012), 7, pp. 33;

Gass, J. et al., Progranulin: an emerging target for FTLD therapies. Brain Res (2012), 1462, pp. 118-128;

Gijselinck, I.,et al., Granulin mutations associated with frontotemporal lobar degeneration and related disorders: an update. Hum Mutat (2008), 29(12), pp. 1373-1386;

Goettsch, C., et al., Sortilin and Its Multiple Roles in Cardiovascular and Metabolic Diseases. Atherosclerosis, Thrombosis and Vascular Biology (2017), 38(1), pp. 19-25

Jansen, P., et al., Roles for the pro-neurotrophin receptor sortilin in neuronal development, aging and brain injury. Nature Neuroscience (2007), 10(11), pp. 1449-1457;

Hu, F. et al., Sortilin-mediated endocytosis determines levels of the frontotemporal dementia protein, progranulin. Neuron (2010), 68(4), pp. 654-667;

Huang, G. et al., Insulin responsiveness of glucose transporter 4 in 3T3-L1 cells depends on the presence of sortilin. Mol Biol Cell (2013), 24(19), pp.3115-3122;

Kaddai, V. et al. Involvement of TNF-$\alpha$ in abnormal adipocyte and muscle sortilin expression in obese mice and humans. Diabetologia (2009) 52, pp. 932-940;

Kjolby, M.et al., Sort1, encoded by the cardiovascular risk locus 1p13.3, is a regulator of hepatic lipoprotein export. Cell Metab (2010), 12(3), pp. 213-223;

Laird, A. S. et al., Progranulin is neurotrophic in vivo and protects against a mutant TDP-43 induced axonopathy. PLoS One (2010), 5(10), e13368;

Lee, W. et al., Targeted manipulation of the sortilin-progranulin axis rescues progranulin haploinsufficiency. Hum Mol Genet (2014), 23(6), pp. 1467-1478;

Martens, L.et al., Progranulin deficiency promotes neuroinflammation and neuron loss following toxin-induced injury. J Clin Invest (2012), 122(11), pp. 3955-3959;

Mazella, J. et al., The 100-kDa neurotensin receptor is gp95/sortilin, a non-G-protein-coupled receptor. J Biol Chem (1998), 273(41), pp. 26273-26276;

Miyakawa, S. et al, Anti-sortilin1 Antibody Up-Regulates Progranulin via Sortilin1 Down-Regulation. Front Neurosci

(2020), 14, pp. 586107;
Møller et al. Sortilin as a Biomarker for Cardiovascular Disease Revisited. Frontiers in Cardiovascular Medicine (2021), 8, 652584;
Mortensen, M.B. et al., Targeting sortilin in immune cells reduces proinflammatory cytokines and atherosclerosis. J Clin Invest (2014), 124(12), pp. 5317-5322;
Nykjaer, A et al., Sortilin is essential for proNGF-induced neuronal cell death. Nature (2014), 427(6977), pp. 843-848;
Nykjaer, A., & Willnow, T. E, Sortilin: a receptor to regulate neuronal viability and function. Trends Neurosci (2012), 35(4), pp. 261-270.
Oh, T.J. et al., Circulating sortilin level as a potential biomarker for coronary atherosclerosis and diabetes mellitus. Cardiovascular Diabetology (2017), 16(92);
Pan, X. et al., Sortilin and retromer mediate retrograde transport of Glut4 in 3T3-L1 adipocytes. Mol Biol Cell (2017), 28(12), pp.1667-1675;
Petersen, C. et al., Molecular identification of a novel candidate sorting receptor purified from human brain by receptor-associated protein affinity chromatography. J Biol Chem (1997), 272(6), pp. 3599-3605;
Pickford, F.et al., Progranulin is a chemoattractant for microglia and stimulates their endocytic activity. Am J Pathol (2011), 178(1), pp. 284-295;
Pottier, C., et al., Potential genetic modifiers of disease risk and age at onset in patients with frontotemporal lobar degeneration and GRN mutations: a genome-wide association study. Lancet Neurol (2018), 17(6), pp. 548-558;
Quistgaard, E. et al., Ligands bind to Sortilin in the tunnel of a ten-bladed beta-propeller domain. Nat Struct Mol Biol (2009), 16(1), pp. 96-98;
Santos, A. M. et al., Sortilin Participates in Light-dependent Photoreceptor Degeneration in Vivo. PLoS ONE (2012), 7(4), pp. e36243-e36243.16. Kuruvilla, R. et al., A neurotrophin signaling cascade coordinates sympathetic neuron development through differential control of TrkA trafficking and retrograde signalling. Cell (2004), 118(2), pp. 243-255;
Shi, J. & Kandror, K. V., Sortilin Is Essential and Sufficient for the Formation of Glut4 Storage Vesicles in 3T3-L1 Adipocytes. Developmental Cell (2005), 9, pp. 99-108;
Schroder, T. et al., The identification of AF38469: an orally bioavailable inhibitor of the VPS10P family sorting receptor Sortilin. Bioorg Med Chem Lett (2014), 24(1), pp. 177-180;
Sheng, J. et al.,Progranulin polymorphism rs5848 is associated with increased risk of Alzheimer's disease. Gene (2014), 542(2), pp. 141-145;Skeldal, S. et al., Mapping of the Interaction Site between Sortilin and the p75 Neurotrophin Receptor Reveals a Regulatory Role for the Sortilin Intracellular Domain in p75 Neurotrophin Receptor Shedding and Apoptosis. J Biol Chem (2012), 21(287), pp. 43798-43809;
Tauris, J., et al., Proneurotrophin-3 May Induce Sortilin-Dependent Death In Inner Ear Neurons. Eur J Neuroscience (2020), 33(4), pp.622-31;
Tang, W. et al., The growth factor progranulin binds to TNF receptors and is therapeutic against inflammatory arthritis in mice. Science (2011), 332(6028), pp. 478-484;
Tao, J.et al., Neuroprotective effects of progranulin in ischemic mice. Brain Res (2012), 1436, pp. 130-136;
Tenk, H.K., et al., ProBDNF induces neuronal apoptosis via activation of a receptor complex of p75NTR and sortilin. J Neuroscience (2005), 10(11), pp. 1449-1457
Van Kampen, J. M.,et al., Progranulin gene delivery protects dopaminergic neurons in a mouse model of Parkinson's disease. PLoS One (2014), 9(5), e97032;
Willnow, T. E.et al., VPS10P-domain receptors - regulators of neuronal viability and function. Nat Rev Neurosci (2008), 9(12), pp. 899-909;
Willnow, T.E., et al., Sortilins: new players in lipoprotein metabolism. Current Opinion in Lipidology (2011), 22(2), pp. 79-85.
Wuts, P.G.M. and Greene, T.W, Greene's Protective Groups in Organic Synthesis, 4th Edition, John Wiley and Sons, New York (2006);
Xu, S.H. et al., Regional and Cellular Mapping of Sortilin Immunoreactivity in Adult Human Brain, Frotiers in Neuroanatomy (2019), 13(31), pp. 1-27;
Yano, H., et al., Proneurotrophin-3 is a neuronal apoptotic ligand: evidence for retrograde-directed cell killing. J Neurosci (2009), 29(47), pp. 14790-14802;
Yin, F., et al., Exaggerated inflammation, impaired host defense, and neuropathology in progranulin-deficient mice. J Exp Med (2010), 207(1), pp. 117-128;
Zheng, Y., et al., C-terminus of progranulin interacts with the beta-propeller region of sortilin to regulate progranulin trafficking. PLoS One (2011), 6(6), e21023;
Zhou, X. et al., Prosaposin facilitates sortilin-independent lysosomal trafficking of progranulin. J Cell Biol (2015), 210(6), pp. 991-1002;
Meneses et al., TDP-43 Pathology in Alzheimer's Disease, Mol Neurodegeneration (2021), 16, 84;
Prudencio et al., Misregulation of human sortilin splicing leads to the generation of a nonfunctional progranulin

receptor, Proc Natl Acad Sci USA (2012), 109(52): 21510-21515;
Beel et al., Progranulin reduces insoluble TDP-43 levels, slows down axonal degeneration and prolongs survival in mutant TDP-43 mice, Mol Neurodegener. (2018), 13: 55.

**Sequences referenced throughout the specification and forming part of the description**

SEQ ID NO: 1 (full length sortilin- isoform 1)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCKAD LGALELWRTS DLGKSFKTIG
301 VKIYSFGLGG RFLFASVMAD KDTTRRIHVS TDQGDTWSMA QLPSVGQEQF
351 YSILAANDDM VFMHVDEPGD TGFGTIFTSD DRGIVYSKSL DRHLYTTTGG
401 ETDFTNVTSL RGVYITSVLS EDNSIQTMIT FDQGGRWTHL RKPENSECDA
451 TAKNKNECSL HIHASYSISQ KLNVPMAPLS EPNAVGIVIA HGSVGDAISV
501 MVPDVYISDD GGYSWTKMLE GPHYYTILDS GGIIVAIEHS SRPINVIKFS
551 TDEGQCWQTY TFTRDPIYFT GLASEPGARS MNISIWGFTE SFLTSQWVSY
601 TIDFKDILER NCEEKDYTIW LAHSTDPEDY EDGCILGYKE QFLRLRKSSM
651 CQNGRDYVVT KQPSICLCSL EDFLCDFGYY RPENDSKCVE QPELKGHDLE
701 FCLYGREEHL TTNGYRKIPG DKCQGGVNPV REVKDLKKKC TSNFLSPEKQ
751 NSKSNSVPII LAIVGLMLVT VVAGVLIVKK YVCGGRFLVH RYSVLQQHAE
801 ANGVDGVDAL DTASHTNKSG YHDDSDEDLL E

SEQ ID NO: 2 (full length sortilin- isoform 2)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT

601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE

SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI
361 VIAHGSVGDA ISVMVPDVYI SDDGGYSWTK MLEGPHYYTI LDSGGIIVAI
401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK
551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

SEQ ID NO: 4 (Murine Sortilin)
>sp|Q6PHU5|SORT_MOUSE Sortilin OS=Mus musculus OX=10090 GN=Sort1 PE=1 SV=1

MERPRGAADGLLRWPLGLLLLLQLLPPAAVGQDRLDAPPPPAPPLLRWAGPVGVSWGLRA

AAPGGPVPRAGRWRRGAPAEDQDCGRLPDFIAKLTNNTHQHVFDDLSGSVSLSWVGDST
G

VILVLTTFQVPLVIVSFGQSKLYRSEDYGKNFKDITNLINNTFIRTEFGMAIGPENSGKV

ILTAEVSGGSRGGRVFRSSDFAKNFVQTDLPFHPLTQMMYSPQNSDYLLALSTENGLWVS

KNFGEKWEEIHKAVCLAKWGPNNIIFFTTHVNGSCKADLGALELWRTSDLGKTFKTIGVK

IYSFGLGGRFLFASVMADKDTTRRIHVSTDQGDTWSMAQLPSVGQEQFYSILAANEDMVF

MHVDEPGDTGFGTIFTSDDRGIVYSKSLDRHLYTTTGGETDFTNVTSLRGVYITSTLSED

NSIQSMITFDQGGRWEHLRKPENSKCDATAKNKNECSLHIHASYSISQKLNVPMAPLSEP

NAVGIVIAHGSVGDAISVMVPDVYISDDGGYSWAKMLEGPHYYTILDSGGIIVAIEHSNR

PINVIKFSTDEGQCWQSYVFTQEPIYFTGLASEPGARSMNISIWGFTESFITRQWVSYTV

DFKDILERNCEEDDYTTWLAHSTDPGDYKDGCILGYKEQFLRLRKSSVCQNGRDYVVAKQ

PSVCPCSLEDFLCDFGYFRPENASECVEQPELKGHELEFCLYGKEEHLTTNGYRKIPGDK

CQGGMNPAREVKDLKKKCTSNFLNPTKQNSKSNSVPIILAIVGLMLVTVVAGVLIVKKYV

CGGRFLVHRYSVLQQHAEADGVEALDSTSHAKSGYHDDSDEDLLE

## Claims

1. A compound of formula (I):

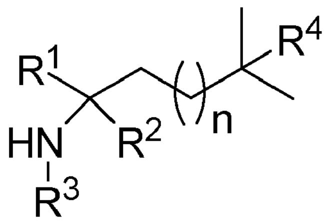

(I)

or a pharmaceutically acceptable salt, solvate, hydrate, tautomer, optical isomer, N-oxide, and/or prodrug thereof; wherein

$R^1$ is

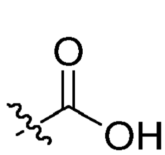 or 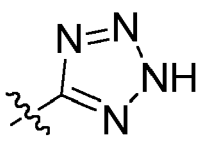 ;

$R^2$ is H or $-CH_3$;

$R^3$ is selected from the group consisting of $C_6$-$C_{10}$ aryl and 5- to 10-membered heteroaryl, optionally substituted with one or more substituents independently selected from -OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, $C_1$-$C_4$ hydroxyalkoxy, acetyl, cyano, $C_6$-$C_{10}$ aryl, 5- to 10-membered heteroaryl, 5- to 10-membered heterocycloalkyl, -O-($C_6$-$C_{10}$ aryl), $-O-CH_2-$($C_6$-$C_{10}$ aryl), and $-NR^5R^6$;

$R^4$ is H or $-CH_3$;

$R^5$ and $R^6$ are each independently H or$C_1$-$C_4$ alkyl; and

n is 0 or 1.

2. The compound according to claim 1, wherein $R^1$ is $-CO_2H$; and/or

$R^2$ is H; and/or
wherein $R^4$ is $-CH_3$; and/or
wherein n is 1.

**3.** The compound according to claim 1 or 2, wherein $R^3$ is selected from the group consisting of phenyl, naphthyl, 5- or 6-membered monocyclic heteroaryl, and 9- or 10-membered fused bicyclic heteroaryl, preferably phenyl, naphthyl, 6-membered monocyclic heteroaryl, and 9- or 10-membered fused bicyclic heteroaryl, more preferably 10-membered fused bicyclic heteroaryl, wherein each group is optionally substituted.

**4.** The compound according to claim 3, wherein each ring atom in the 5- or 6-membered monocyclic heteroaryl group and the 9- or 10-membered fused bicyclic heteroaryl group of $R^3$ is independently C or N, preferably one to three ring atoms are N and the remaining ring atoms are C.

**5.** The compound according to claim 4, wherein one or two ring atoms in the 5- or 6-membered monocyclic heteroaryl group of $R^3$ are N and the remaining ring atoms are C.

**6.** The compound according to any of claims 3 to 5, wherein the 6-membered monocyclic heteroaryl group of $R^3$ is selected from the group consisting of pyridyl, pyrimidinyl, pyrazinyl, pyridazinyl, and triazinyl, preferably pyridyl, pyrimidinyl, and pyrazinyl, more preferably one of the following groups:

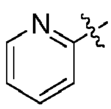, 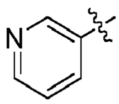, 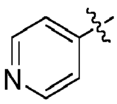, 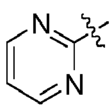, 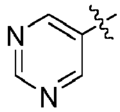, 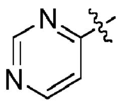, and 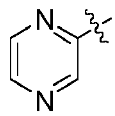,

wherein each group is optionally substituted.

**7.** The compound according to any of claims 3 to 6, wherein the 9- or 10-membered fused bicyclic heteroaryl group of $R^3$ is selected from the group consisting of indolyl, indazolyl, benzimidazolyl, benzotriazolyl, azaindolyl, azaindazolyl, pyrazolopyrimidinyl, quinolinyl, isoquinolinyl, quinoxalinyl, phthalazinyl, quinazolinyl, cinnolinyl, naphthyridinyl, pyridopyrimidinyl, and pyridopyrazinyl, preferably azaindolyl, pyridopyrimidinyl, quinolinyl, isoquinolinyl, quinoxalinyl, and quinazolinyl, more preferably one of the following groups:

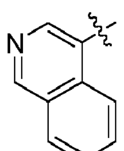, 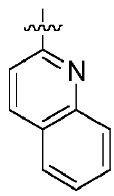, 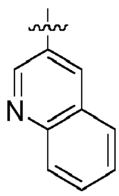, 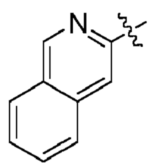, 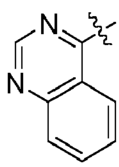,

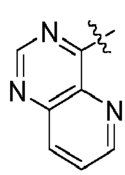, 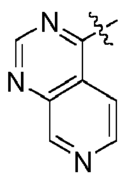, 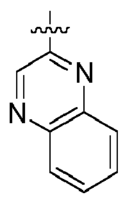, and 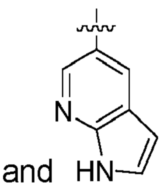,

most preferably

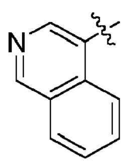 and 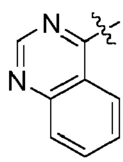,

wherein each group is optionally substituted.

**8.** The compound according to any preceding claim, wherein the optional substituents for $R^3$ are independently selected from the group consisting of halo, cyano, $C_1$-$C_4$ alkyl, $C_1$-$C_4$ alkoxy, $C_1$-$C_4$ haloalkyl, phenyl, 6-membered heterocycloalkyl, -O-phenyl, and -O-$CH_2$-phenyl, preferably halo, cyano, $C_1$-$C_2$ alkyl, $C_1$-$C_2$ alkoxy, $C_1$-$C_2$ haloalkyl, phenyl, morpholinyl, -O-phenyl, and -O-$CH_2$-phenyl.

**9.** The compound according to any preceding claim, wherein $R^3$ is selected from the group consisting of:

(i) phenyl and 6-membered monocyclic heteroaryl, optionally substituted with one or more substituents independently selected from the group consisting of halo, $C_1$-$C_2$ alkyl, $C_1$-$C_2$ alkoxy, phenyl, -O-phenyl, -O-$CH_2$-phenyl, and morpholinyl; and
(ii) naphthyl and 9- or 10-membered fused bicyclic heteroaryl, optionally substituted with one or more substituents independently selected from the group consisting of halo, cyano, $C_1$-$C_2$ alkyl, $C_1$-$C_2$ alkoxy, $C_1$-$C_2$ haloalkyl, and phenyl.

**10.** The compound according to claim 9, wherein $R^3$ is selected from the group consisting of:

(i) phenyl optionally substituted with one or more substituents independently selected from the group consisting of halo and -O-phenyl;
(ii) 6-membered monocyclic heteroaryl optionally substituted with one or more substituents independently selected from the group consisting of halo, $C_1$-$C_2$ alkyl, $C_1$-$C_2$ alkoxy, phenyl, -O-phenyl, -O-$CH_2$-phenyl, and morpholinyl;
(iii) naphthyl optionally substituted with one or more halo atoms; and
(iv) 9- or 10-membered fused bicyclic heteroaryl optionally substituted with one or more substituents independently selected from the group consisting of halo, cyano, $C_1$-$C_2$ alkyl, $C_1$-$C_2$ alkoxy, $C_1$-$C_2$ haloalkyl, and phenyl.

**11.** The compound according to any preceding claim, wherein $R^3$ is selected from the group consisting of:

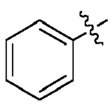 , 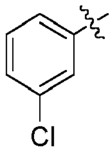 , 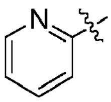 , 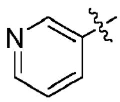 , 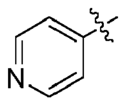 , 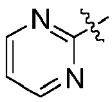 , 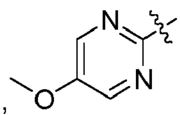 , 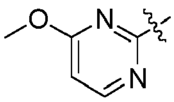

, 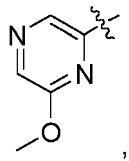 , 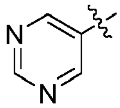 , 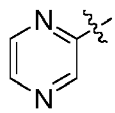 , 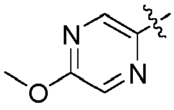 , 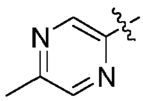 , 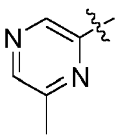 , 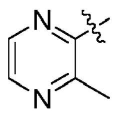 , 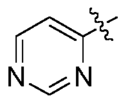

, 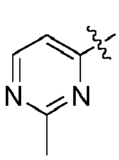 , 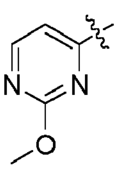 , 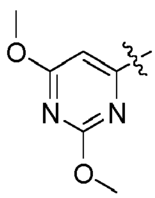 , 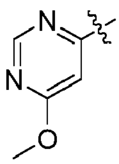 , 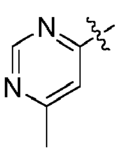 , 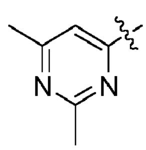 , 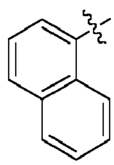 , 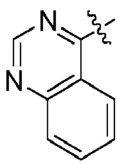 ,

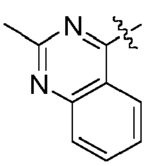 , 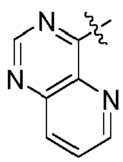 , 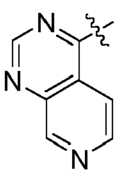 , 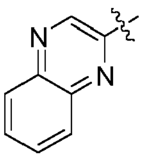 , 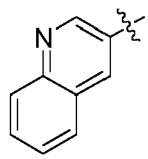 , 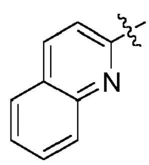 , 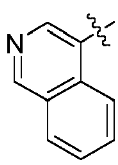 ,

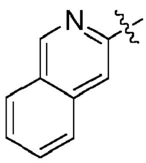, 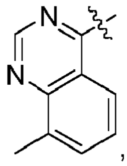, 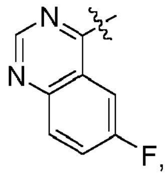, 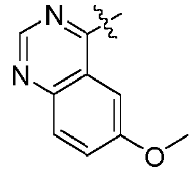, 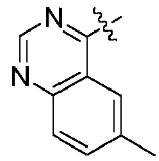, 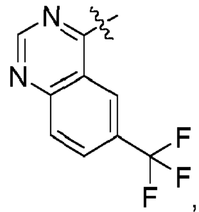,

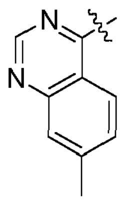, 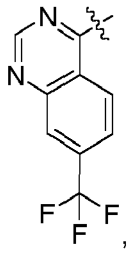, 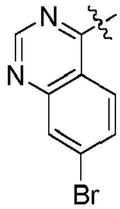, 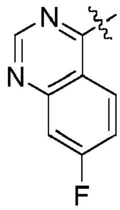, 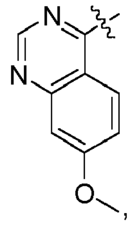, 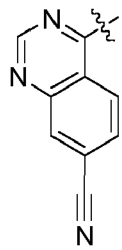, 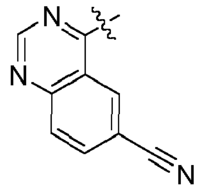,

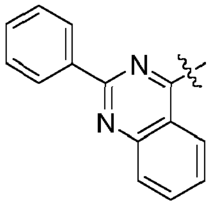, 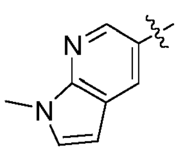, 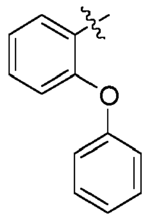, 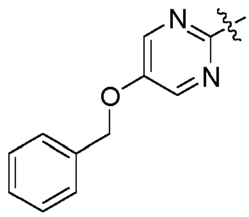, 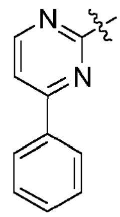, 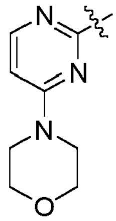,

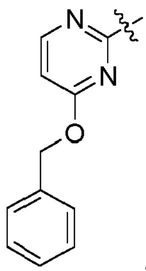, 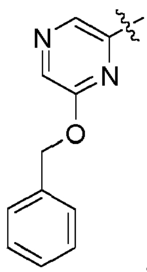, 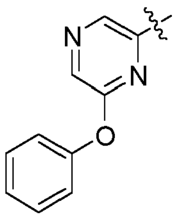, and 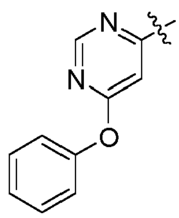.

**12.** The compound according to any preceding claim, wherein the compound is:

(S)-2-anilino-5,5-dimethylhexanoic acid;
(S)-5,5-dimethyl-2-(2-pyridylamino)hexanoic acid;
(S)-5,5-dimethyl-2-(4-pyridylamino)hexanoic acid;
(S)-5,5-dimethyl-2-(2-pyrazinylamino)hexanoic acid;
(S)-5,5-dimethyl-2-(2-pyrimidinylamino)hexanoic acid;
(S)-5,5-dimethyl-2-(5-pyrimidinylamino)hexanoic acid;
(S)-5,5-dimethyl-2-(4-pyrimidinylamino)hexanoic acid;
(S)-5,5-dimethyl-2-(4-quinazolinylamino)hexanoic acid;
(S)-5,5-dimethyl-2-(1,3,5-triaza-4-naphthylamino)hexanoic acid;
(S)-2-[5-(benzyloxy)-2-pyrimidinylamino]-5,5-dimethylhexanoic acid
(S)-2-(5-methoxy-2-pyrimidinylamino)-5,5-dimethylhexanoic acid
(S)-2-(4-methoxy-2-pyrimidinylamino)-5,5-dimethylhexanoic acid
(S)-5,5-dimethyl-2-(4-morpholino-2-pyrimidinylamino)hexanoic acid
(S)-5,5-dimethyl-2-(2-quinoxalinylamino)hexanoic acid
(S)-5,5-dimethyl-2-(5-methyl-2-pyrazinylamino)hexanoic acid
(S)-5,5-dimethyl-2-(3-methyl-2-pyrazinylamino)hexanoic acid
(S)-2-(2-methoxy-4-pyrimidinylamino)-5,5-dimethylhexanoic acid
(S)-2-(6-methoxy-4-pyrimidinylamino)-5,5-dimethylhexanoic acid
(S)-5,5-dimethyl-2-(2-methyl-4-pyrimidinylamino)hexanoic acid

(S)-5,5-dimethyl-2-(6-methyl-4-pyrimidinylamino)hexanoic acid
(S)-2-(2,6-dimethyl-4-pyrimidinylamino)-5,5-dimethylhexanoic acid
(S)-5,5-dimethyl-2-(8-methyl-4-quinazolinylamino)hexanoic acid
(S)-5,5-dimethyl-2-(6-methyl-2-pyrazinylamino)hexanoic acid
(S)-5,5-dimethyl-2-(6-phenoxy-2-pyrazinylamino)hexanoic acid
(S)-2-(4-isoquinolylamino)-5,5-dimethylhexanoic acid
(S)-2-(m-chlorophenylamino)-5,5-dimethylhexanoic acid
(S)-5,5-dimethyl-2-(3-pyridylamino)hexanoic acid
[4,4-dimethyl-1-(2H-tetraazol-5-yl)pentyl]-4-isoquinolylamine
[4,4-dimethyl-1-(2H-tetraazol-5-yl)pentyl]-3-quinolylamine;
[4,4-dimethyl-1-(2H-tetraazol-5-yl)pentyl]-2-quinolylamine;
[4,4-dimethyl-1-(2H-tetraazol-5-yl)pentyl]-1-naphthylamine;
N-phenyl[4-methyl-1-(2H-tetraazol-5-yl)pentyl]amine;
N-2-pyridyl[4-methyl-1-(2H-tetraazol-5-yl)pentyl]amine;
[4-methyl-1-(2H-tetraazol-5-yl)pentyl]-1-naphthylamine;
N-phenyl[4,4-dimethyl-1-(2H-tetraazol-5-yl)pentyl]amine;
[3-methyl-1-(2H-tetraazol-5-yl)butyl]-2-quinolylamine;
[3-methyl-1-(2H-tetraazol-5-yl)butyl]-2-pyrazinylamine;
[3-methyl-1-(2H-tetraazol-5-yl)butyl]-3-isoquinolylamine;
N-phenyl[3,3-dimethyl-1-(2H-tetraazol-5-yl)butyl]amine;
[3,3-dimethyl-1-(2H-tetraazol-5-yl)butyl]-3-quinolylamine;
N-2-pyrimidinyl[3,3-dimethyl-1-(2H-tetraazol-5-yl)butyl]amine;
[3-methyl-1-(2H-tetraazol-5-yl)butyl]-4-isoquinolylamine;
N-2-pyrimidinyl[4,4-dimethyl-1-(2H-tetraazol-5-yl)pentyl]amine;
[3,3-dimethyl-1-(2H-tetraazol-5-yl)butyl]-4-isoquinolylamine;
[3-methyl-1-(2H-tetraazol-5-yl)butyl]-5-pyrimidinylamine;
N-5-pyrimidinyl[3,3-dimethyl-1-(2H-tetraazol-5-yl)butyl]amine;
N-2-pyrazinyl[3,3-dimethyl-1-(2H-tetraazol-5-yl)butyl]amine;
[3,3-dimethyl-1-(2H-tetraazol-5-yl)butyl](1-methyl-1H-1,7-diazainden-5-yl)amine;
[4,4-dimethyl-1-(2H-tetraazol-5-yl)pentyl](1-methyl-1H-1,7-diazainden-5-yl)amine;
[3,3-dimethyl-1-(2H-tetraazol-5-yl)butyl]-2-quinolylamine;
[4-methyl-1-(2H-tetraazol-5-yl)pentyl]-3-quinolylamine;
[3-methyl-1-(2H-tetraazol-5-yl)butyl]-3-quinolylamine;
N-2-pyrimidinyl[4-methyl-1-(2H-tetraazol-5-yl)pentyl]amine;
[4-methyl-1-(2H-tetraazol-5-yl)pentyl]-2-quinolylamine;
N-3-pyridyl[4-methyl-1-(2H-tetraazol-5-yl)pentyl]amine;
[3,3-dimethyl-1-(2H-tetraazol-5-yl)butyl]-3-isoquinolylamine;
N-2-pyridyl[3,3-dimethyl-1-(2H-tetraazol-5-yl)butyl]amine;
N-3-pyridyl[3,3-dimethyl-1-(2H-tetraazol-5-yl)butyl]amine;
[3-methyl-1-(2H-tetraazol-5-yl)butyl]-3-pyridylamine;
[4-methyl-1-(2H-tetraazol-5-yl)pentyl]-4-isoquinolylamine;
[3-methyl-1-(2H-tetraazol-5-yl)butyl]-2-pyrimidinylamine;
[3-methyl-1-(2H-tetraazol-5-yl)butyl]-2-pyridylamine;
[3-methyl-1-(2H-tetraazol-5-yl)butyl]aniline;
N-2-pyridyl[4,4-dimethyl-1-(2H-tetraazol-5-yl)pentyl]amine;
N-3-pyridyl[4,4-dimethyl-1-(2H-tetraazol-5-yl)pentyl]amine;
[4,4-dimethyl-1-(2H-tetraazol-5-yl)pentyl](6-fluoro-4-quinazolinyl)amine;
[(R)-4,4-dimethyl-1-(2H-tetraazol-5-yl)pentyl](6-fluoro-4-quinazolinyl)amine;
[(S)-4,4-dimethyl-1-(2H-tetraazol-5-yl)pentyl](6-fluoro-4-quinazolinyl)amine;
[3,3-dimethyl-1-(2H-tetraazol-5-yl)butyl]-4-quinazolinylamine;
[4,4-dimethyl-1-(2H-tetraazol-5-yl)pentyl](6-methoxy-4-quinazolinyl)amine;
[4-methyl-1-(2H-tetraazol-5-yl)pentyl](6-methoxy-4-quinazolinyl)amine;
[4-methyl-1-(2H-tetraazol-5-yl)pentyl](6-methyl-4-quinazolinyl)amine;
[4-methyl-1-(2H-tetraazol-5-yl)pentyl](6-fluoro-4-quinazolinyl)amine;
[4,4-dimethyl-1-(2H-tetraazol-5-yl)pentyl](7-fluoro-4-quinazolinyl)amine;
[4,4-dimethyl-1-(2H-tetraazol-5-yl)pentyl](6-methyl-4-quinazolinyl)amine;
[4,4-dimethyl-1-(2H-tetraazol-5-yl)pentyl](7-methyl-4-quinazolinyl)amine;
[4,4-dimethyl-1-(2H-tetraazol-5-yl)pentyl](7-methoxy-4-quinazolinyl)amine;

[4,4-dimethyl-1-(2H-tetraazol-5-yl)pentyl][6-(trifluoromethyl)-4-quinazolinyl]amine;
[4,4-dimethyl-1-(2H-tetraazol-5-yl)pentyl][7-(trifluoromethyl)-4-quinazolinyl]amine;
[4,4-dimethyl-1-(2H-tetraazol-5-yl)pentyl]-1,3,7-triaza-4-naphthylamine;
[4,4-dimethyl-1-(2H-tetraazol-5-yl)pentyl](2-methyl-4-quinazolinyl)amine;
[4,4-dimethyl-1-(2H-tetraazol-5-yl)pentyl](7-bromo-4-quinazolinyl)amine;
[4-methyl-1-(2H-tetraazol-5-yl)pentyl](7-methoxy-4-quinazolinyl)amine;
[4,4-dimethyl-1-(2H-tetraazol-5-yl)pentyl](2-phenyl-4-quinazolinyl)amine;
[(S)-4,4-dimethyl-1-(2H-tetraazol-5-yl)pentyl]-4-isoquinolylamine;
[(R)-4,4-dimethyl-1-(2H-tetraazol-5-yl)pentyl]-4-isoquinolylamine;
[(R)-4,4-dimethyl-1-(2H-tetraazol-5-yl)pentyl]-2-quinolylamine;
[(S)-4,4-dimethyl-1-(2H-tetraazol-5-yl)pentyl]-2-quinolylamine;
[(S)-4,4-dimethyl-1-(2H-tetraazol-5-yl)pentyl]-4-quinazolinylamine;
[(R)-4,4-dimethyl-1-(2H-tetraazol-5-yl)pentyl]-4-quinazolinylamine;
N-3-pyridyl[(S)-4,4-dimethyl-1-(2H-tetraazol-5-yl)pentyl]amine;
N-3-pyridyl[(R)-4,4-dimethyl-1-(2H-tetraazol-5-yl)pentyl]amine;
[4-methyl-1-(2H-tetraazol-5-yl)pentyl]-4-quinazolinylamine;
[4,4-dimethyl-1-(2H-tetraazol-5-yl)pentyl]-4-quinazolinylamine;
[3-methyl-1-(2H-tetraazol-5-yl)butyl]-4-quinazolinylamine;
[3-methyl-1-(2H-tetraazol-5-yl)butyl]-4-pyridylamine;
[3-methyl-1-(2H-tetraazol-5-yl)butyl]-4-pyrimidinylamine;
4-[4,4-dimethyl-1-(2H-tetraazol-5-yl)pentylamino]-7-quinazolinecarbonitrile;
4-[4,4-dimethyl-1-(2H-tetraazol-5-yl)pentylamino]-6-quinazolinecarbonitrile;
(S)-5,5-dimethyl-2-(o-phenoxyphenylamino)hexanoic acid;
(S)-2-anilino-4,4-dimethylvaleric acid;
(S)-2-anilino-2,5,5-trimethylhexanoic acid;
(S)-5,5-dimethyl-2-(4-phenyl-2-pyrimidinylamino)hexanoic acid;
(S)-2-[6-(benzyloxy)-2-pyrazinylamino]-5,5-dimethylhexanoic acid;
(S)-2-(6-methoxy-2-pyrazinylamino)-5,5-dimethylhexanoic acid;
(S)-2-(5-methoxy-2-pyrazinylamino)-5,5-dimethylhexanoic acid;
(S)-2-[4-(benzyloxy)-2-pyrimidinylamino]-5,5-dimethylhexanoic acid;
(S)-5,5-dimethyl-2-(6-phenoxy-4-pyrimidinylamino)hexanoic acid;
(S)-2-(2,6-dimethoxy-4-pyrimidinylamino)-5,5-dimethylhexanoic acid;
or a pharmaceutically acceptable salt, solvate, hydrate, tautomer, optical isomer, N-oxide, and/or prodrug thereof.

**13.** A pharmaceutical composition comprising a compound according to any one of claims 1-12 and a pharmaceutically acceptable carrier, excipient, and/or diluent.

**14.** The compound according to any one of claims 1-12, or the pharmaceutical composition of claim 13, for use in therapy.

**15.** The compound according to any one of claims 1-12, or the pharmaceutical composition of claim 13, for use in the treatment or prevention of a neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular disease, kidney disease, psoriasis, hereditary eye conditions, chronic pain, hearing loss or diseases **characterized by** misfolded tau;

preferably wherein the neurodegenerative disorder is selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy;
preferably wherein the neurodegenerative disorder is **characterised by** misfolded TAR DNA-binding protein 43, such as amyotrophic lateral sclerosis, Alzheimer's disease, Frontotemporal Lobar Degeneration, or frontotemporal dementia;
preferably wherein the psychiatric disorder is selected from bipolar disorder, major depression, post-traumatic stress disorder, and anxiety disorders.
preferably wherein the inflammatory disorder is selected from inflammatory diseases and neuroinflammation;
preferably wherein the lysosomal storage disorder is selected from the group consisting of NCL/Batten Disease

caused by mutations in CLN gene *CLN1 (PPT1), CLN2 (TPP1), CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7), CLCN6,* and/or *SGSH;* Pompe disease, Fabry disease, Gaucher disease, Niemann-Pick disease Types A, B, and C; GM1 gangliosidosis, GM2 gangliosidosis (including Sandhoff and Tay-Sachs), mucopolysacharidoses (MPS) types I (Hurler disease)/II (Hunter disease)/IIIa (Sanfilippo A)/IIIB (Sanfilippo B)/IIIc (Sanfilippo C)/IIId (Sanfilippo D)/IVA (Morqui" A)/'VB/VI/VII (Sly)/IX, mucolipisosis III (I-cell) and IV, multiple sulfatase deficiency; sialidosis, galactosialidosis, α-mannosidosis, β-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Danon disease, Griscelli disease Types ½/3, Hermansky Pudliak Disease, and Chédiak-Higashi syndrome;
preferably wherein the cancer is selected from breast cancer, lung cancer, ovarian cancer, prostate cancer, thyroid cancer, pancreatic cancer, glioblastoma and colorectal cancer;
preferably wherein the cardiovascular disease is selected from atherosclerosis, cardiomyopathy, heart attack, arrhythmias, heart failure, and ischemic heart disease; and
preferably wherein the hearing loss is selected from noise-induced hearing loss, ototoxicity induced hearing loss, age-induced hearing loss, idiopathic hearing loss, tinnitus and sudden hearing loss.

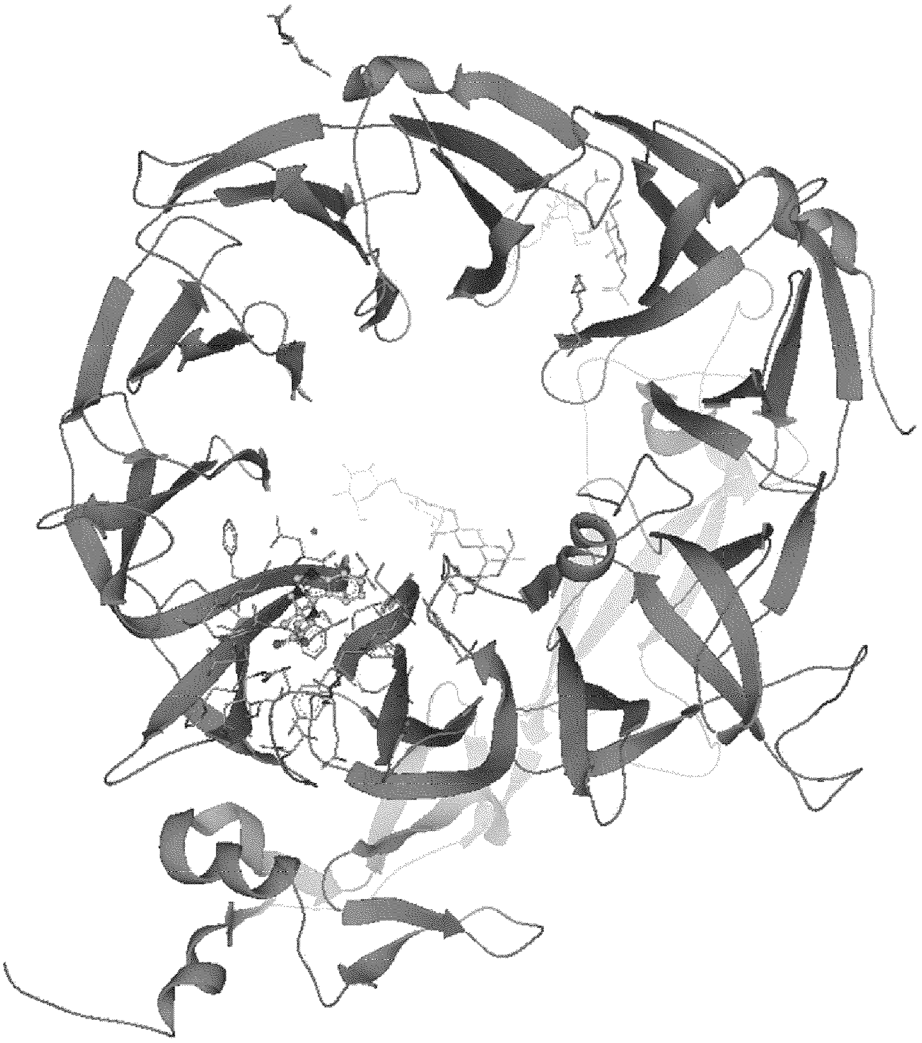

Figure 1

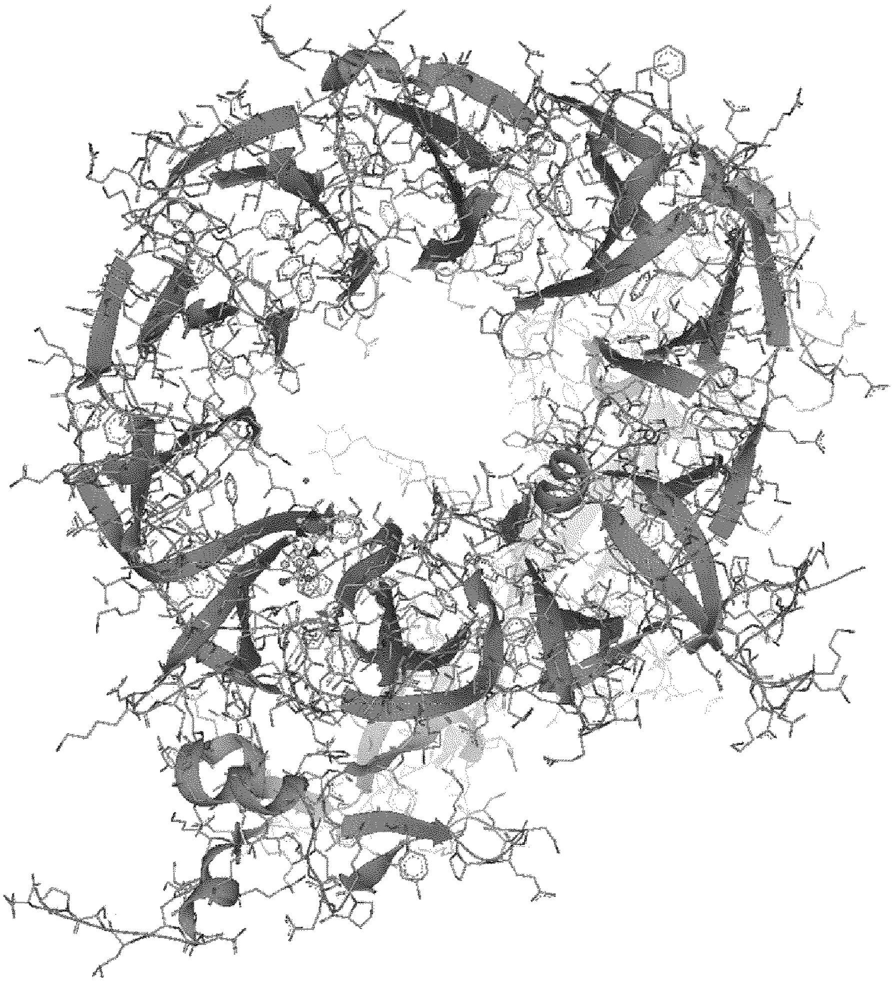

Figure 2

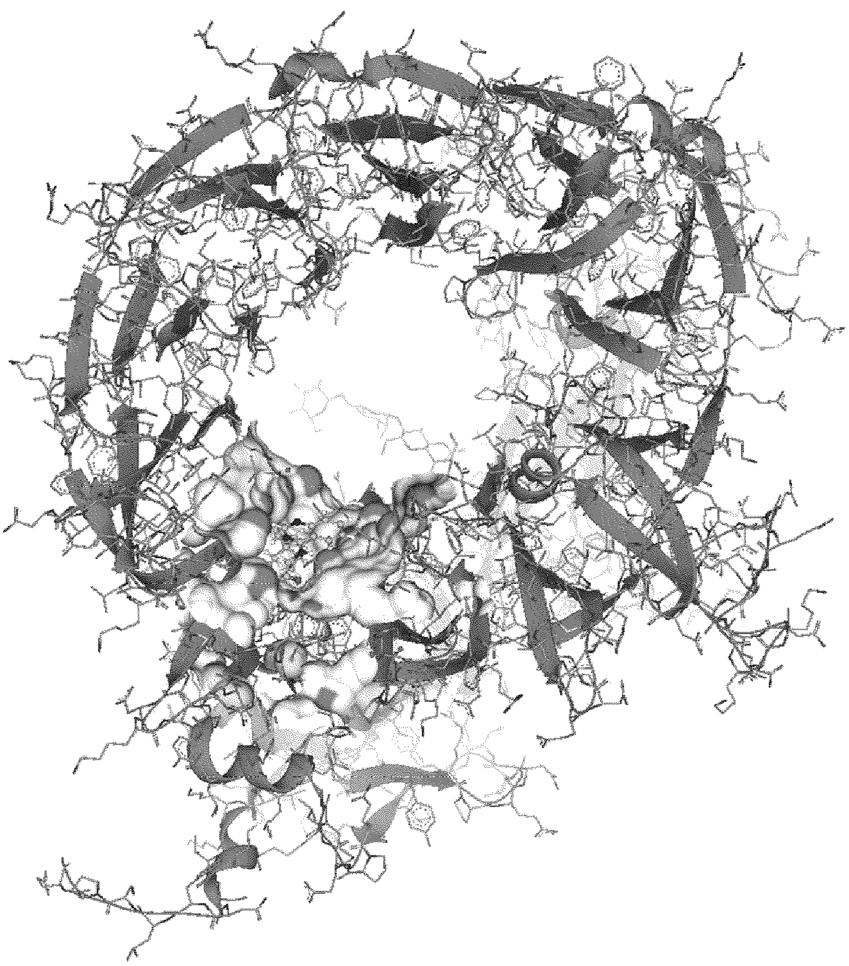

Figure 3

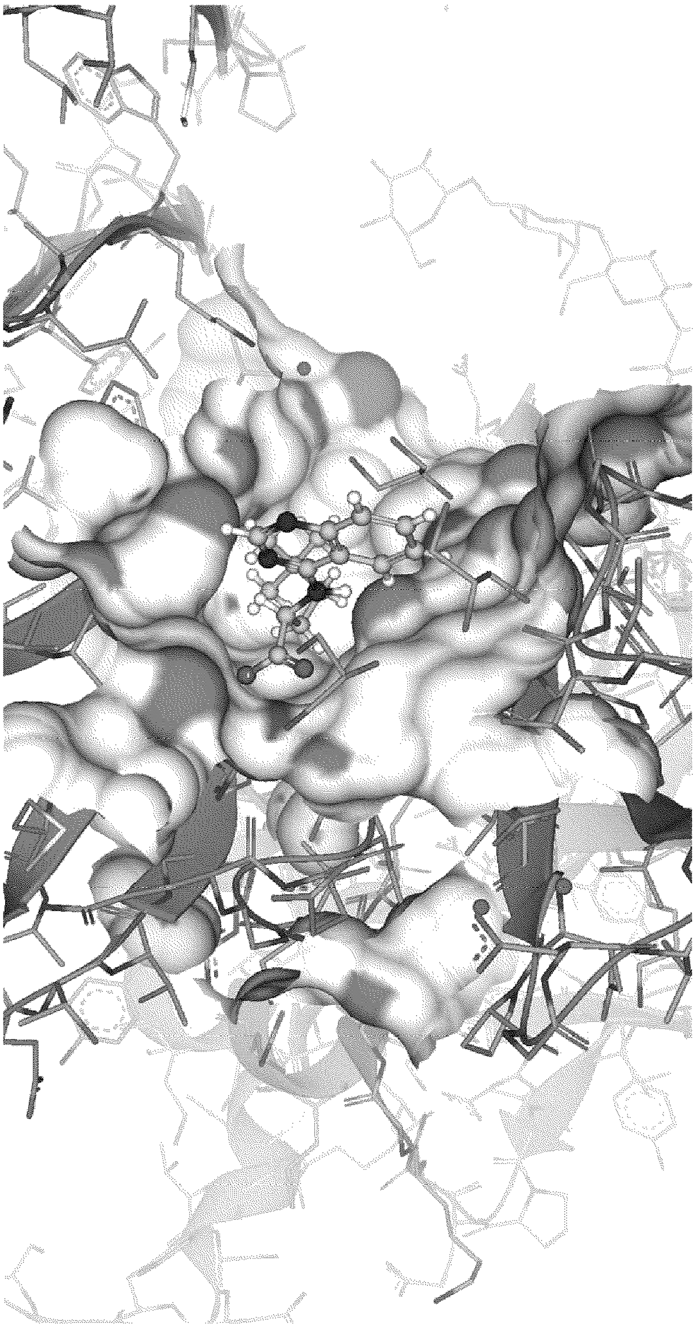

Figure 4

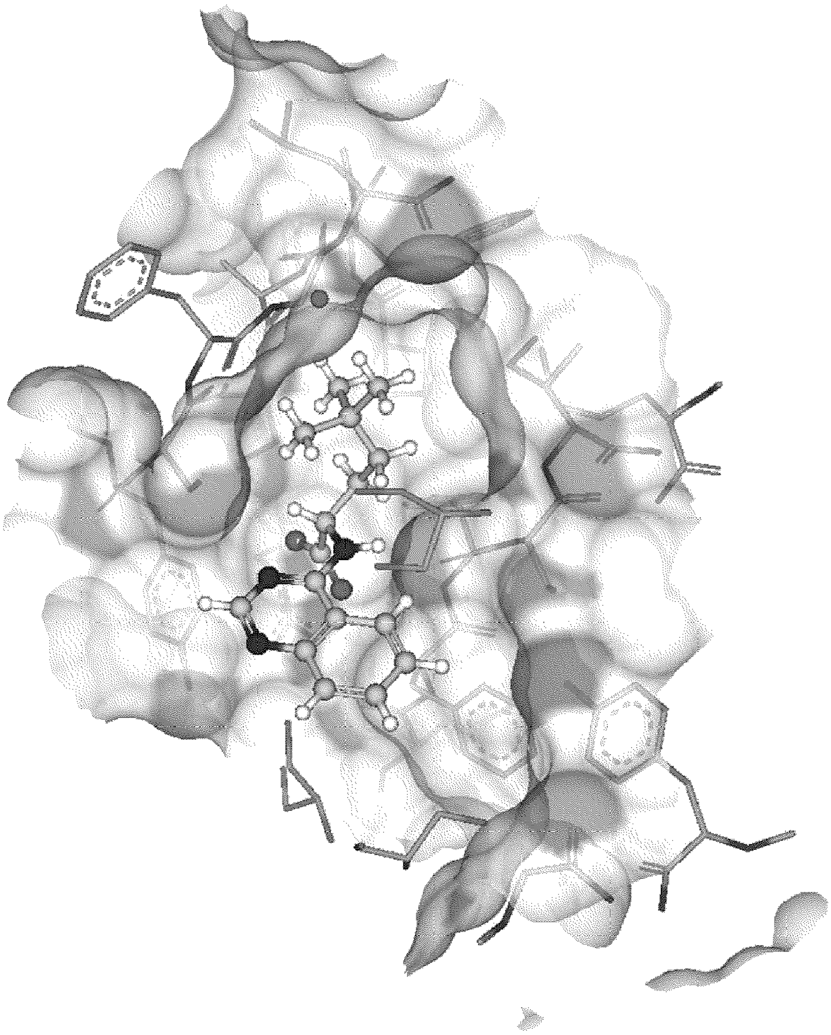

Figure 5

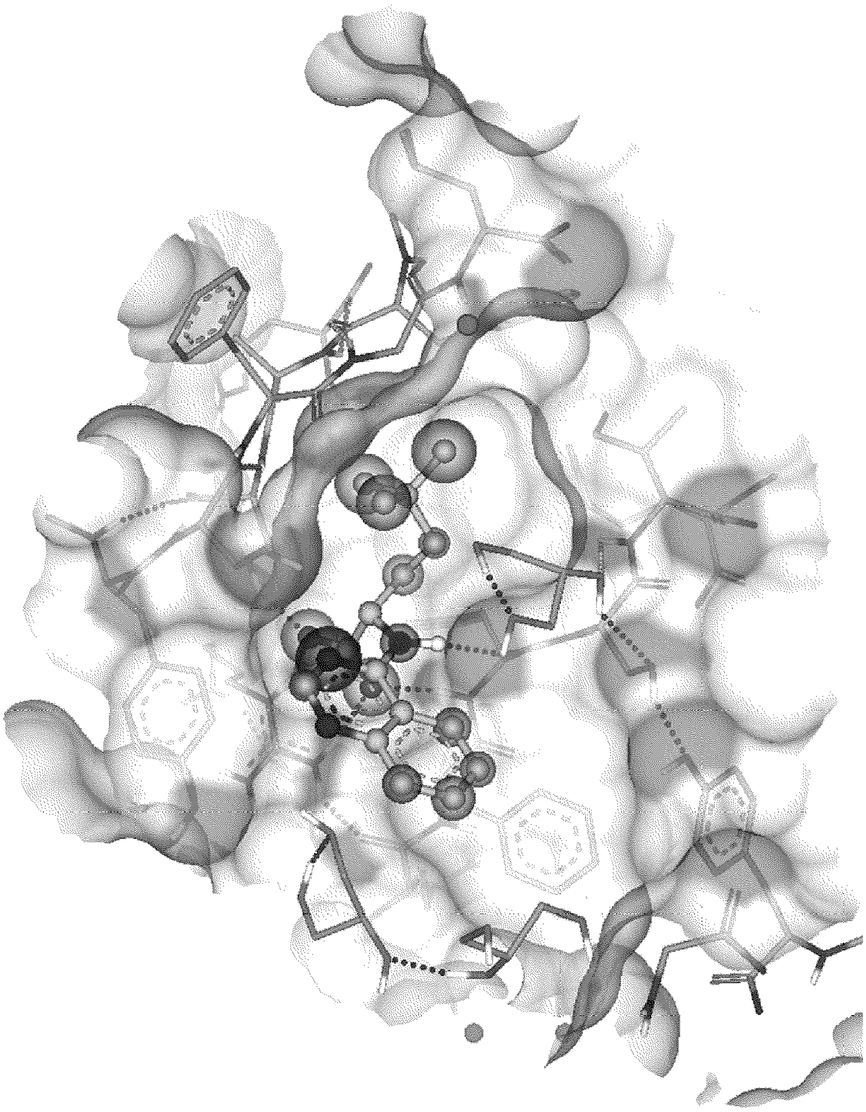

Figure 6

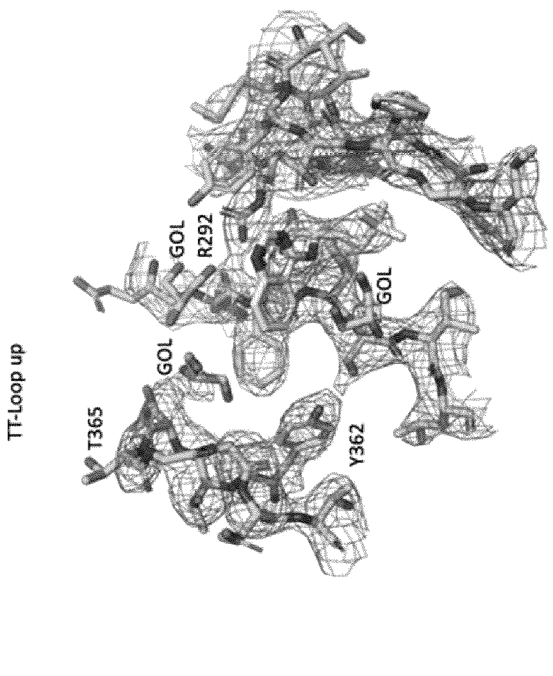


Figure 7A

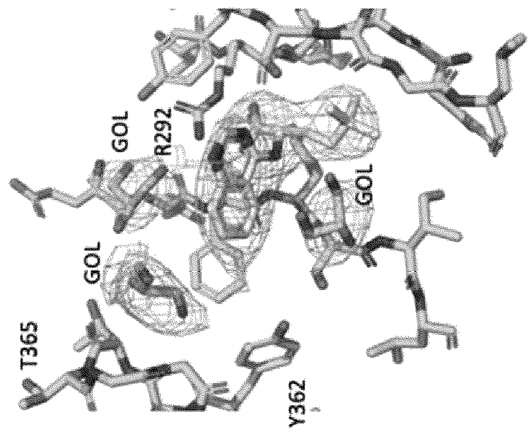


Figure 7B

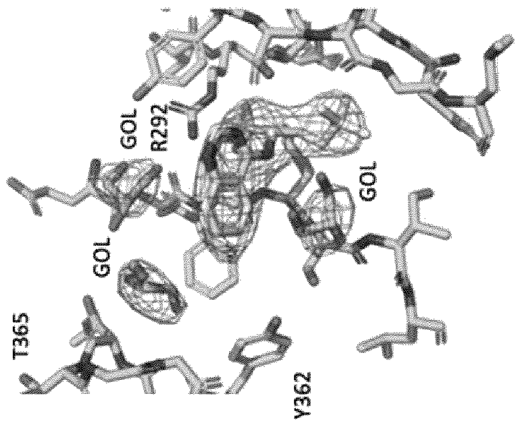


Figure 7C

Europäisches Patentamt
European Patent Office
Office européen des brevets

# EUROPEAN SEARCH REPORT

Application Number
EP 23 16 0615

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOUNG HAILEY A. ET AL: "N- Arylation of Amino Acid Esters to Expand Side Chain Diversity in Ketoxime Peptide Ligations", THE JOURNAL OF ORGANIC CHEMISTRY, vol. 85, no. 3, 3 December 2019 (2019-12-03), pages 1748-1755, XP93056660, ISSN: 0022-3263, DOI: 10.1021/acs.joc.9b02810<br>* page 1753 under general procedure for N-aryl aminoacid coupling to resin-bound peptide. Compound 3k is transformed in its acid. *<br>----- | 1-3,9,10 | INV.<br>C07D213/36<br>C07D239/42<br>C07D239/47<br>C07D239/94<br>C07D241/20<br>C07D241/44<br>C07D257/04<br>C07D401/12<br>C07D403/12<br>C07D471/04<br>A61P25/18<br>A61P25/28<br>A61P35/00<br>A61K31/44<br>C07C15/04 |
| A | WO 2022/238565 A2 (INSUSENSE APS [DK]) 17 November 2022 (2022-11-17)<br>* page 4, line 12 - line 24; claim 1 *<br>----- | 1-15 | |
| A | WO 2022/223805 A1 (INSUSENSE APS [DK]) 27 October 2022 (2022-10-27)<br>* page 4, line 4 - line 16; claim 1 *<br>----- | 1-15 | TECHNICAL FIELDS SEARCHED (IPC)<br>C07D<br>A61P<br>C07C |

The present search report has been drawn up for all claims

1

| Place of search | Date of completion of the search | Examiner |
|---|---|---|
| Munich | 30 June 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 16 0615

This annex lists the patent family members relating to the patent documents cited in the above-mentioned European search report. The members are as contained in the European Patent Office EDP file on The European Patent Office is in no way liable for these particulars which are merely given for the purpose of information.

30-06-2023

| Patent document cited in search report | Publication date | Patent family member(s) | Publication date |
|---|---|---|---|
| WO 2022238565 A2 | 17-11-2022 | EP 4089102 A1 | 16-11-2022 |
| | | WO 2022238565 A2 | 17-11-2022 |
| WO 2022223805 A1 | 27-10-2022 | EP 4079748 A1 | 26-10-2022 |
| | | WO 2022223805 A1 | 27-10-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160331746 A
- WO 2021116290 A1


### Non-patent literature cited in the description


- **SILVERMAN, R. B.** The Organic Chemistry of Drug Design and Drug Action. Elsevier Academic Press, 2004, 498-549
- **GREENE ; WUTS.** Protective Groups in Organic Synthesis. John Wiley and Sons, 2006
- **ANDERSEN et al.** *Acta Cryst. D,* 2017
- **KABSCH, W.** *Acta Cryst. D,* 2010
- **AFONINE et al.** *Acta Cryst. D,* 2012
- **EMSLEY P. et al.** *Acta Cryst. D,* 2010
- **ANDERSEN, J et al.** Identification of the first small-molecule ligand of the neuronal receptor sortilin and structure determination of the receptor-ligand complex. *Acta Crystallogr D Biol Crystallogr,* 2014, vol. 70, 451-460
- **BAKER, M. et al.** Mutations in progranulin cause tau-negative frontotemporal dementia linked to chromosome 17. *Nature,* 2006, vol. 442 (7105), 916-919
- **BROUWERS, N. et al.** Genetic variability in progranulin contributes to risk for clinically diagnosed Alzheimer disease. *Neurology,* 2008, vol. 71 (9), 656-664
- **BUTTENSHON, H.N. et al.** Increased serum levels of sortilin are associated with depression and correlated with BDNF and VEGF. *Nature Translational Psychiatry,* 2015, vol. 5 (e677), 1-7
- **CARECCHIO, M.** Cerebrospinal fluid biomarkers in Progranulin mutations carriers. *J Alzheimers Dis,* 2011, vol. 27 (4), 781-790
- **CARRASQUILLO, M. et al.** Genome-wide screen identifies rs646776 near sortilin as a regulator of progranulin levels in human plasma. *Am J Hum Genet,* 2010, vol. 87 (6), 890-897
- **CHEN, Z. Y. et al.** Sortilin controls intracellular sorting of brain-derived neurotrophic factor to the regulated secretory pathway. *J Neurosci,* 2005, vol. 25 (26), 6156-6166
- **CRUTS, M. et al.** Loss of progranulin function in frontotemporal lobar degeneration. *Trends Genet,* 2008, vol. 24 (4), 186-194
- **DE MUYNCK, L. et al.** The neurotrophic properties of progranulin depend on the granulin E domain but do not require sortilin binding. *Neurobiol Aging,* 2013, vol. 34 (11), 2541-2547
- **EGASHIRA, Y. et al.** The growth factor progranulin attenuates neuronal injury induced by cerebral ischemia-reperfusion through the suppression of neutrophil recruitment. *J Neuroinflammation,* 2013, vol. 10, 105
- **GALIMBERTI, D. et al.** GRN variability contributes to sporadic frontotemporal lobar degeneration. *J Alzheimers Dis,* 2010, vol. 19 (1), 171-177
- **GALIMBERTI, D. et al.** Progranulin as a therapeutic target for dementia. *Expert Opin Ther Targets,* 2018, vol. 22 (7), 579-585
- **GAO, A. et al.** Implications of Sortilin in Lipid Metabolism and Lipid Disorder Diseases. *DNA and Cell Biology,* 2017, vol. 36 (12), 1050-1061
- **GASS, J. et al.** Progranulin regulates neuronal outgrowth independent of sortilin. *Mol Neurodegener,* 2012, vol. 7, 33
- **GASS, J. et al.** Progranulin: an emerging target for FTLD therapies. *Brain Res,* 2012, vol. 1462, 118-128
- **GIJSELINCK, I.** Granulin mutations associated with frontotemporal lobar degeneration and related disorders: an update. *Hum Mutat,* 2008, vol. 29 (12), 1373-1386
- **GOETTSCH, C. et al.** Sortilin and Its Multiple Roles in Cardiovascular and Metabolic Diseases. *Atherosclerosis, Thrombosis and Vascular Biology,* 2017, vol. 38 (1), 19-25
- **JANSEN, P. et al.** Roles for the pro-neurotrophin receptor sortilin in neuronal development, aging and brain injury. *Nature Neuroscience,* 2007, vol. 10 (11), 1449-1457
- **HU, F. et al.** Sortilin-mediated endocytosis determines levels of the frontotemporal dementia protein, progranulin. *Neuron,* 2010, vol. 68 (4), 654-667
- **HUANG, G. et al.** Insulin responsiveness of glucose transporter 4 in 3T3-L1 cells depends on the presence of sortilin. *Mol Biol Cell,* 2013, vol. 24 (19), 3115-3122
- **KADDAI, V. et al.** Involvement of TNF-$\alpha$ in abnormal adipocyte and muscle sortilin expression in obese mice and humans. *Diabetologia,* 2009, vol. 52, 932-940

- **KJOLBY, M. et al.** Sort1, encoded by the cardiovascular risk locus 1p13.3, is a regulator of hepatic lipoprotein export. *Cell Metab,* 2010, vol. 12 (3), 213-223
- **LAIRD, A. S. et al.** Progranulin is neurotrophic in vivo and protects against a mutant TDP-43 induced axonopathy. *PLoS One,* 2010, vol. 5 (10), e13368
- **LEE, W. et al.** Targeted manipulation of the sortilin-progranulin axis rescues progranulin haploinsufficiency. *Hum Mol Genet,* 2014, vol. 23 (6), 1467-1478
- **MARTENS, L.** Progranulin deficiency promotes neuroinflammation and neuron loss following toxin-induced injury. *J Clin Invest,* 2012, vol. 122 (11), 3955-3959
- **MAZELLA, J. et al.** The 100-kDa neurotensin receptor is gp95/sortilin, a non-G-protein-coupled receptor. *J Biol Chem,* 1998, vol. 273 (41), 26273-26276
- **MIYAKAWA, S. et al.** Anti-sortilin1 Antibody Up-Regulates Progranulin via Sortilin1 Down-Regulation. *Front Neurosci,* 2020, vol. 14, 586107
- **MØLLER et al.** Sortilin as a Biomarker for Cardiovascular Disease Revisited. *Frontiers in Cardiovascular Medicine,* 2021, vol. 8, 652584
- **MORTENSEN, M.B. et al.** Targeting sortilin in immune cells reduces proinflammatory cytokines and atherosclerosis. *J Clin Invest,* 2014, vol. 124 (12), 5317-5322
- **NYKJAER, A et al.** Sortilin is essential for proNGF-induced neuronal cell death. *Nature,* 2014, vol. 427 (6977), 843-848
- **NYKJAER, A. ; WILLNOW, T. E.** Sortilin: a receptor to regulate neuronal viability and function. *Trends Neurosci,* 2012, vol. 35 (4), 261-270
- **OH, T.J. et al.** Circulating sortilin level as a potential biomarker for coronary atherosclerosis and diabetes mellitus. *Cardiovascular Diabetology,* 2017, vol. 16 (92
- **PAN, X. et al.** Sortilin and retromer mediate retrograde transport of Glut4 in 3T3-L1 adipocytes. *Mol Biol Cell,* 2017, vol. 28 (12), 1667-1675
- **PETERSEN, C. et al.** Molecular identification of a novel candidate sorting receptor purified from human brain by receptor-associated protein affinity chromatography. *J Biol Chem,* 1997, vol. 272 (6), 3599-3605
- **PICKFORD, F. et al.** Progranulin is a chemoattractant for microglia and stimulates their endocytic activity. *Am J Pathol,* 2011, vol. 178 (1), 284-295
- **POTTIER, C. et al.** Potential genetic modifiers of disease risk and age at onset in patients with frontotemporal lobar degeneration and GRN mutations: a genome-wide association study. *Lancet Neurol,* 2018, vol. 17 (6), 548-558
- **QUISTGAARD, E. et al.** Ligands bind to Sortilin in the tunnel of a ten-bladed beta-propeller domain. *Nat Struct Mol Biol,* 2009, vol. 16 (1), 96-98
- **SANTOS, A. M. et al.** Sortilin Participates in Light-dependent Photoreceptor Degeneration in Vivo. *PLoS ONE,* 2012, vol. 7 (4
- **KURUVILLA, R. et al.** A neurotrophin signaling cascade coordinates sympathetic neuron development through differential control of TrkA trafficking and retrograde signalling. *Cell,* 2004, vol. 118 (2), 243-255
- **SHI, J. ; KANDROR, K. V.** Sortilin Is Essential and Sufficient for the Formation of Glut4 Storage Vesicles in 3T3-L1 Adipocytes. *Developmental Cell,* 2005, vol. 9, 99-108
- **SCHRODER, T. et al.** The identification of AF38469: an orally bioavailable inhibitor of the VPS10P family sorting receptor Sortilin. *Bioorg Med Chem Lett,* 2014, vol. 24 (1), 177-180
- **SHENG, J. et al.** Progranulin polymorphism rs5848 is associated with increased risk of Alzheimer's disease. *Gene,* 2014, vol. 542 (2), 141-145
- **SKELDAL, S. et al.** Mapping of the Interaction Site between Sortilin and the p75 Neurotrophin Receptor Reveals a Regulatory Role for the Sortilin Intracellular Domain in p75 Neurotrophin Receptor Shedding and Apoptosis. *J Biol Chem,* 2012, vol. 21 (287), 43798-43809
- **TAURIS, J. et al.** Proneurotrophin-3 May Induce Sortilin-Dependent Death In Inner Ear Neurons. *Eur J Neuroscience,* 2020, vol. 33 (4), 622-31
- **TANG, W. et al.** The growth factor progranulin binds to TNF receptors and is therapeutic against inflammatory arthritis in mice. *Science,* 2011, vol. 332 (6028), 478-484
- **TAO, J. et al.** Neuroprotective effects of progranulin in ischemic mice. *Brain Res,* 2012, vol. 1436, 130-136
- **TENK, H.K. et al.** ProBDNF induces neuronal apoptosis via activation of a receptor complex of p75NTR and sortilin. *J Neuroscience,* 2005, vol. 10 (11), 1449-1457
- **VAN KAMPEN, J. M.** Progranulin gene delivery protects dopaminergic neurons in a mouse model of Parkinson's disease. *PLoS One,* 2014, vol. 9 (5), e97032
- **WILLNOW, T. E. et al.** VPS10P-domain receptors - regulators of neuronal viability and function. *Nat Rev Neurosci,* 2008, vol. 9 (12), 899-909
- **WILLNOW, T.E. et al.** Sortilins: new players in lipoprotein metabolism. *Current Opinion in Lipidology,* 2011, vol. 22 (2), 79-85
- **WUTS, P.G.M. ; GREENE, T.W.** Greene's Protective Groups in Organic Synthesis. John Wiley and Sons, 2006
- **XU, S.H. et al.** Regional and Cellular Mapping of Sortilin Immunoreactivity in Adult Human Brain. *Frotiers in Neuroanatomy,* 2019, vol. 13 (31), 1-27

- **YANO, H. et al.** Proneurotrophin-3 is a neuronal apoptotic ligand: evidence for retrograde-directed cell killing. *J Neurosci,* 2009, vol. 29 (47), 14790-14802
- **YIN, F. et al.** Exaggerated inflammation, impaired host defense, and neuropathology in progranulin-deficient mice. *J Exp Med,* 2010, vol. 207 (1), 117-128
- **ZHENG, Y. et al.** C-terminus of progranulin interacts with the beta-propeller region of sortilin to regulate progranulin trafficking. *PLoS One,* 2011, vol. 6 (6), e21023
- **ZHOU, X. et al.** Prosaposin facilitates sortilin-independent lysosomal trafficking of progranulin. *J Cell Biol,* 2015, vol. 210 (6), 991-1002
- **MENESES et al.** TDP-43 Pathology in Alzheimer's Disease. *Mol Neurodegeneration,* 2021, vol. 16, 84
- **PRUDENCIO et al.** Misregulation of human sortilin splicing leads to the generation of a nonfunctional progranulin receptor. *Proc Natl Acad Sci USA,* 2012, vol. 109 (52), 21510-21515
- **BEEL et al.** Progranulin reduces insoluble TDP-43 levels, slows down axonal degeneration and prolongs survival in mutant TDP-43 mice. *Mol Neurodegener,* 2018, vol. 13, 55